# FASCICULE DE BREVET EUROPEEN

(11) **EP 2 285 772 B1**
(45) Date de publication et mention de la délivrance du brevet: **13.07.2022**
(21) Numéro de dépôt: 08872753.2
(22) Date de dépôt: 16.12.2008
(51) Int. Cl.: C07C 237/12, C12N 15/88, C07C 279/14, C07D 233/58, C07C 323/41, A61K 47/54, A61K 31/7052, A61K 31/715, C07D 233/64, A61K 8/02, A61K 9/127, A61K 9/00, A61K 8/64, A61K 8/46

(54) **NOUVELLE CLASSE DE LIPIDES CATIONIQUES POUR LE TRANSPORT D'AGENTS ACTIFS DANS LES CELLULES**
NEUE KLASSE KATIONISCHER LIPIDE FÜR DEN TRANSPORT VON WIRKSTOFFEN IN ZELLEN
NEW CLASS OF CATIONIC LIPIDS FOR TRANSPORTING ACTIVE AGENTS INTO CELLS

(30) Priorité: 19.12.2007 FR 0708861
(43) Date de publication de la demande: 23.02.2011
(73) Titulaire: Oz Biosciences Sas, 13288 Marseille cedex 09 (FR)
(72) Inventeur: ZELPHATI, Olivier, 13830 Roquefort-la-Bédoule (FR); MOUTARD, Stéphane, 13010 Marseille (FR)
(74) Mandataire: Santarelli
(86) Numéro de dépôt international: PCT/FR2008/001755
(87) Numéro de publication internationale: WO 2009/106713

(56) Documents cités:
- EP-A- 0 775 751
- EP-A- 0 784 984
- EP-A1- 0 162 747
- EP-A2- 1 046 394
- WO-A-95/18146
- WO-A1-03/082809
- WO-A2-02/072068
- WO-A2-2007/079394
- US-A- 5 565 569
- US-A- 5 719 131
- MICHIKO WATANABE ET AL: "Alkylbenzyldimethylammonium Salts as Inhibitors for the Ice Nucleating Activity of Erwinia ananas", AGRICULTURAL AND BIOLOGICAL CHEMISTRY, AGRICULTURAL CHEMICAL SOCIETY OF JAPAN, JP, vol. 52, no. 1, 1 January 1988 (1988-01-01), pages 201-206, XP008121479, ISSN: 0002-1369
- Christopher L. Penney ET AL: "A simple method for the synthesis of long-chain alkyl esters of amino acids", The Journal of Organic Chemistry, vol. 50, no. 9, 1 May 1985 (1985-05-01), pages 1457-1459, XP055018028, ISSN: 0022-3263, DOI: 10.1021/jo00209a018
- Kai-Hsuan Chang ET AL: "Lithocholic acid analogues, new and potent [alpha]-2,3-sialyltransferase inhibitors", CHEMICAL COMMUNICATIONS - CHEMCOM., no. 6, 1 January 2006 (2006-01-01), page 629, XP055318467, ISSN: 1359-7345, DOI: 10.1039/b514915k
- VALIVETY R ET AL: "CHEMO-ENZYMATIC SYNTHESIS OF AMINO ACID-BASED SURFACTANTS", JOURNAL OF THE AMERICAN OIL CHEMISTS' SOCIETY (JAOCS), SPRINGER, DE, vol. 74, no. 7, 1 January 1997 (1997-01-01), pages 879-886, XP001037619, ISSN: 0003-021X, DOI: 10.1007/S11746-997-0232-8
- Stanislav Chladek: "Aminoacyl Derivatives of Nucleosides, Nucleotides, and Polynucleotides. XIV. A General Synthesis of Adenosine 2'(3')-O-Peptidyl Derivatives", J. Org. Chem., 1 January 1972 (1972-01-01), pages 2863-2867, XP055318496, Retrieved from the Internet: URL:http://pubs.acs.org/doi/pdf/10.1021/jo 00983a015 [retrieved on 2016-11-10]
- BORISSOVA R ET AL: "BIODEGRADEBLE MICROSPHERES. 17. LYSOSOMAL DEGRADATION OF PRIMAQUINE-PEPTIDE SPACER ARMS", JOURNAL OF PHARMACEUTICAL SCIENCES, AMERICAN PHARMACEUTICAL ASSOCIATION, WASHINGTON, US, vol. 84, no. 2, 1 February 1995 (1995-02-01), pages 256-262, XP000541779, ISSN: 0022-3549, DOI: 10.1002/JPS.2600840227
- VAVROVA K ET AL: "Synthetic ceramide analogues as skin permeation enhancers : structure-activity relationships", BIOORGANIC & MEDICINAL CHEMISTRY, PERGAMON, GB, vol. 11, no. 24, 1 December 2003 (2003-12-01), pages 5381-5390, XP002284922, ISSN: 0968-0896, DOI: 10.1016/J.BMC.2003.09.034
- WANG G J ET AL: "Inhibitory effects of l-arginine derivatives on endothelium-dependent vasorelaxing response to acetylcholine of the rat aorta", EUROPEAN JOURNAL OF MEDICINAL CHEMISTRY, EDITIONS SCIENTIFIQUE ELSEVIER, PARIS, FR, vol. 39, no. 7, 1 July 2004 (2004-07-01), pages 611-617, XP027179792, ISSN: 0223-5234 [retrieved on 2004-07-27]
- J-M Weibel ET AL: "Synthesis and Evaluation as a Gene Transfer Agent of a 1,2-Dimyristoyl-sn-glycero-3-pantalysine Salt", Chemistry Letters 1995, 1 January 1995 (1995-01-01), pages 473-474, XP055521294, Retrieved from the Internet: URL:https://www.journal.csj.jp/doi/pdf/10. 1246/cl.1995.473 [retrieved on 2018-11-06]
- Khursheed Anwer ET AL: "Synthetic Glycopeptide-Based Delivery Systems for Systemic Gene Targeting to Hepatocytes", Pharmaceutical Research, 1 April 2000 (2000-04-01), pages 451-459, XP055521307, New York DOI: 10.1023/A:1007533121682 Retrieved from the Internet: URL:https://link.springer.com/content/pdf/ 10.1023/A:1007533121682.pdf
- OKUSAWA T ET AL: "Relationship between Structures and Biological Activities of Mycoplasmal Diacylated Lipopeptides and Their Recognition by Toll-Like Receptors 2 and 6", INFECTION AND IMMUNITY,, vol. 72, no. 3, 1 March 2004 (2004-03-01), pages 1657-1665, XP002468539, ISSN: 0019-9567, DOI: 10.1128/IAI.72.3.1657-1665.2004
- TANG FUXING ET AL: "Introduction of a Disulfide Bond into a Cationic Lipid Enhances Transgene Expression of Plasmid DNA", BIOCHEMICAL AND BIOPHYSICAL RESEARCH COMMUNICATIONS, vol. 242, no. 1, 1998, pages 141-145, XP029094729, ISSN: 0006-291X, DOI: 10.1006/BBRC.1997.7923

## Description

La présente invention a pour objet une nouvelle famille de lipides cationiques et leur utilisation comme vecteurs de délivrance *in vitro, ex vivo* et *in vivo* d'agents biologiquement actifs, notamment d'acides nucléiques, de peptides, de protéines, de polysaccharides et de lipides dans les cellules vivantes, dans les tissus, dans les organes et/ou dans les organismes, humains, animaux et/ou végétaux.

La délivrance intracellulaire d'agents biologiquement actifs trouve application dans de nombreux domaines allant de la biologie à la médecine. En biologie, l'introduction dans les cellules d'acides nucléiques (transfection) (gène ou plasmides, ADN codant linéaire, chromosomes artificiels, ARN messager, ARN interférents, ARN double brin comme les ARNsh), d'oligonucléotides antisens, de ribozymes peut être utilisée notamment pour l'étude de la régulation de l'expression de gènes ou pour l'élucidation de leur fonction. De même, le transport intracellulaire de peptides, de protéines, de polysaccharides, de lipides et de toute autre molécule biologiquement active permet d'appréhender et d'étudier les mécanismes biologiques fondamentaux. En médecine, ces techniques ont été développées pour la délivrance de peptides ou de protéines thérapeutiques, d'oligonucléotides antisens et de ribozymes (thérapies antisens), d'ARNsi et de gènes (thérapie génique) pour pallier entre autres à une déficience métabolique, à un désordre génétique ou à une infection.

Il existe de nombreuses méthodes pour introduire des acides nucléiques dans les cellules que l'on peut classer en trois catégories: physiques, biochimiques et biologiques. Les méthodes biologiques font appels à des organismes infectieux comme les virus ou les bactéries. Les approches physiques comprennent notamment l'électroporation, la micro-injection, la sonoporation ou la magnétofection. Enfin, les méthodes biochimiques de transfection associent des réactifs chimiques aux acides nucléiques (Conwell, C.G. et col. (2005) Adv. Genet. 53, 1-18). D'une part, le phosphate de calcium ou le dextran DEAE sont utilisés dans des méthodes de co-précipitation, d'autre part des polymères cationiques substitués ou non par des ligands, des lipides cationiques sous forme de systèmes organisés ou non (liposomes, vésicules unilamellaires ou multilaméllaires, phases hexagonales, micelles) et/ou un mélange des ces différentes entités, forment des complexes (polyplexes, lipoplexes ou lipopolyplexes) avec les acides nucléiques via des interactions électrostatiques et permettent leur passage à travers les membranes cellulaires.

Parmi les principaux lipides cationiques connus, on peut notamment citer de manière non exhaustive et à titre d'exemples:
- des lipides cationiques monovalents sous forme de sels d'ammonium quaternaires, tels que le DOTMA (chlorure de N-[1-(2,3-dioleyloxy)propyl]-N,N,N-trimethylammonium) (Felgner, P.L. et col. (1987) Proc. Natl. Acad. Sci. USA 84 (21), 7413-7417) commercialisé en association avec un lipide neutre, le DOPE (dioléoylphosphatidyléthanolamine), sous le nom de Lipofectin^{™} ; les analogues métabolisables du DOTMA tels que le DOTAP (1,2-dioleoy-3-trimethylammonium propane) (Leventis, R. et Silvius, J.R. (1990) Biochim. Biophys. Acta 1023, 124-132) ; le DMRIE (Felgner, J.H. et col. (1994) J. Biol. Chem. 269(4), 2550-2561) ; ou bien encore le DDAB (bromure de dioctadécyldiméthyl ammonium), commercialisé en association avec un lipide neutre sous la dénomination de TransfectACE^{™} ;
- des lipides cationiques monovalents sous forme de sels de pyridinium tels que le SAINT-2 (chlorure de N-méthyl-4-(dioleyl)méthylpyridinium) (Ruiters, M.H.J., PCT WO2006/043809) ;
- des lipides cationiques multivalents sous forme de lipospermines tels que le DOGS (5-carboxyspermylglycine-dioctadécylamine) (Behr, J.-P. et col. (1989) Proc. Natl. Acad. Sci. USA 86 (18), 6982-6986) et le DOSPA (Trifluoroacétate de 2,3-dioleyloxy-N-[2(sperminecarboxamido)ethyl]-N,N-dimethyl-propanammonium), fournies sous les dénominations commerciales respectives de Transfectam^{™} et Lipofectamine^{™} ;
- des lipides cationiques multivalents sous forme de lipopolylysines (Zhou, X. et col. (1991) Biochem. Biophys. Acta 1065, 8-14) ;
- des dérives cationiques du cholestérol tel que le DC-Chol (3β[N-(N',N'-dimethylaminomethane)-carbamoyl]cholestérol) (Gao, X. & Huang, L. (1991) Biochem. Biophys. Res. Commun. 179 (1), 280-285).
- on citera également ceux décrits dans le document US 5,719,131 qui intègrent un groupement spermine ou spermidine.
- WO 9958152 A1 décrit de nouveaux matériaux et procédés pour l'administration de substances, telles que l'ADN ou des polypeptides, dans des cellules. Ces composés comportant un pont disulfure ont démontré une certaine efficacité restreinte à la délivrance d'ADN.

La délivrance d'acides nucléiques par des lipides cationiques formulés (lipofection) présente de nombreux avantages car ils ne sont pas immunogéniques, contrairement aux agents viraux, sont simples à utiliser, permettent de délivrer des acides nucléiques sans limite de taille, et peuvent être produits en grande quantité. De plus, la lipofection est la méthode la plus employée dans les laboratoires de recherches pour transfecter les cellules *in vitro* grâce à sa facilité d'utilisation, son efficacité en présence ou en absence de sérum pour une grande variété de types cellulaires, notamment les cellules adhérentes et sa versatilité pour la délivrance des acides nucléiques.

Toutefois, ces vecteurs synthétiques sont peu ou ne sont pas efficaces pour la transfection de certains types cellulaires, notamment les cellules en suspension comme les lymphocytes, les cellules souches, les cellules primaires (ne se divisant pas)... L'efficacité réduite des lipides cationiques à transfecter certaines cellules comme les lignées cellulaires en suspension s'explique par une faible capacité des lipoplexes à s'attacher à la surface de ces cellules. En effet, contrairement aux cellules adhérentes, celles-ci possèdent très peu de protéines transmembranaires polyanioniques comme les Heparan Sulfate Proteoglycans (HSPGs) qui sont les principaux points d'encrage des lipoplexes sur les membranes plasmiques (Kopatz, I. et col. (2004) J. Gene Med. 6, 769-776 ; Wiethoff, C.M. et col. (2001) J. Biol. Chem. 276, 32806-32813 ; Mislick K.A. et col. (1996) Proc. Natl. Acad. Sci. USA 93, 12349-12354). Néanmoins, diverses approches utilisant des ligands spécifiques tels que la transferrine (Kursa, M. (2003) Bioconjugate Chem. 14, 222-231 ; Kakudo, T. et col. (2004) Biochemistry 43, 5618-5628), le facteur épidermique de croissance (epidermal growth factor EGF), des anticorps monoclonaux (Guillem, V.M. et col. (2002) J Controlled Released 83, 133-146 ; Guillem, V.M. et col. (2002) J. Gene Med. 4, 170-182 ; Puls, R.L. et col. (1999) Gene Ther. 6, 1774-1778 ; Thurnher, M. et col. (1994) Glycobiology 4 (4), 429-435) ou des peptides (Wagner, E. (1999) Adv. Drug Deliv. Rev. 38 (3), 279-289 ; Uduehi A. et col. (2003) Biotechnol. Appl. Biochem. 38, 201-209), couplés ou associés à un polymère cationique ou à un co-lipide formulé avec les lipides cationiques, ont été testés avec succès. Les cellules quiescentes sont également très difficiles à transfecter au moyen de la lipofection, car en l'absence de moyens de ciblage du noyau tels que des séquences de localisation nucléaire, les complexes présents dans le cytosol ont du mal à passer à travers les pores de la membrane nucléaire et à être ainsi transcrit. Plusieurs approches utilisant des complexes ternaires (lipoplexes: lipides cationiques et acides nucléiques plus un élément de ciblage nucléaire) à base d'histones, de séquences de localisation nucléaire peptidiques, ont été étudiées pour contourner ce problème (Khalil I.A. (2006) Pharmacol. Rev. 58, 32-45 ; Medina-Kauwe, L.K. (2005) Gene Ther. 12, 1734-1751; Escriou, V. et col. (2003) Adv. Drug Deliv. Rev. 55 (2), 295-306 ; Ma, H. et col. (2001) Curr. Pharm. Biotechnol. 2 (1), 1-17; Hagstrom, J.E. (1996) Biochim. Biophys. Acta 1284 (1), 47-55).

Cependant, l'addition de ces éléments de ciblage aux lipides cationiques rend la formulation et la préparation des complexes délicates et leur utilisation est ainsi très limitée. De plus, leur efficacité *in vitro* et *in vivo* demeure incertaine.

Par conséquent, le développement de nouveaux lipides cationiques intégrant dans la même molécule les fonctions de complexation aux acides nucléiques et de ciblages permettrait de s'affranchir de l'utilisation d'éléments de ciblage supplémentaires (membrane et noyau) et apporterait une solution à ces obstacles majeurs.

Par ailleurs, l'efficacité *in vivo* de la lipofection demeure trop faible (Evans, C.H. et col. (2006) Adv. Drug Deliv. Rev. 58, 243-258) et de nouveaux lipides plus performants sont nécessaires. Leur application *in vivo* est limitée par la dégradation enzymatique des complexes, leur pharmacologie et par la présence de protéines et de polysaccharides dans les fluides corporels et le mucus, qui inhibent fortement la transfection. Les principaux paramètres qui affectent les efficacités de transfection des lipoplexes ont été largement étudiés (Solodin, I. et col. (1995) Biochemistry 34 (41), 13537-13544 ; Templeton, N. S. et col. (1997) Nat. Biotech. 15 (7), 647-652 ; Thierry, A. R. et col. (1995) Proc. Natl. Acad. Sci. USA 92 (21), 9742-9746 ; Li, S. et col. (1997) Gene Ther. 4 (9), 891-900 ; Liu, Y. et col. (1997) Nat. Biotechnol. 15 (2), 167-173 ; Liu, F. et col. (1997) Gene Ther. 4 (6), 517-523 ; Song, Y. K. et col. (1997) Hum. Gene Ther. 8 (13), 1585-1594; Hong, et col. (1997) FEBS Letters 400 (2), 233-237). Ces études ont montré qu'on obtenait les meilleurs niveaux d'expression de gènes *in vivo* en utilisant des ratios lipides/acides nucléiques relativement haut.

Néanmoins, l'utilisation d'un large excès de lipides cationiques ou de lipoplexes s'accompagne souvent d'une toxicité significative.

Ainsi, la cytotoxicité des lipides cationiques demeure un des inconvénients majeurs de cette méthode, aussi bien *in vitro* qu'*in vivo.* Elle est due, principalement, à une mauvaise biodégradabilité des lipides cationiques qui ne sont pas naturellement présent dans les cellules. De nombreux efforts ont été effectués dans cette optique. Ainsi, l'équipe de Scherman a développé des lipides présentant une liaison sensible au milieu réducteur dans les chaînes grasses afin de faciliter leur métabolisme (WO 9938821). D'autre part, Boomer et col. ont incorporé un groupement vinyl éther, sensible au pH, et plus particulièrement au milieu acide, dans le bras espaceur de leur lipide cationique afin d'induire un clivage rapide du lipide dans le milieu acide des endosomes. Ce clivage entraîne une déstabilisation de la structure des lipoplexes qui sont enchâssés dans la membrane des endosomes et permet une libération précoce de l'ADN des endosomes vers le cytosol avant que celle-ci ne soit dégradée par les nucléases (Boomer, J.A. (2002) Gene Transfer Pharm. Res. 19 (9), 1292-1301). Dans le même but, l'équipe de Szoka a construit une famille de lipides cationiques munie d'un bras espaceur contenant un groupement ortho ester linéaire ou cyclique, stable à pH physiologique, et qui s'hydrolyse à pH acide (Chen, H. et col. (2007), J. Med. Chem. 50 (18), 4269-4278). Enfin, la présence de groupements esters dans les lipides cationiques permet une meilleure biodégradabilité de ceux-ci dans les cellules, car les groupements esters sont facilement clivés par les estérases endogènes. Il existe de nombreux exemples de lipides cationiques contenant des groupements esters parmi lesquels on peut citer les tétraesters DMTM(Gly) et DOTM(Gly), synthétisés par l'équipe de Nantz, et dont l'influence sur la diminution de la cytotoxicité à été étudiée (Aberle, A.M. et col. (1998) Biochemistry 37 (18), 6533-6540).

Tous ces lipides ont montré une meilleure biodégradabilité en milieu intracellulaire, induisant une très faible cytotoxicité. Néanmoins, ces entités n'ont pas intégré les éléments de ciblage décrit précédemment et ont ainsi une efficacité réduite sur les cellules en suspension et non mitotique. C'est pourquoi il est nécessaire de concevoir de nouveaux lipides complètement biodégradables dans les cellules et contenant les éléments de ciblage mentionnés ci-dessus.

Actuellement il existe un grand nombre de lipides cationiques commerciaux (réactif de transfection) développés spécifiquement pour le transport d'acides nucléiques. Les procédures de transfection utilisant ces réactifs sont communément utilisées dans la plupart des laboratoires biomédicaux. Curieusement, beaucoup moins de progrès ont été réalisés dans la conception de réactifs dédiés au transport d'autres biomolécules tels que les peptides et les protéines, malgré des ressources considérables consacrées à l'isolation et l'évaluation de peptides, d'anticorps, d'antigènes et de protéines recombinantes. En fait, toutes les protéines recombinantes et les anticorps monoclonaux actuellement utilisés en clinique sont dirigés vers une cible extracellulaire et non intracellulaire (Krejsa, C. et col. (2006) Nat Rev Drug Discov 5, 507-521). S'il existait une méthode efficace pour délivrer des peptides, des protéines ou toutes autres biomolécules à l'intérieur des cellules, il ne serait pas nécessaire de restreindre les candidats potentiels à un traitement thérapeutique aux molécules secrétées ou membranaires. L'introduction directe de peptides et/ou de protéines dans les cellules peut être utile dans des domaines variés tels que la régulation du cycle cellulaire, le contrôle de l'apoptose, l'immunologie et la régulation de la transcription. Cette approche peut ainsi permettre aux chercheurs d'étudier la fonction des molécules transportées, de bloquer ou d'induire une fonction intracellulaire dans les cellules vivantes, de développer leur utilisation thérapeutique potentielle contre un grand nombre de maladies aussi diverses que le cancer, les inflammations et les infections ainsi que de développer de nouveaux vaccins. Par exemple, le transport d'anticorps monoclonaux dans les cellules peut être utilisé pour bloquer de manière spécifique une cible intracellulaire. Cette approche a déjà été démontrée via la transfection d'ADN codant pour des anticorps (" Intrabodies ") (Mhashilkar, A. M. et col. (1997) J Virol 71, 6486-6494; Chen, S. Y. et col. (1994) Hum Gene Ther 5, 595-601 ; Shaki-Loewenstein, S. et col. (2005) J Immunol Methods 303, 19-39 ; Williams, B. R. et col. (2006) Curr Med Chem 13, 1473-1480). Toutefois, cette stratégie est longue et laborieuse, et le transport direct d'anticorps recombinants peut s'avérer être une méthode beaucoup plus attractive car ces anticorps sont notamment très faciles à produire en grande quantité.

Plusieurs approches ont été étudiées pour transporter des peptides, des protéines et d'autres biomolécules fonctionnelles dans des cellules. La micro-injection et l'électroporation ont été utilisées pour introduire des protéines fonctionnelles à l'intérieur des cellules avec des degrés variables de succès (Marrero, M. B. et col. (1998) J. Biol. Chem. 270, 15734-15738 ; Fenton, M. et col. (1998) J. Immunol. Methods 212, 41-48 ; Abarzua, P. et col. (1995) Cancer Res. 55, 3490-3494). L'approche la plus étudiée pour le transport de macromolécules, de peptides ou de protéines dans les cellules emploie une classe spéciale de peptides et/ou de protéines ayant la capacité de passer à travers les membranes cellulaires par un mécanisme dit de transduction. (Schwarze, S. R. et col. (2000) Trends Cell Biol. 10, 290-295 ; Murriel, C. L. et col. (2006) Expert Opin Drug Deliv 3, 739-746). C'est en fait une courte séquence peptidique riche en résidus basiques (essentiellement des lysines et des arginines) appelée PTD ("Protein Transduction Domain") ou CPP ("Cell-Penetrating Peptides") qui confère à ces protéines la propriété de transloquer à travers les parois des cellules et également d'atteindre le noyau des cellules. Les trois principaux exemples de PTD sont les protéines HIV-1 TAT (Trans-activating transcriptional activator : Trans-activateur de transcription) (Green, M. et col. (1988) Cell 55, 1179-1188 ; Frankel, A. D. et col. (1988) Cell 55, 1189-1193), HSV-1 VP22 (Herpes Simple Virus Type I VP 22 Transcription Factor) (Elliott, G. et col. (1997) Cell 88, 223-233) et Antp (Drosophile Antennapedia Homeotic Transcription Factor) (Joliot, A. et col. (1991) Proc. Natl. Acad. Sci. USA 88, 1864-1868).

Toutefois, une des principales limitations de l'utilisation des PTDs est la nécessité de former une liaison covalente, soit par voie chimique, soit par clonage, entre la séquence PTD et la molécule d'intérêt, ce qui n'est pas sans conséquence sur l'activité biologique de cette dernière. Par exemple, il peut entraîner une modification de la conformation de la protéine ou bien encore interférer avec sa fonction par encombrement stérique.

De plus, l'efficacité de ces systèmes est fortement dépendante de la structure et de la taille de la molécule à transporter. Ils sont très efficaces pour des peptides ou de petites protéines solubles présentant une versatilité structurale, mais sont limités pour des systèmes complexes et multimériques.

Ainsi, les PTDs ne sont pas efficaces pour le transport d'ADN contrairement aux lipides cationiques. Les scientifiques doivent donc actuellement employer des méthodes sophistiquées, longues et limitées dans leur application pour le transport intracellulaire de molécules biologiquement actives. A l'heure actuelle, la méthode indirecte consistant à transfecter un ADN codant pour une protéine demeure celle la plus utilisée pour obtenir cette protéine dans des cellules.

Il existe donc un besoin crucial de développer un système de transport simple et robuste, similaire aux agents de transfection, pour la délivrance intracellulaire de peptides, de protéines, d'anticorps et autres biomolécules.

En effet, une formulation lipidique permettant d'associer rapidement et de manière non covalente les protéines et autres molécules, de protéger celles-ci de l'environnement biologique et de les transporter à l'intérieur des cellules sans limite de taille ou sans altérer leur fonction apporterait un bénéfice indéniable dans tous les aspects de la biologie cellulaire, la génomique, la génomique fonctionnelle et la protéomique. Or, la majorité des lipides cationiques employés en transfection s'avèrent complètement inefficaces pour le transport des autres biomolécules à l'intérieur des cellules. Néanmoins, quelques exemples ont été rapportés sur l'utilisation de lipides cationiques pour le transport de protéines dans les cellules (Debs, R. J. et col. (1990) J. Biol. Chem. 265, 10189-10192 ; Baubonis, W. et col. (1993) Nucleic Acids Res 21, 2025-2029 ; Huang, L. et col. (1995) Biochem. Biophys. Res. Commun. 217, 761-768 ; Farhood, H. et col. (1995) Anal. Biochem. 225, 89-93 ; Guillaume, C. et col. (2000) J. Pharm. Sci. 89, 639-645 ; Sells, M. A. et col. (1995) BioTechniques 19, 72-78 ; Walker, C. et col. (1992) Proc. Natl. Acad. Sci. USA 89, 7915-7918 ; Zelphati, O. et col. (2001) J Biol Chem 276, 35103-35110 ; Dalkara, D. et col. (2004) Mol Ther 9, 964-969). La lipofectin, le DC-Chol et le TransACE ont été utilisés pour co-transporter de l'ADN associée à des protéines (Baubonis et col. (1993) ; Debs et col. (1990) ; Farhood et col. (1995)). Cependant, leur efficacité reste très faible, et a nécessité l'addition d'agent lysosomotropique (Debs et col. (1990)). De plus, le rôle de ces lipides cationiques demeure questionnable, les protéines utilisées étant en mesure d'entrer dans les cellules seules (Farhood et col. (1995) ; Huang et col. (1995)). Walker et col. ont également utilisé un lipide cationique (DOTAP) pour transporter un antigène dans les cellules, mais la discrimination entre la présentation de l'antigène à l'intérieur ou à l'extérieur des cellules est difficile à établir et la fonctionnalité de la protéine transportée n'est pas démontrée (Walker et col. (1992)). Des lipides phosphonocationiques (GLB73 et GLB43) ont encore été utilisés *in vitro* et *in vivo* pour transporter la β-galactosidase, mais sans mettre en évidence l'influence des lipides sur le nombre de cellules positives à la β-galactosidase, aucune autre protéine fonctionnelle n'ayant été testée par ailleurs (Guillaume et col. (2000)). Dans la plupart des exemples mentionnés ci-dessus, l'efficacité de transport a été évaluée après fixation et perméabilisation des cellules, ce qui complique l'interprétation des résultats. Plus récemment, Zelphati et col. ont développé un lipide cationique trifluoroacétylé pour la vectorisation intracellulaire de protéines (Zelphati et col. (2001)). Celui-ci a permis de transporter dans des cellules vivantes un certain nombre de protéines biologiquement actives et s'est avéré plus efficace que de nombreux lipides cationiques commerciaux. Des études similaires ont ensuite été réalisées avec les lipides DOGS et Chol-Sper (Dalkara, D. et col. (2006) J Control Release 116, 353-359 ; Dalkara et col. (2004)). Jusqu'à l'heure actuelle, l'utilisation de lipides cationiques semble moins bien adaptée au transport de protéines qu'au transport d'acides nucléiques, entre autres parce qu'il n'existe toujours aucun système lipidique connu qui permette la délivrance de protéines en présence de sérum.

Ainsi, la vectorisation intracellulaire de protéines, de peptides et d'autres molécules, hormis les acides nucléiques, reste une approche extrêmement isolée et limitée.

Bien qu'il existe un certain nombre de produits commerciaux à base de lipides cationiques, efficaces pour la délivrance d'acides nucléiques dans les cellules en culture, et quelques uns plus spécialement mis au point pour le transport de protéines, aucun d'entre eux n'est adapté pour le transport de tous les types de biomolécules en présence de sérum. Par ailleurs, ces agents de transfection sont peu efficaces sur les cellules en suspension et primaires et s'accompagnent souvent d'une forte toxicité qui limite leur action *in vivo.*

La présente invention a pour objet de fournir une nouvelle famille universelle de lipides cationiques, compatibles avec la présence de sérum et non toxiques, permettant une délivrance efficace *in vitro, ex vivo* et *in vivo* de tous les types de biomolécules telles que les acides nucléiques, les peptides, les protéines, les polysaccharides et les lipides dans les cellules vivantes.

En effet, la structure innovante de ces nouveaux lipides cationiques permet d'allier :
1. les propriétés amphiphiles des lipides cationiques pour former des structures organisées (liposomes, micelles...) permettant le transport et la vectorisation de molécules actives vers leur cible, leurs propriétés à former des complexes non covalents avec les acides nucléiques chargés négativement (lipoplexes), et leurs propriétés à déstabiliser les membranes cellulaires.
2. les propriétés de la tête cationique, constituée par des acides aminés basiques présents dans les PTD, connus pour leur aptitude à s'attacher aux cellules et passer les barrières transmembranaires dont la barrière nucléaire. En outre, ces séquences d'acides aminés basiques interagissent naturellement avec plusieurs types de biomolécules dont les acides nucléiques, les peptides et les protéines et sont complètement non toxiques. Par ailleurs, ces nouveaux composés sont capables de transfecter les cellules avec une grande efficacité en présence de sérum grâce à leur faible capacité à interagir avec les composantes du milieu sérique.
3. la capacité à se dégrader rapidement en milieu intracellulaire grâce à la présence d'un bras espaceur entre la partie lipophile et la tête cationique munie d'un groupement fonctionnel incorporant une liaison sensible à son environnement (pH, oxydo-réduction, enzymes...) et clivable en milieu cytosolique. Les composés issus de la dégradation qui en résultent sont des molécules naturelles (acides gras, acides aminés naturels) facilement métabolisés par la cellule. Le caractère biodégradable de ces molécules les rend par conséquent non cytotoxiques.

Pour atteindre ce but, l'invention a pour objet un composé amphipathique cationique de formule (I) tel que défini dans les revendications.

En particulier, l'invention concerne un composé amphipathique cationique de Formule (I) : 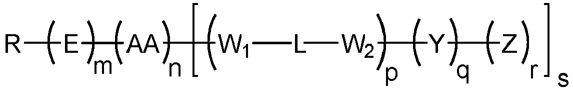 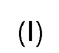 dans laquelle :
R répond à la formule (II) : 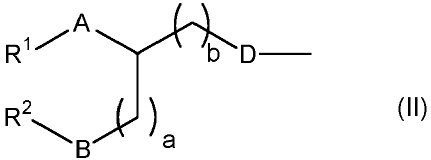
dans laquelle :
   - R¹ et R², identiques ou différents, représentent un groupe hydrocarboné, linéaire, ramifié et/ou cyclique, saturé ou insaturé, comprenant de 6 à 24 atomes de carbone ;
   - A et B, identiques, représentent un groupement -C(O)-O- ; -CO-NH- ; -NH-CO- ; -NH- ou -O- ;
   - A et B, différents, représentent un groupement -C(O)-O- ; -O-C(O)- ; - CO-NH- ; -NH-CO- ; -NH- ou -O- ;
   - a est un entier compris entre 1 et 6 ;
   - b est un entier compris entre 0 et 6 ;
   - D représente un groupement -NH-, -CO-, ou -S- ;
   - E représente un groupe hydrocarboné, linéaire ou ramifié, de formule (V) : -G₁-X₁-G₁-, dans laquelle
      - X₁ représente un groupe alkylène, linéaire ou ramifié, formant pont et comprenant de 1 à 8 atomes de carbone, et
      - G₁ représente un groupe -CO- ou -NH- ;
   - m est 1 ;
   - AA représente le reste d'un acide aminé ;
   - n est un entier égal à 0 ou à 1 ;
   - W₁ répond à la formule (VI) : -G₂-X₂- et W₂ répond à la formule (VII) : -X₃-G₃- dans lesquelles X₂ et X₃, identiques ou différents, représentent un groupe alkylène, linéaire ou ramifié, formant pont et comprenant de 1 à 8 atomes de carbone, tandis que G₂ et G₃, identiques ou différents, représentent un groupe -CO-, -NH- ou -O- ;
   - L représente un groupement ester (-CO-O-), disulfide (-S-S-), éther de vinyle (-O-C=C-), acylhydrazone (-CO-NR-N=CR'R") ;
   - p est un entier égal à 0 ou à 1 ;
   - Y est un groupement ramifié qui répond à la formule (VIII) : -CO-X₄-NH-X₅-N-[X₆-NH]₂- ou (IX) : -NH-X₅-N-[X₆-NH]₂-, dans lesquelles X₄, X₅ et X₆, identiques ou différents, représentent un groupe alkylène, linéaire ou ramifié, formant pont et comprenant de 1 à 8 atomes de carbone ;
   - q est un entier compris entre 0 et 8 ;
   - Z représente un acide aminé basique ou la sérine ;
   - r est un entier compris entre 1 et 16, étant entendu que si q est égal à 1 alors r est au moins égal à 2 et que si r est supérieur à 1, alors les groupements Z peuvent être identiques ou différents ;
   - s est un entier égal à 1 ou 2 ;
   et ses sels d'addition physiologiquement acceptables.

L'invention concerne également un composé choisi parmi

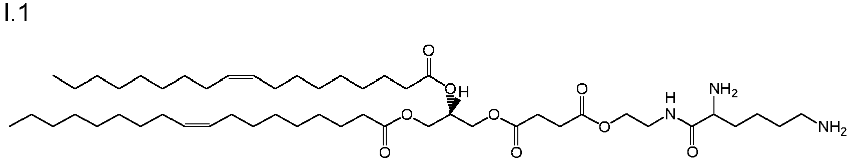

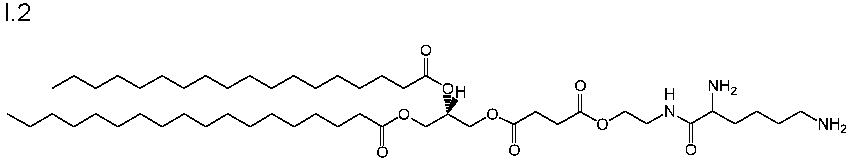

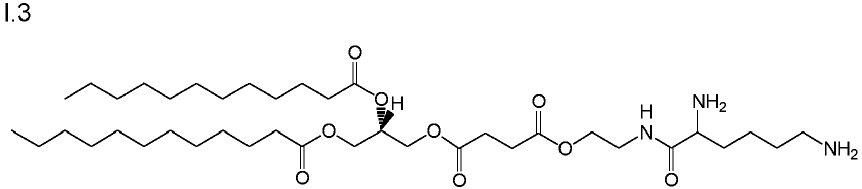

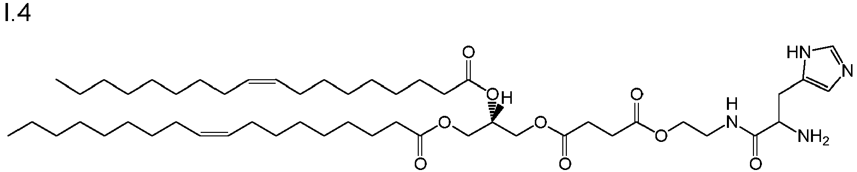

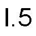

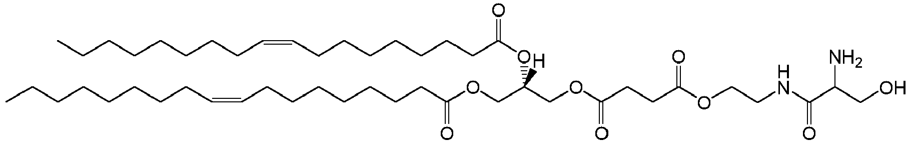

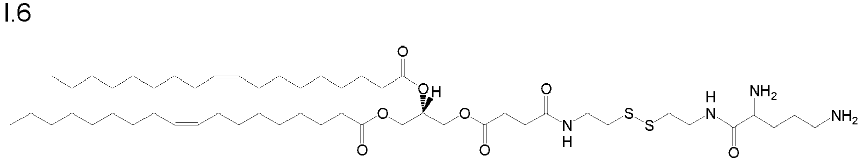

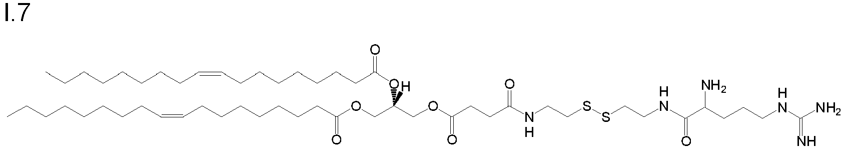

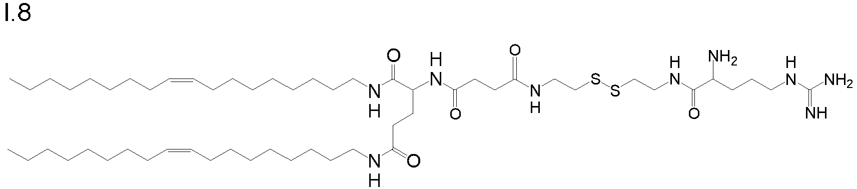

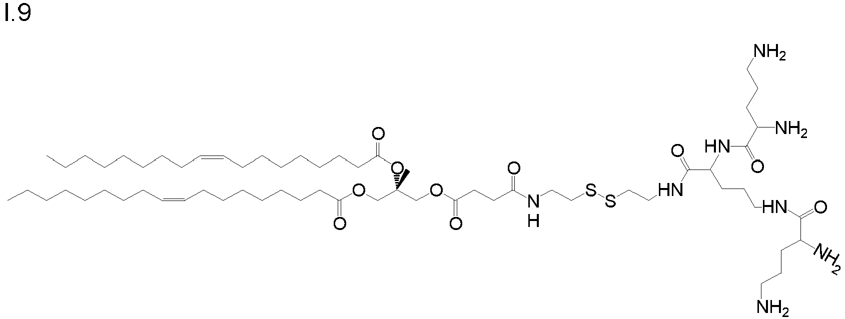

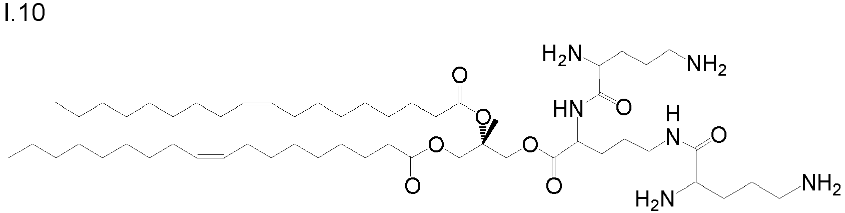

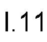

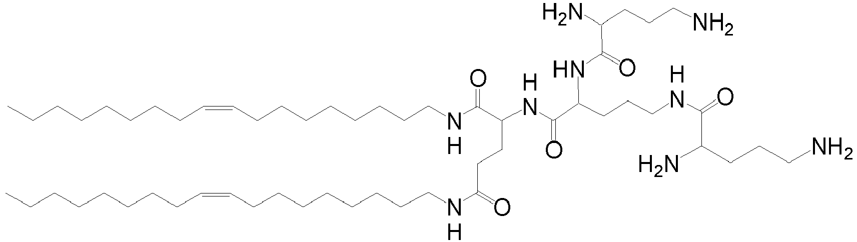

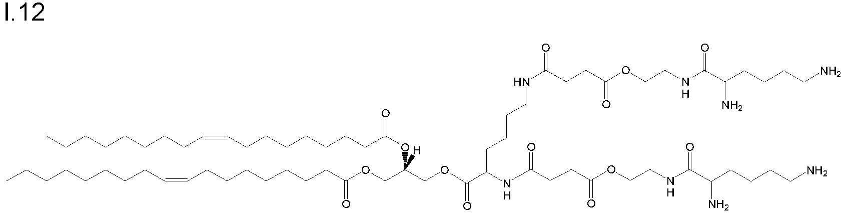

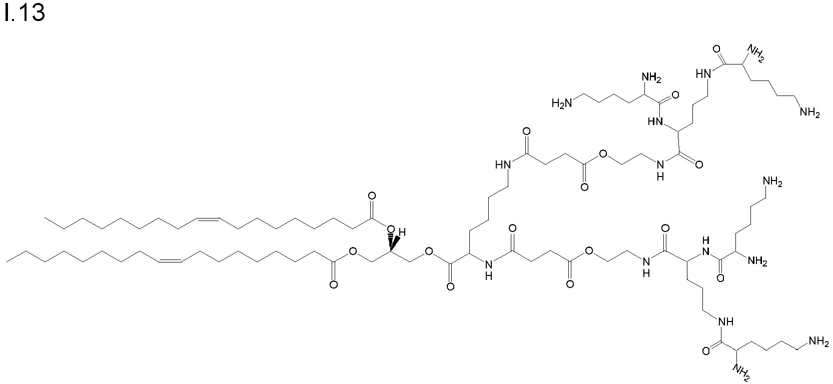

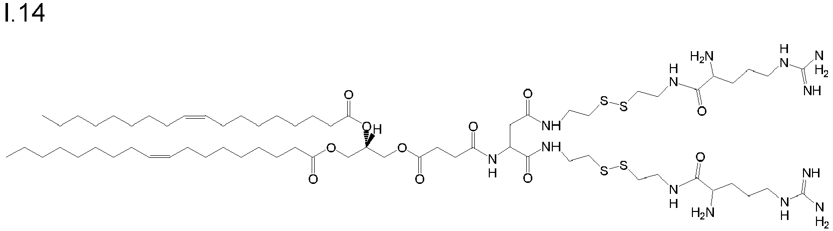

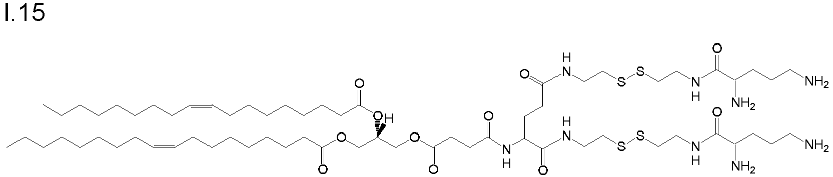

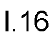

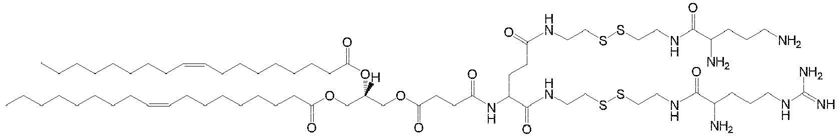

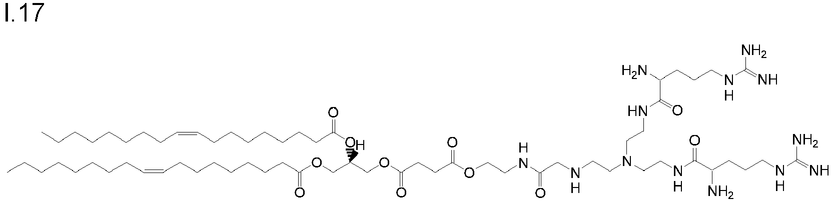

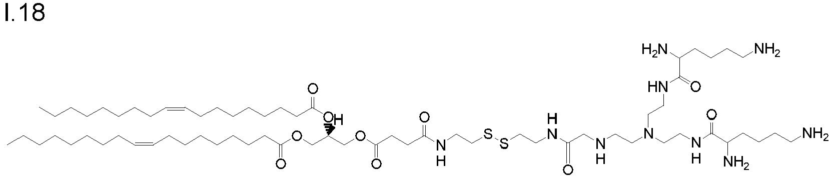

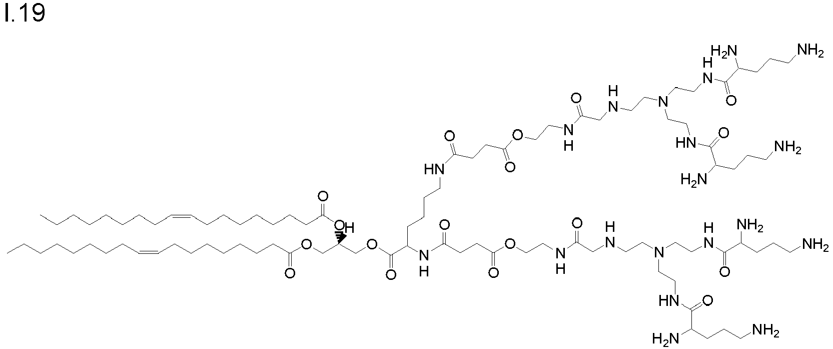

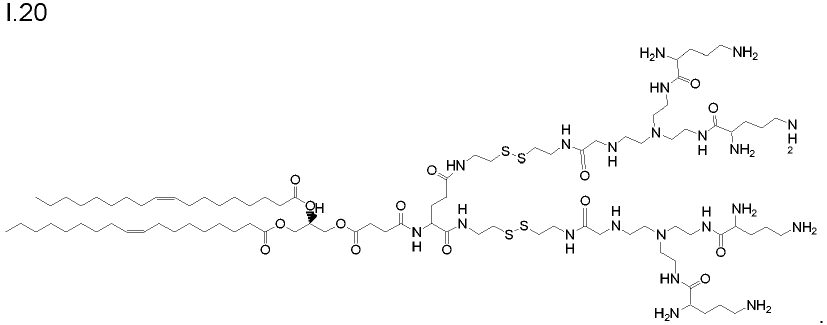

L'invention concerne également une composition comprenant un composé tel que défini précédemment. L'invention concerne également un composé tel que défini précédemment pour son utilisation dans le transfert d'acides nucléiques, de polypeptides ou de tout autre molécule biologiquement active dans les cellules.

L'invention concerne également l'utilisation d'un composé tel que défini précédemment pour la préparation d'une composition destinée au transfert d'acides nucléiques, de polypeptides ou de tout autre molécule biologiquement active dans les cellules.

L'invention concerne également une composition tel que définie précédemment pour son utilisation dans le transfert d'acides nucléiques, de polypeptides ou de tout autre molécule biologiquement active dans les cellules.

L'invention concerne également une méthode *in vitro ou ex vivo* de transfert d'une molécule d'intérêt biologique dans les cellules comprenant les étapes suivantes :
1. la mise en contact de la molécule d'intérêt biologique avec un composé, tel que défini précédemment, ou avec une composition telle que définie précédemment, pour former un complexe molécule active / agent de transfert,
2. la mise en contact des cellules avec le complexe formé en 1.

L'invention concerne également un kit de transfert de matériel biologique, caractérisé en ce qu'il comprend au moins un composé tel que défini précédemment ou une composition telle que définie précédemment.

L'invention concerne également un kit de transfert pour la mise en œuvre de la méthode telle que définie précédemment, caractérisé en ce qu'il comprend au moins un composé tel que défini précédemment ou une composition telle que définie précédemment.

Il est également décrit (non compris dans l'invention revendiquée) un composé amphipathique cationique de formule (I): 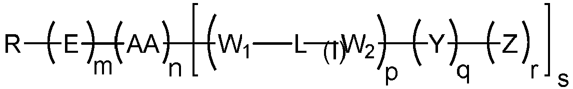 dans laquelle :
- R représente une région lipophile qui peut comprendre
   - une ou plusieurs chaînes alkyle, comprenant 6 à 24 atomes de carbone, préférentiellement entre 10 et 18 atomes de carbone, ramifiée ou linéaire, insaturée ou saturée, éventuellement fluorée ; ou
   - un ou plusieurs groupes cycliques ou polycycliques connus pour être lipophile comme un groupe stéroïde (par exemple un dérivé du cholestérol), un groupe polyaromatique (par exemple dérivé du naphtalène, du dansyle, de l'anthracène), ou un groupe dérivé d'alcaloïde ; ou
   - un lipide naturel ou synthétique.

Eventuellement, R peut être constitué d'une combinaison de ces différents groupements. R peut comporter un ou plusieurs hétéroatomes.
- E représente un groupe hydrocarboné, linéaire ou ramifié, pouvant comprendre de 1 à 15 atomes de carbone, préférentiellement de 1 à 8, et pouvant comporter éventuellement un ou plusieurs hétéroatomes ;
- m est un entier égal à 0 ou à 1 ;
- AA représente le reste d'un acide aminé ;
- n est un entier égal à 0 ou à 1 ;
- W₁ et W₂, identiques ou différents, représentent un groupe hydrocarboné, linéaire ou ramifié, pouvant comprendre de 1 à 15 atomes de carbone, préférentiellement de 1 à 6, pouvant éventuellement comprendre un ou plusieurs hétéroatomes ;
- L représente un groupement fonctionnel pouvant incorporer au moins une liaison sensible à son environnement, stable en milieu extracellulaire et rapidement clivée dans le milieu intracellulaire car sensible
   - à des stimuli tels que la décroissance du pH (par exemple des groupements éther de vinyle ou acylhydrazone sensibles au milieu acide) ou un changement du potentiel d'oxydoréduction (par exemple une liaison disulfide, clivée en milieu réducteur),
   - aux enzymes (par exemple une liaison ester, clivée par les estérases endogènes) ; ou encore
   - aux radiations lumineuses (porteuses de groupements photosensibles par exemple) ;
- p est un entier égal à 0 ou à 1 ;
- Y est un groupe hydrocarboné ramifié, pouvant comprendre de 1 à 20 atomes de carbone, préférentiellement de 1 à 12, et/ou un ou plusieurs hétéroatomes, et pouvant être couplé de manière covalente éventuellement au groupe W₂ ou AA ou E ou R d'une part, et au moins à deux groupes Y et/ou Z d'autre part ;
- q est un entier compris entre 0 et 8, de préférence entre 0 et 3 ;
- Z représente un acide aminé basique ou la sérine ;
- r est un entier compris entre 1 et 16, de préférence entre 1 et 8, étant entendu que si q est égal à 1 alors r est au moins égal à 2 et que si r est supérieur à 1, alors les groupements Z peuvent être identiques ou différents;
- s est un entier égal à 1 ou 2 ;
et ses sels d'addition physiologiquement acceptables

Dans ce qui précède et ce qui suit, sauf indication contraire, on entend par "hétéroatome", un atome choisi parmi l'azote, l'oxygène, le soufre et les halogènes comme par exemple le brome, l'iode, le chlore et le fluor.

Concernant W₁ et W₂ tels que décrits précédemment (non compris dans l'invention revendiquée), lorsque identiques ou différents, ils représentent un groupe hydrocarboné, linéaire ou ramifié, pouvant comprendre de 1 à 15 atomes de carbone, préférentiellement de 1 à 6, comprenant un ou plusieurs hétéroatomes, le ou les hétéroatomes peuvent être choisis parmi l'azote, l'oxygène, le soufre et les halogènes comme par exemple le brome, l'iode, le chlore et le fluor, préférentiellement parmi l'azote, le soufre et les halogènes comme par exemple le brome, l'iode, le chlore et le fluor.

De même, on entend par "reste d'un acide aminé", le groupe d'atomes qui subsiste de cet acide aminé lorsque celui-ci est lié de manière covalente, d'une part au groupement E ou R, et d'autre part à un ou plusieurs groupes W₁ ou Y ou Z.

De même encore on entend par "groupe hydrocarboné" tout groupe composé d'un ou plusieurs atomes de carbone, éventuellement lié(s) à un ou plusieurs atome(s) d'hydrogène.

Dans la formule (I) décrite ci-dessus (non compris dans l'invention revendiquée), R répond de préférence à la formule (II) : 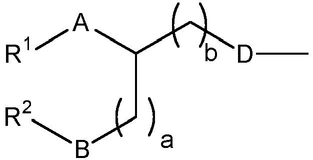 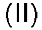 dans laquelle :
- R¹ et R², identiques ou différents, représentent un groupe hydrocarboné, linéaire, ramifié et/ou cyclique, saturé ou insaturé, comprenant de 6 à 24 atomes de carbone, préférentiellement de 10 à 18.
- A et B, identiques ou différents, représentent un groupement -C(O)-O- ; -O-C(O)- ; -CO-NH- ; -NH-CO- ; -NH- ou -O-.
- a est un entier compris entre 1 et 6, de préférence a est un entier égal à 1 ou 2.
- b est un entier compris entre 0 et 6, de préférence b est un entier égal à 0 ou 1.
- D représente un groupement -NH-, -CO-, -O- ou -S-.

Parmi les structures correspondant à la formule (II) décrite ci-dessus (non compris dans l'invention revendiquée), R répond préférentiellement à la formule (III) (Exemple I.1 ci-dessous) : 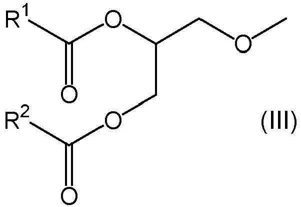 ou bien encore à la formule (IV) (Exemple I.8 ci-dessous) : 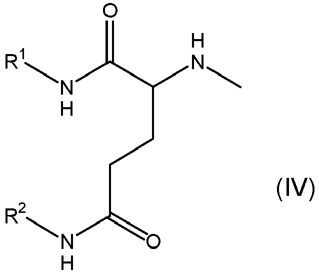 dans lesquelles R¹ et R² ont la même signification que précédemment.

Dans les formules (III) et (IV) décrites ci-dessus (non compris dans l'invention revendiquée), R¹ et R² représentent de préférence, une chaîne alkyle, alcényle ou alcynyle en C₁₂ à C₁₈.

Dans la formule (I) décrite ci-dessus (non compris dans l'invention revendiquée), E peut être absent ou E peut servir de bras espaceur et répond alors à la formule (V) : -G₁-X₁-G₁-, dans laquelle X₁ représente un groupe alkylène formant pont et pouvant comprendre de 1 à 8 atomes de carbone, préférentiellement de 1 à 4, tandis que G₁ peut représenter un groupe -CO- ou -NH-.

Parmi les composés décrits ci-dessus (non compris dans l'invention revendiquée), E répond de préférence à la formule CO-X₁-CO dans laquelle X₁ à la même signification que précédemment et est lié par une liaison amide à la région lipophile R d'une part, et soit au reste AA, soit directement au groupe W₁ ou Y ou Z d'autre part.

Selon l'invention, l'acide aminé, dont le reste est symbolisé par AA dans la formule (I), peut être, de préférence, choisi parmi les vingt acides aminés qui entrent dans la constitution des protéines, à savoir l'acide aspartique, l'acide glutamique, l'alanine, l'arginine, l'asparagine, la cystéine, la glutamine, la glycine, l'histidine, l'isoleucine, la leucine, la lysine, la méthionine, la phénylalanine, la proline, la sérine, la thréonine, la tyrosine, le tryptophane et la valine. En particulier, AA peut être choisi parmi l'acide aspartique, l'acide glutamique, l'isoleucine, la leucine, la lysine et la phénylalanine, l'acide aspartique (exemple I.14), l'acide glutamique (exemple I.15) et la lysine (exemple I.12) étant particulièrement préférés. Toutefois, cet acide aminé peut également être choisi parmi des acides aminés plus rares comme par exemple, la β-aianine, l'acide γ-aminobutyrique, l'acide α-aminoadipique, l'hydroxyproline, l'hydroxylysine, la phénylsérine, l'acide α,ε-diaminopimélique, l'ornithine et tous autres acides aminés modifiés, tout acide aminé étant susceptible de convenir puisqu'il comporte, par définition, deux groupes fonctionnels, l'un acide carboxylique, l'autre amine, autorisant sa liaison covalente, d'une part, au bras espaceur E ou à R, d'autre part, à au moins un groupe W₁ ou Y ou Z. Le choix de l'acide aminé dépend notamment de la valeur que l'on souhaite donner à s dans la formule (I), dans la mesure où il doit comporter au moins trois groupements fonctionnels pour que s puisse être égal à 2, alors qu'il suffit qu'il ne comporte que deux groupements fonctionnels pour avoir s égal à 1. Conformément à l'invention, on préfère que AA soit le reste d'un acide aminé appartenant à la série L. Toutefois, il est également possible que AA soit le reste d'un acide aminé de la série D.

Selon l'invention, dans la formule (I), W₁ répond à la formule (VI) : -G₂-X₂- et W₂ répond à la formule (VII) : -X₃-G₃- dans lesquelles X₂ et X₃, identiques ou différents, représentent un groupe alkylène, linéaire ou ramifié, formant pont et comprenant de 1 à 8 atomes de carbone, préférentiellement de 1 à 4, tandis que G₂ et G₃, identiques ou différents, représentent un groupe -CO-, -NH- ou -O-. W₁ forme une liaison covalente avec un groupe AA, E ou R d'une part, et avec le groupe L d'autre part, alors que W₂ forme une liaison covalente avec un groupe L d'une part, et avec un groupe Y ou Z d'autre part.

Selon l'invention, dans la formule (I), L représente un groupement ester (-CO-O-), disulfide (-S-S-), éther de vinyle (-O-C=C-), acylhydrazone (-CO-NR-N=CR'R"), les groupements esters (exemple I.1) et disulfides (exemple I.6) étant particulièrement préférés. Ces groupements forment une liaison covalente avec les groupements W₁ d'une part et W₂ d'autre part.

Selon l'invention, dans la formule (I), Y répond à la formule (VIII) : -CO-X₄-NH-X₅-N-[X₆-NH]₂- ou (IX): -NH-X₅-N-[X₆-NH]₂-, dans lesquelles X₄, X₅ et X₆, identiques ou différents, représentent un groupe alkylène, linéaire ou ramifié, formant pont et comprenant de 1 à 8 atomes de carbone, préférentiellement de 1 à 4. Dans les formules (VIII) et (IX), X₄ représente, de préférence, un méthylène, tandis que X₅ et X₆ peuvent représenter de préférence, un groupe alkylène formant pont et comprenant de 1 à 4 atomes de carbone et, mieux encore, 2 atomes de carbone. Y possède donc trois groupements fonctionnels lui permettant de former une liaison covalente avec éventuellement W₂ ou AA ou E ou R d'une part, et au moins deux groupes Y et/ou Z d'autre part.

Selon l'invention, dans la formule (I), Z représente un acide aminé basique choisi de préférence parmi la lysine (exemple I.1), l'ornithine (exemple I.6), l'arginine (exemple I.7), l'histidine (exemple I.4), ou la sérine. L'acide aminé Z possède une fonction acide carboxylique qui autorise sa liaison covalente avec éventuellement Y ou W₂ ou AA ou E ou R d'une part, et une ou deux fonctions basiques réactives (amine, alcool...) permettant de former éventuellement une liaison covalente avec un ou deux autres groupes Z d'autre part. Conformément à l'invention, on préfère que l'acide aminé basique appartienne à la série L. Toutefois, il est également possible qu'il appartienne à la série D.

Selon l'invention le composé amphipathique cationique de formule (I) peut être en solution sous la forme de sel ; le contre ion peut alors être un anion organique ou inorganique acceptable d'un point de vue physiologique, avantageusement choisi parmi des anions organiques tels que CF₃COO⁻ et CH₃COO⁻ ou inorganiques tels que Br, CI⁻, I⁻ et F⁻.

Parmi les composés de formule (I) convenant aux fins de l'invention, on peut citer à titre non-exhaustif les composés dont la formule est la suivante :

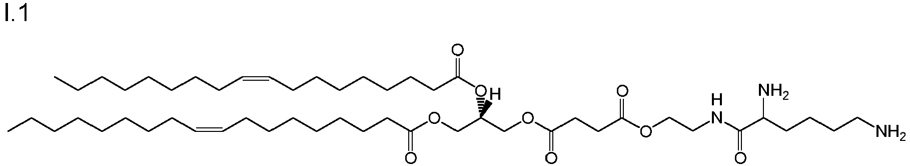

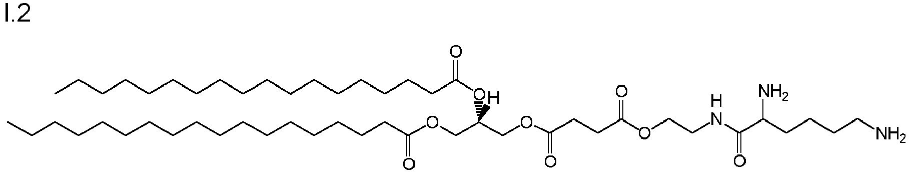

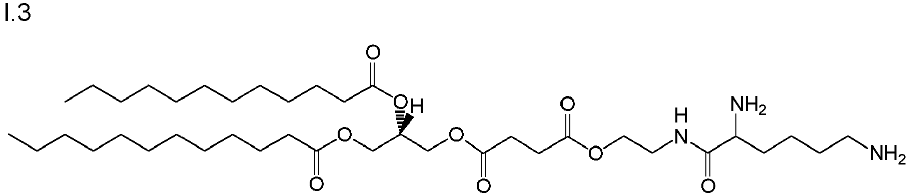

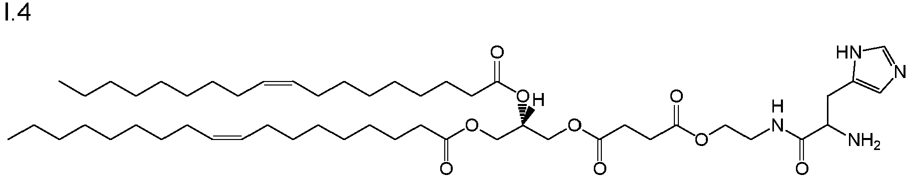

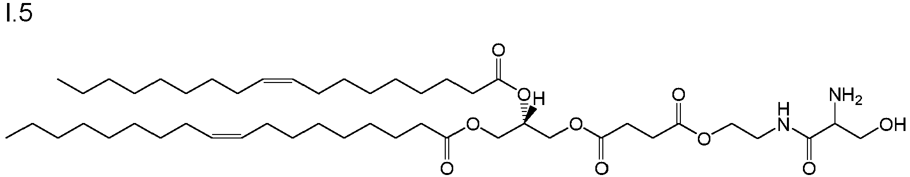

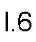

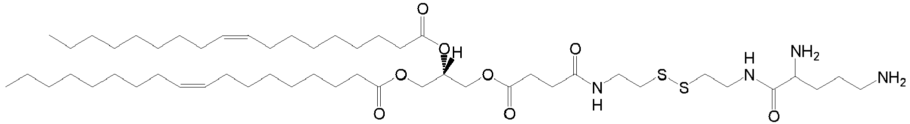

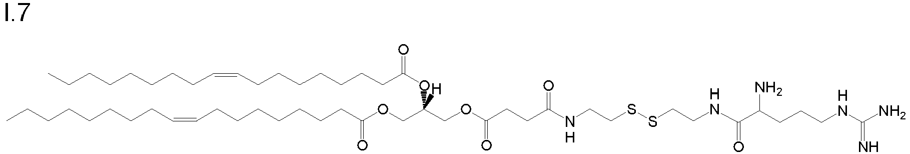

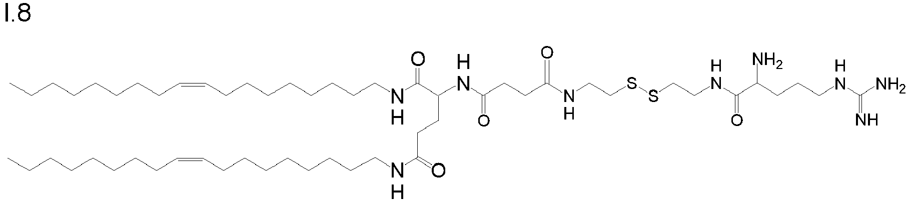

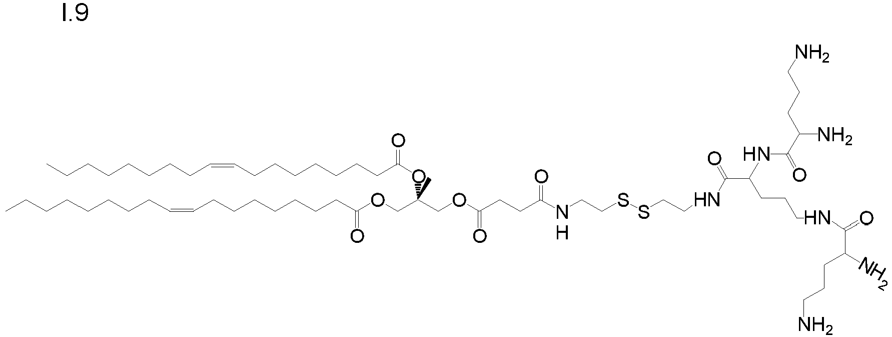

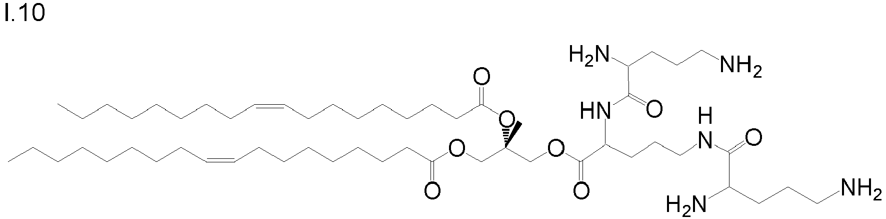

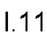

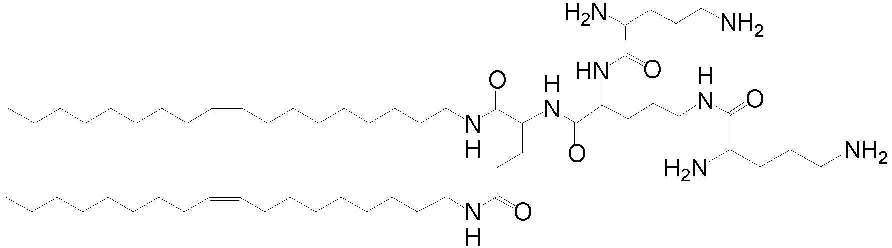

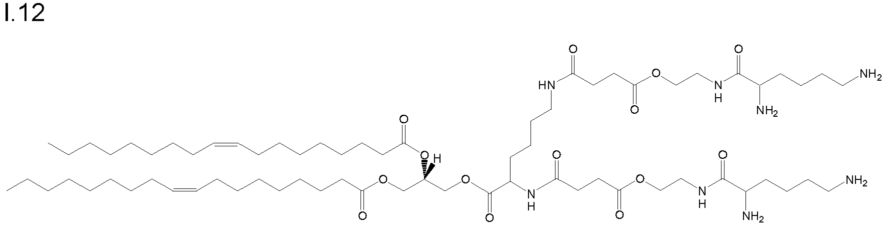

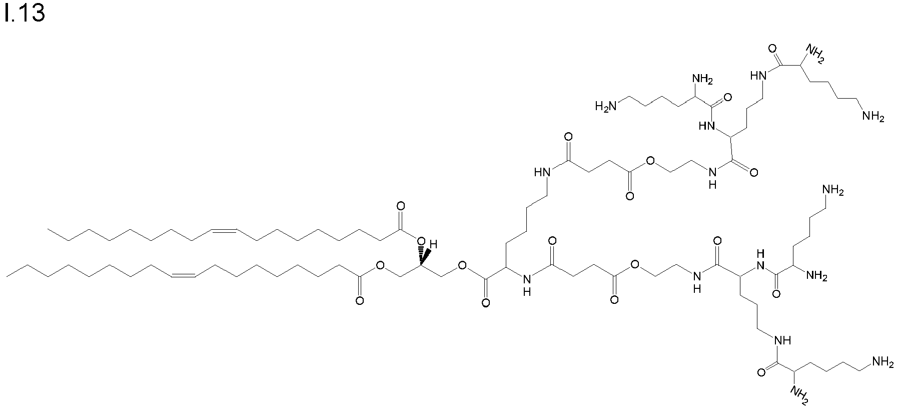

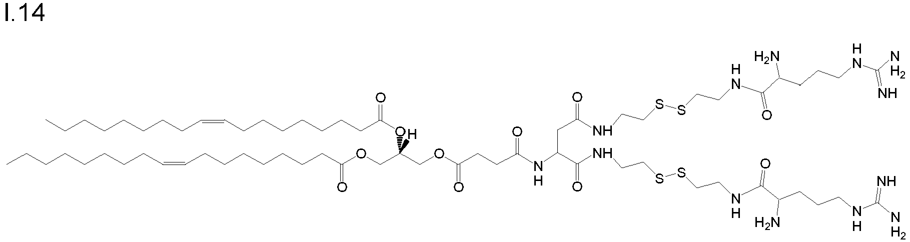

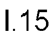

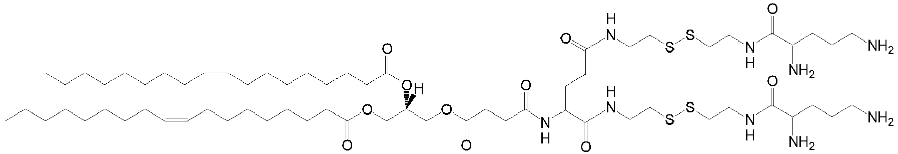

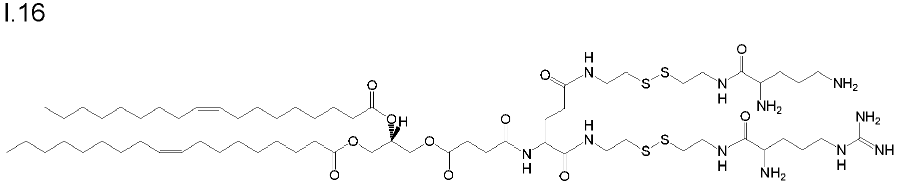

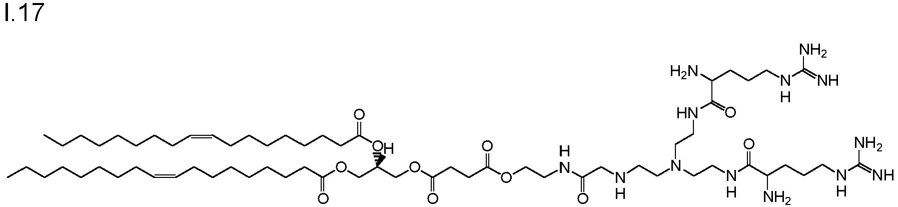

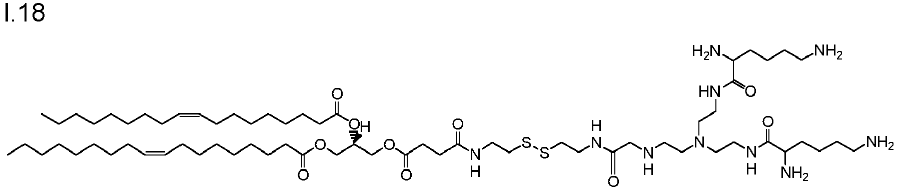

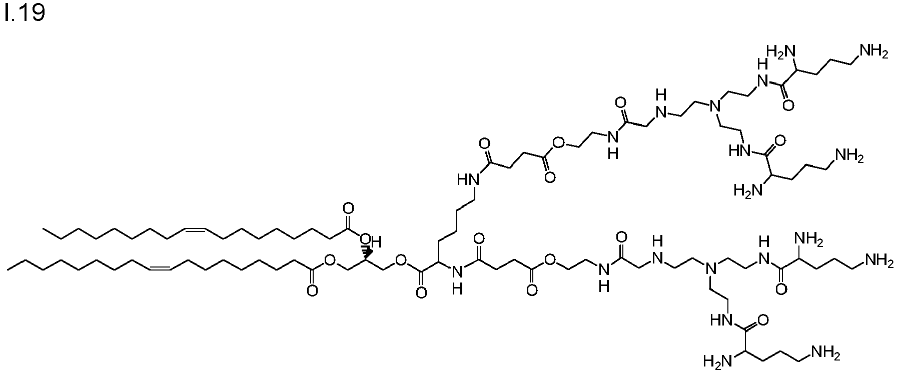

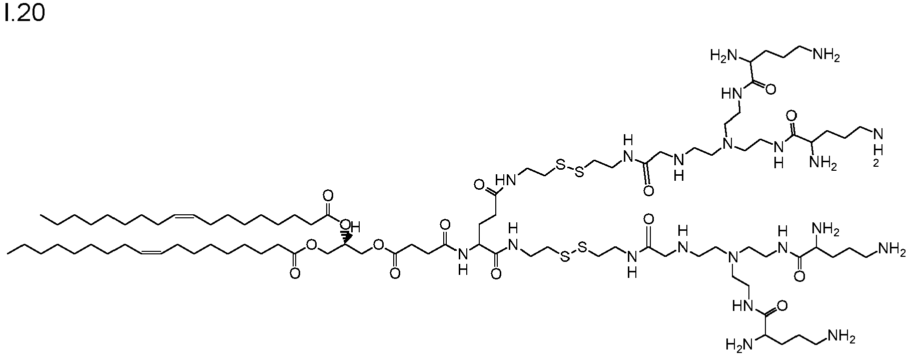

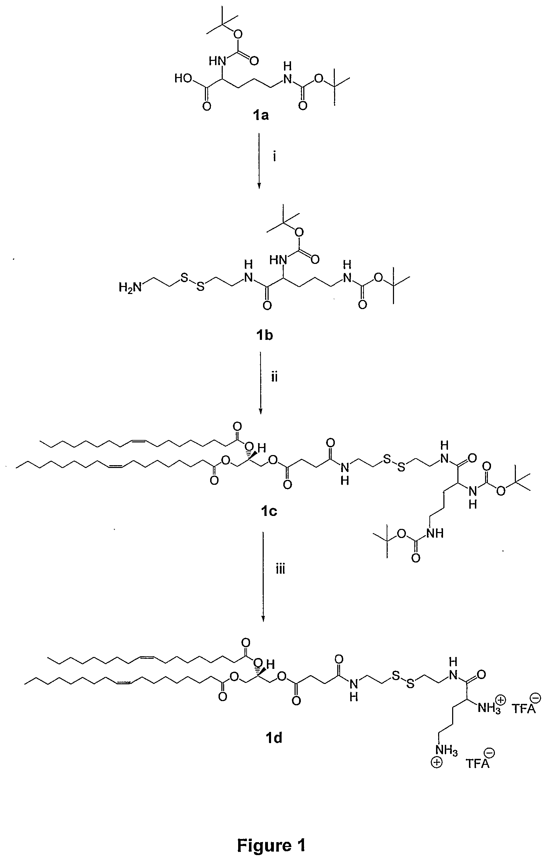

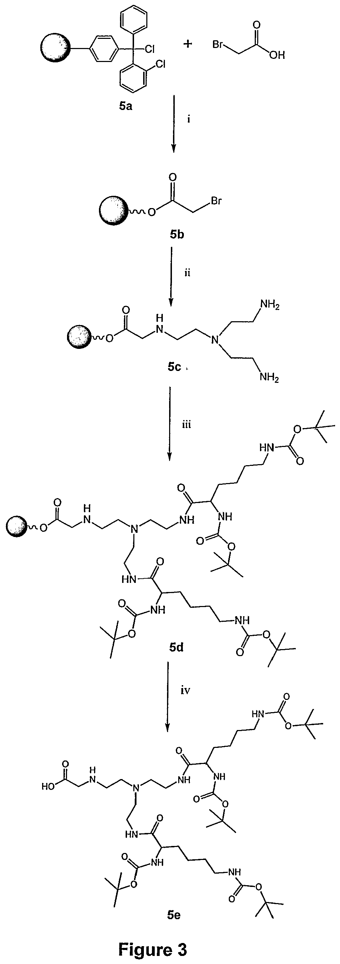

Les composés selon l'invention peuvent être préparés à partir de matériels de départ facilement accessibles dans le commerce, en utilisant des méthodes de synthèse et de purification bien connues par l'homme du métier. Les schémas réactionnels pour la préparation de certains composés préférés de la présente invention sont illustrés dans les figures 1 à 3 et les méthodes de préparation de ces composés sont décrites en détails dans les exemples ci-dessous. Par ailleurs, une méthode de synthèse sur support solide (Byk G. et col. (1997) Tetrahedron Lett. 38 (18), 3219-3222) a été employée pour la préparation de certains polyacides aminés basiques comme intermédiaire de synthèse pour la préparation de composés de l'invention. Cette méthode est décrite en détail dans un des exemples ci-dessous et le schéma réactionnel est illustré dans la figure 3.

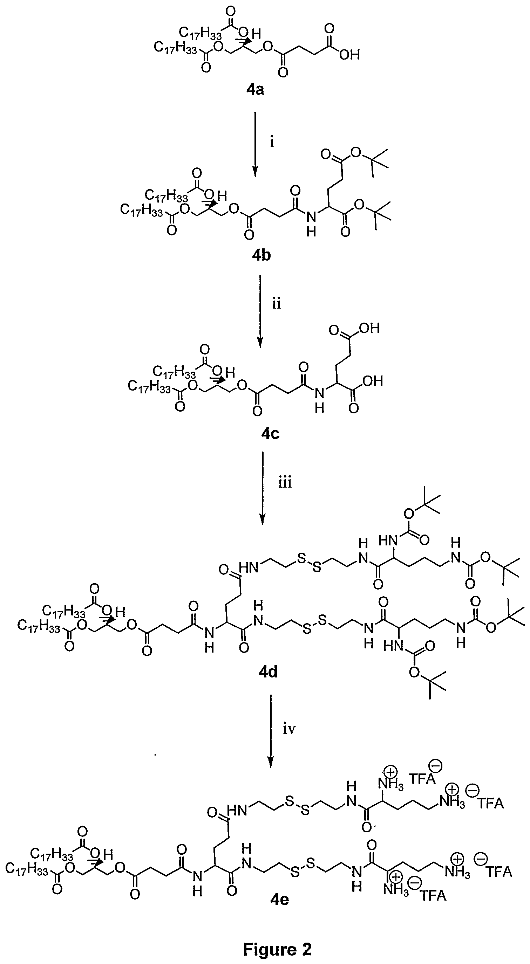

Les composés convenant aux fins de l'invention sont obtenus sous forme de sels qui peuvent être préparés par des techniques standard, comme illustré par exemple dans les schémas réactionnels des figures 1 et 2. Dans ces exemples, l'étape de formation de sels de trifluoroacétate correspond également à une étape de déprotection des fonctions amines et/ou guanidines des acides aminés basiques portés par les composés de l'invention.

Un autre objet de l'invention concerne des compositions comprenant au moins un composé répondant à la formule (I), particulièrement des compositions cosmétiques et/ou pharmaceutique, ou encore des réactifs de laboratoire.

Encore un autre objet de l'invention concerne une composition comprenant un composé répondant à la formule (I) telle que définie précédemment, encore appelé agent de transfert, et au moins un acide nucléique ou un polynucléotide. Préférentiellement, l'agent de transfert et l'acide nucléique peuvent être présents en quantité telle que le rapport de charges positives de l'agent sur les charges négatives de l'acide nucléique peut être compris entre 0,1 et 50, préférentiellement entre 0,5 et 20. Ce rapport peut être ajusté aisément par l'homme du métier en fonction de l'agent utilisé, de l'acide nucléique et du type de cellules à transfecter. Avantageusement, selon l'invention, la composition peut comprendre une quantité d'agent de transfert comprise entre 1 et 12 nanomoles par µg d'acide nucléique, et de manière préférée entre 1 et 9 nanomoles d'agent de transfert par µg d'acide nucléique.

Aux fins de la présente invention, l'acide nucléique peut être un acide désoxyribonucléique (ADN) ou un acide ribonucléotide (ARN) ou un acide nucléique modifié tels qu'un acide nucléique peptidique («Peptide Nucleic Acids », PNA), des oligonucléotides morpholinos ou des aptamères. Son origine n'importe pas : naturelle ou artificielle. Il peut être d'origine animale, humaine, végétale, bactérienne ou virale. Sa fonction à titre d'agent thérapeutique peut être un gène codant pour un polypeptide et/ou une protéine d'intérêt dans une cellule hôte ou peut être une fonction antisens venant contrôler l'expression d'un gène, sa transcription en ARN ou sa traduction en protéine. Il peut aussi agir en tant que ribozyme ou ARN interférents (ARNsi ou ARNsh ou ARNmi) avec l'expression d'un gène.

Sous un aspect particulier l'acide nucléique code de manière opérante pour un polypeptide d'intérêt pharmaceutique qui, lors de son expression dans la cellule hôte, permet de pallier un disfonctionnement de l'organisme récepteur. Une composition selon l'invention est par conséquent utile en recherche et développement *in vitro* et *in vivo* ou en thérapie génique *in vivo* et ex *vivo.* L'acide nucléique peut aussi coder de manière opérante pour un polypeptide capable de générer une réponse immune à son encontre chez les humains ou les animaux ou d'induire une réponse immune. Par conséquent, une composition selon l'invention trouve une application particulière dans la thérapie génique, le domaine des vaccins, et de l'immunothérapie, notamment pour traiter ou prévenir des cancers ou des infections bactériennes ou virales.

Pour usage dans les domaines de la thérapie génique, des vaccins et de l'immunothérapie, l'acide nucléique est avantageusement de l'ADN et comprend de préférence une cassette d'expression constituée d'une ou plusieurs séquences d'ADN codant le polypeptide d'intérêt sous contrôle d'un ou plusieurs promoteurs et d'un terminateur transcriptionnel actifs dans les cellules cibles. Il peut être aussi un ARN de type ARNsi ou oligonucléotides antisense.

Un autre objet de l'invention concerne une composition comprenant un agent de transfert répondant à la formule (I) telle que définie précédemment, et au moins un polypeptide ou une protéine. Préférentiellement, l'agent de transfert et le polypeptide ou la protéine peuvent être présents en quantité telle que la quantité d'agent de transfert (composé de Formule (I)) peut être comprise entre 1 et 10 nanomoles d'agent de transfert selon l'invention par µg de polypeptide, et de manière préférée entre 1 et 3 nanomoles d'agent par µg de polypeptide. Aux fins de la présente invention, le polypeptide peut être un peptide ou une protéine d'intérêt pharmaceutique. La délivrance de polypeptides dans les cellules représente une alternative à la thérapie génique pour développer de nouvelles approches thérapeutiques dirigées contre de nombreuses maladies telles que le cancer, les désordres inflammatoires et génétiques, les infections et les déficiences métaboliques comme le diabète. Parmi ces peptides et ces protéines d'intérêt, on peut citer par exemple les anticorps, les antigènes, les lymphokines, les interleukines, les facteurs de nécroses et d'apoptoses, les interférons, les facteurs de croissance, les activateurs de tissue plasminogène, le facteur VIII:c, l'érythropoïétine, l'insuline, la calcitocine, la thimidine kinase, etc.. Une telle composition selon l'invention ouvre également de nouveaux champs d'investigation en protéomique pour l'élucidation de mécanismes moléculaires complexes. Par exemple, la délivrance dans les cellules de protéines impliquées dans les phénomènes d'apoptose peut aider à l'élucidation des mécanismes de mort cellulaire programmée.

Un autre objet de l'invention concerne une composition comprenant un agent de transfert répondant à la formule (I) telle que définie précédemment, et au moins une molécule biologiquement active autre qu'un acide nucléique ou un polypeptide. Il peut s'agir d'un principe actif, d'un polysaccharide, d'un lipide, d'un peptoide, etc... Par exemple, les peptoides peuvent être utilisés avec succès comme analogue des peptides d'intérêt thérapeutique.

Les compositions peuvent en outre comporter des adjuvants capables de s'associer au composé répondant à la formule (I), aux molécules biologiquement actives ou au complexe agent de transfert / molécules biologiquement actives et d'en améliorer le pouvoir transfectant et la pharmacologie. Ainsi, les compositions selon l'invention peuvent comprendre comme adjuvant un ou plusieurs lipides neutres (zwitterioniques ou dépourvu de charges ioniques), anioniques ou cationiques. Préférentiellement, les lipides utilisés sont des lipides neutres à deux chaînes grasses, du cholestérol ou des dérivés du cholestérol. Ils peuvent être choisis plus particulièrement parmi la dioléoylphosphatidyléthanolamine (DOPE) (Farhood H. et col., Biochim Biophys. Acta (1985), 1235-1289), l'oléoylpalmitoylphosphatidyléthanolamine (POPE), les distéaroyl-, dipalmitoyl-, dimyristoyl-, dilauroylphosphatidyléthanolamines (DSPE, DPPE, DMPE, DLPE), ainsi que leurs dérivés N-méthylés un à trois fois (DOPC, DPPC, DMPC), les phosphatidylglycérols, les glycosyldiacylglycérols, les cérébrosides (tels que notamment les galactocérébrosides), les sphingolipides (tels que notamment les sphyngomyélines), les asialogangliosides (tels que notamment les asialoGM1 et GM2), ou encore les éthers lipides. Des lipides comportant une seule chaîne grasse peuvent aussi être utilisés, parmi lesquels les lysophosphatides, les lysophosphatidylcholines, les lysophosphatidyléthanolamines, les lysophosphatidylglycérols, les lysophosphatidylsérines ou encore les acides lysophosphatidique. Ces différents lipides peuvent être naturels ou synthétiques.

Les adjuvants pouvant entrer dans la composition selon l'invention peuvent aussi être un ou plusieurs polymères, naturels ou synthétiques, co-polymères et/ou dendrimères. Ces polymères peuvent être cationiques tels que les polyamines, parmi lesquelles la polyéthylènimine, la polylysine, la polyornithine, ou encore le polybrène et le chitosane. Les polymères peuvent également être anioniques tels que l'acide polyglutamique, l'acide polypropylacrylique, l'acide hyaluronique et l'acide polylactique-co-glycolique (PGLA), ou neutres tels que le polyéthylène glycol (PEG) ou encore certains polysaccharides tels que les galactomannanes.

Les compositions selon l'invention peuvent aussi comprendre comme adjuvant des nanoparticules, notamment des particules magnétiques, des particules à base de composés organiques ou inorganiques. Les adjuvants peuvent également être des polypeptides, des protéines, des oses, du glycérol, des cyclodextrines, des histones, l'acide désoxycholique et tout autre « activateur » (« enhancer ») qui améliore l'efficacité de livraison et la pharmacologie.

Les compositions peuvent aussi comporter des adjuvants capables de cibler spécifiquement un déterminant à la surface et/ou à l'intérieur des cellules. Ces éléments de ciblage peuvent être attachés de manière covalente ou non covalente au composé répondant à la formule (I) ou à toutes autres molécules contenues dans la composition comprenant le composé de formule (I). Ces éléments de ciblage peuvent être des ligands de récepteurs exprimés à la surface des cellules ciblés, par exemple un sucre, un folate, la transferrine, l'insuline, une hormone, un peptide, un anticorps, un métabolite, des vitamines ou toute autre molécule pouvant reconnaître un récepteur extracellulaire. Ils peuvent aussi être un élément de vectorisation intracellulaire pour cibler des compartiments spécifiques tels que les mitochondries, le noyau ou le cytoplasme, comme par exemple un signal de localisation nucléaire ou mitochondriale. D'une manière générale, l'élément de ciblage peut-être un sucre, un peptide, une protéine, un anticorps, un fragment d'anticorps, un ligand ou un fragment de ligands. L'adjuvant peut aussi être un fluorophore tel que la rhodamine, la fluorescéine ou la biotine.

Les compositions selon l'invention peuvent aussi comprendre des virus, par exemple des lentivirus, des rétrovirus, des adénovirus, le virus de l'herpès, des baculovirus, et/ou des organismes unicellulaires, par exemple des bactéries, des levures, des champignons ou des parasites.

Préférentiellement, les compositions selon l'invention ont un ratio molaire adjuvant / agent de transfert compris entre 0 et 20, et plus préférentiellement entre 0,5 et 3.

L'invention s'étend également à toute composition telle que définie ci-dessus et comprenant en outre un ou plusieurs autres agents connus pour transfecter les acides nucléiques, les polypeptides ou tout autre molécule biologiquement active.

Un autre objet de la présente invention concerne l'utilisation d'un agent de transfert tel que défini précédemment pour le transfert d'acides nucléiques, de polypeptides ou de tout autre molécule biologiquement active dans les cellules. Cette utilisation peut être dans tout domaine où le transport d'une molécule d'intérêt biologique est nécessaire. On pense en particulier aux domaines cosmétique et/ou pharmaceutique, ou encore des réactifs de laboratoire.

L'invention concerne également l'utilisation d'un composé de Formule (I) pour la préparation d'une composition destinée au transfert d'acides nucléiques, de polypeptides ou de tout autre molécule biologiquement active dans les cellules.

L'invention concerne en outre l'utilisation d'une composition comprenant au moins un composé de Formule (I) pour le transfert d'acides nucléiques, de polypeptides ou de tout autre molécule biologiquement active dans les cellules.

Les compositions comprenant l'agent de transfert selon l'invention peuvent être formulées en vue d'administration par voie topique, cutanée, orale, rectale, vaginale, parentérale, intranasale, intraveineuse, intramusculaire, sous-cutanée, intraoculaire, transdermique, intratrachéale, intrapéritonéale, etc...

De préférence, les compositions pharmaceutiques de l'invention contiennent un véhicule pharmaceutiquement acceptable pour une formulation injectable, notamment pour une injection directe au niveau de l'organe désiré ou pour une administration par voie topique. Il peut s'agir en particulier de solutions stériles, isotoniques, ou de compositions sèches, notamment lyophilisées, qui, par addition selon le cas d'eau stérilisée ou de sérum physiologique, permettent la constitution de solutions injectables. Les doses d'acide nucléique, de polypeptide ou de tout autre molécule biologiquement active, utilisés pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée. En ce qui concerne plus particulièrement le mode d'administration, il peut s'agir soit d'une injection directe, dans les tissus ou les voies circulatoires, soit d'un traitement de cellules en culture suivi de leur réimplantation par injection ou greffe.

L'invention se rapporte en outre à une méthode de transfert d'une molécule d'intérêt biologique dans des cellules *in vitro* ou ex *vivo,* comprenant les étapes suivantes :
1. la mise en contact de la molécule d'intérêt biologique avec un agent de transfert répondant à la formule (I) tel que défini ci-dessus, ou avec une composition telle que définie précédemment pour former un complexe molécule active / agent de transfert,
2. la mise en contact des cellules avec le complexe formé en 1.

Selon l'invention, à l'étape 2, l'ordre d'addition des cellules et/ou des complexes est indifférent.

Préférentiellement, cette méthode est utilisée *in vitro* et/ou dans des expériences réalisées avec des cellules préalablement isolées.

Dans le cas d'une composition comprenant un ou plusieurs autres agents de transfection et/ou un ou plusieurs adjuvants, la méthode selon l'invention peut en outre comprendre une ou plusieurs étapes de mise en contact de l'agent de transfert selon l'invention avec différents agents de transfection et/ou avec le ou les adjuvants. Avantageusement selon l'invention, l'étape 1 peut être précédée d'une étape de mise en contact du composé de formule (I) avec d'autres agents de transfection et/ou avec le ou les adjuvants.

Les complexes agent de transfert selon l'invention / molécule biologiquement active sont formés en mélangeant deux solutions contenant l'une la composition à base de l'agent de transfert selon l'invention, en présence ou en absence d'un ou plusieurs adjuvant et/ou d'un ou plusieurs autres agents de transfection, et l'autre la molécule biologiquement active à délivrer en présence ou en absence d'un ou plusieurs adjuvant et/ou d'un ou plusieurs autres agents de transfection. Les complexes se forment en quelques secondes et peuvent être chargés négativement, positivement, ou être neutres, en fonction de la quantité de lipide ajoutée à la molécule à transporter.

La mise en contact des cellules avec le complexe peut être réalisée par incubation des cellules avec ledit complexe (utilisation *in vitro* ou *ex vivo*). L'incubation est réalisée de préférence en présence par exemple de 0,01 à 1000 µg de molécule d'intérêt biologique pour 10⁶ cellules. Pour une administration *in vivo,* des doses de molécules actives allant de 0,01 à 10 mg peuvent être utilisées.

Les agents de transfert selon l'invention sont particulièrement intéressants pour leur utilisation dans le transfert de molécules biologiquement actives dans les cellules primaires ou dans des lignées établies. Il peut s'agir de cellules eucaryotes telles que endothéliales, épithéliales, fibroblastiques, hépatiques, hématopoïétiques (lymphocytes, monocytes, macrophages, dendritiques, etc...), musculaires, nerveuses (neurones, cellules gliales, astrocytes), etc... Il peut également s'agir de cellules procaryotes (bactéries) et végétales, de cellules d'insectes, des levures ou de parasites. Elles peuvent se présenter sous forme différenciées ou pluripotentes.

L'invention a aussi pour objet des kits de transfert de matériel biologiques comprenant au moins un composé de Formule (I) ou au moins une composition comprenant au moins un composé de Formule (I), et éventuellement d'autres solutions utiles à la réalisation d'un transfert de matériel biologique.

Les intéressantes propriétés des composés selon l'invention permettent d'envisager de nombreuses autres utilisations comme en particulier l'utilisation comme adjuvant dans une composition, particulièrement dans un vaccin.

Les définitions suivantes sont fournies pour faciliter la compréhension de certains termes utilisés fréquemment dans cet exposé :
- Par "molécule biologiquement active " ou "molécule active" ou "agent biologiquement actif " ou "molécule d'intérêt biologique " ou "principe actif", on entend toute molécule ou macromolécule ayant une activité spécifique dans les cellules et qui trouve des applications dans de nombreux domaines allant de la biologie cellulaire à la médecine. Il peut s'agir d'acides nucléiques (ADN, ARN...), de polypeptides, de protéines, de principes actifs, de polysaccharides, de peptoides, etc...
- Par " polypeptide " on entend tout enchaînement d'acides aminés quelle que soit sa taille. Ainsi, ce terme recouvre notamment les notions de peptides et de protéines.

Outre les dispositions qui précèdent, la présente invention comprend également d'autres caractéristiques et avantages qui ressortiront des exemples et figures qui suivent, et qui doivent être considérés comme illustrant l'invention sans en limiter la portée.

Notamment, la demanderesse propose à titre non limitatif divers protocoles opératoires ainsi que des intermédiaires réactionnels susceptibles d'être mis en œuvre pour préparer les agents de transfert selon l'invention. Il est bien entendu à la portée de l'homme du métier de s'inspirer de ces protocoles ou produits intermédiaires pour mettre au point des procédés analogues en vue de conduire à ces mêmes composés.

### Figures :

Figure 1 : Schéma réactionnel pour la synthèse du 181GSCO **1d** (composé de Formule I.6) dans lequel à chaque étape les réactifs et conditions utilisés sont :
   i) Successivement
      a. N,N'-Dicyclohexylcarbodiimide (DCC), N-Hydroxysuccinimide (NHS), Dichlorométhane / N,N'-Diméthylformamide (CH₂Cl₂ / DMF), Température Ambiante (TA) (environ 25°C) ;
      b. Dichlorure de cystamine, Triéthylamine (TEA), DMF, TA ;
   ii) Successivement
      a. Dioleoylglycerosuccinate (DOGS), DCC, NHS, CH₂Cl₂/ DMF, TA ;
      b. TEA, CH₂Cl₂, TA ;
   iii) Acide trifluoroacétique (CF₃COOH), CH₂Cl₂, TA.
Figure 2 : Schéma réactionnel pour la synthèse du 181GSGlu(CO)₂ **4e** (composé de Formule I.15) dans lequel à chaque étape les réactifs et conditions utilisés sont :
   i) Successivement
      a. N,N'-Diisopropylcarbodiimide (DIC), Hydroxybenzotriazole (HOBt), CH₂Cl₂/ DMF, TA;
      b. L-Glutamate de di-*tert* butyl (H-Glu(OtBu)-OtBu), CH₂Cl₂, TA ;
   ii) CF₃COOH, CH₂Cl₂, TA;
   iii) Successivement
      a. DIC, HOBt, CH₂Cl₂/ DMF, TA ;
      b. Boc-Orn(Boc)-NH-(CH₂)₂-S-S-(CH₂)₂-NH₂ 1b, TEA, CH₂Cl₂, TA ;
   iv)CF₃COOH, CH₂Cl₂, TA.
Figure 3 : Schéma réactionnel pour la synthèse sur support solide de la bis-lysine **5e**, utilisé ensuite comme intermédiaire de synthèse pour la préparation du 181GSCL₂ **5f** (composé de Formule I.18) dans lequel à chaque étape les réactifs et conditions utilisés sont :
   i) Diisopropyléthylamine (DIPEA), CH₂Cl₂, TA ;
   ii) Tris-(2-aminoethyl)amine, CH₂Cl₂, TA ;
   iv)Nα,Nε-di-*tert*-butyloxycarbonyl-L-Lysine (Boc-Lys(Boc)-OH), DIC, HOBt, CH₂Cl₂, TA ;
   v) CF₃CH₂OH, CH₂Cl₂, TA.
Figure 4A: histogramme représentant l'activité de transfert (% de cellules transfectées) *in vitro* de la formulation lipidique 181GSCO (composé de Formule I.6) / DOPE pour le transport d'ADN (pCMV-EGFP) dans plusieurs lignées cellulaires (Vero, NiH3-T3, A549, PC-12).
Figure 4B : histogramme représentant l'activité de transfert *in vitro* de la formulation lipidique 181GSCO (composé de Formule I.6) / DOPE pour le transport d'ADN (pCMV-LacZ) dans les cellules Vero (quantité de β-galactosidase), en fonction de la quantité d'ADN (µg) et de la quantité de lipide formulé (0,5, 1, 2 µl).
Figure 5 : Histogramme représentant l'activité de transfert (intensité de fluorescence) *in vitro* de 181GSCR (composé de Formule I.7), formulé avec du DOPE ou du cholestérol, pour le transport d'ADN (pCMV-EGFP) dans les cellules NiH3-T3, en fonction de la quantité d'ADN et de la quantité de lipide formulé.
Figure 6 : Histogramme représentant l'activité de transfert *in vitro* de la formulation lipidique 181GSCO (composé de Formule I.6) formulée ou non avec différents ratio de DOPE dans les cellules Hela-GFP pour le transport d'ARN Si (anti-GFP) (% d'inhibition de l'expression de la GFP).
Figure 7. Histogramme représentant l'activité de transfert *in vitro* de la formulation lipidique 181GSCR (composé de Formule I.7) / DOPE dans les cellules Hela-GFP pour le transport d'ARN Si (anti-GFP) (% d'inhibition de l'expression de la GFP), en fonction de la concentration en ARN Si (en nM) et du temps d'incubation exprimé en heures.
Figure 8A, 8B, 8C : Photos représentant l'activité de transfert *in vitro* des formulations :
   - 181GSCO (composé de Formule I.6) / DOPE, pour le transport
      ∘ d'IgG de chèvre marqué à la fluorescéine dans des cellules NiH3-T3 (Fig. 8A),
      ∘ de R-Phycoerythrine dans des cellules Vero (Fig. 8B),
      ∘ de β-Galactosidase dans des cellules Hela, (Fig. 8C),
   - 181GSCR (composé de Formule I.7) / DOPE, pour le transport
      ∘ d'IgG de chèvre marqué à la fluorescéine dans des cellules NiH3-T3 (Fig. 8D), et
   - 181GluO2 (composé de Formule I.11) / DOPE pour le transport
      ∘ de R-Phycoerythrine dans des cellules Hela (Fig. 8E),

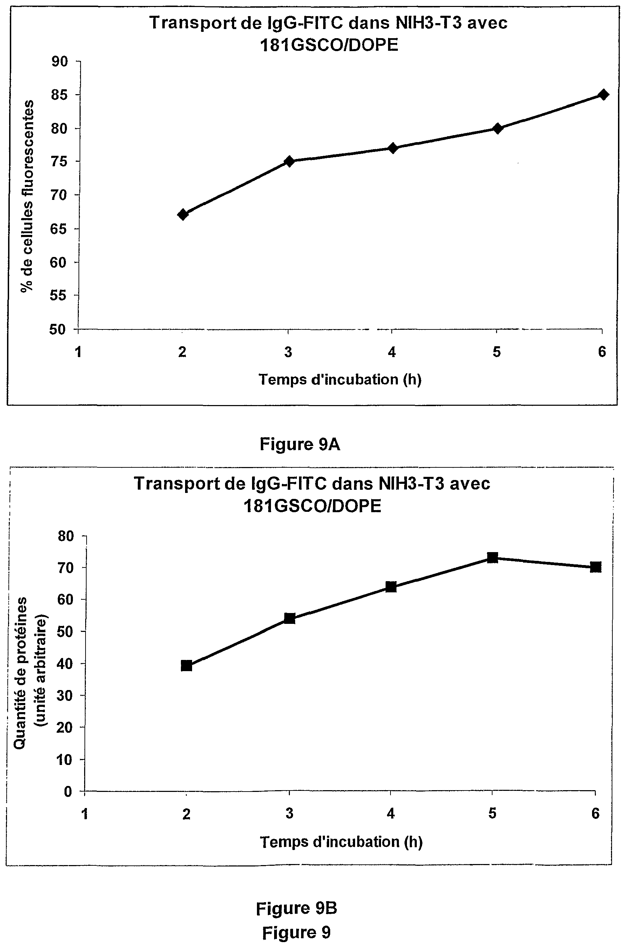

Figure 9A et B : Courbes représentant l'activité de transfert *in vitro* de la formulation 181GSCO (composé de Formule I.6) / DOPE dans les cellules NiH3-T3 pour le transport d'un anticorps IgG de chèvre marqué à la fluorescéine. L'activité de transfert est déterminée en % de cellules fluorescentes (Fig. 9A) et en quantité de protéines internalisées dans les cellules (Fig.9B) en fonction du temps d'incubation.

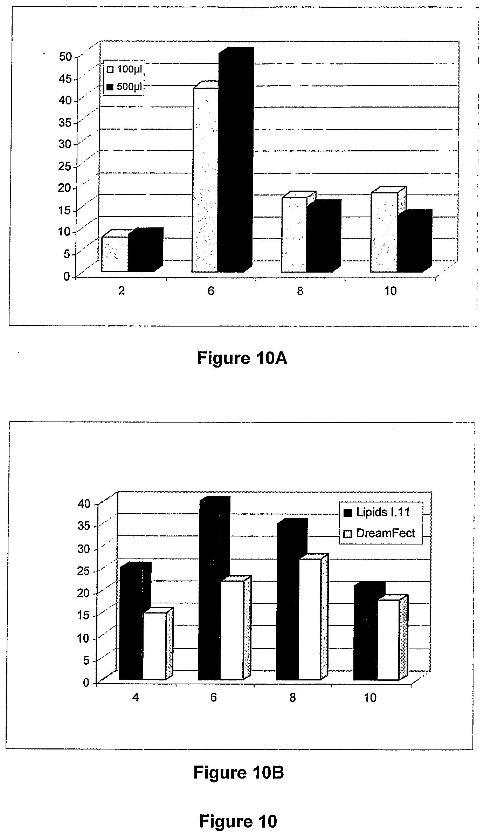

Figure 10A et B : Histogrammes représentant l'activité de transfert *in vitro* de la formulation lipidique 181GluO2 (composé de Formule I.11) / DOPE dans les cellules en suspension Jurkat pour le transport d'ADN (pCMV-EGFP), en fonction de la quantité de lipide formulé (µl). L'activité de transfert est déterminée en quantité de GFP exprimées dans les cellules (Fig. 10A), et en % de cellules fluorescentes (Fig. 10B).

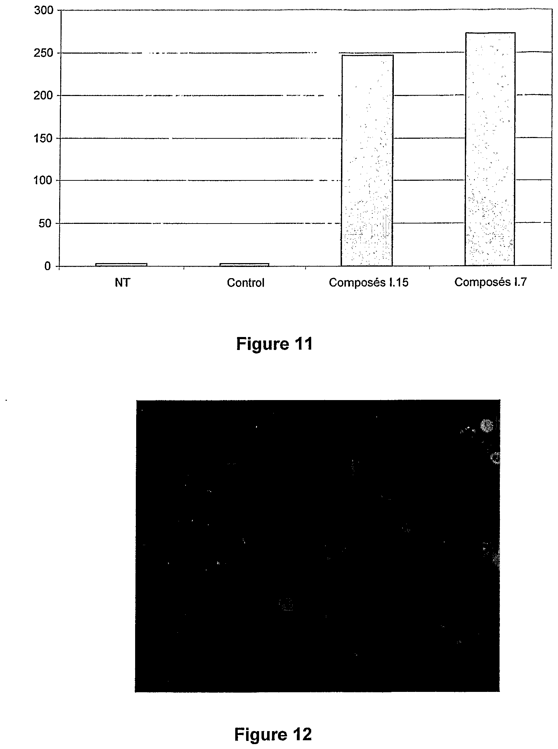

Figure 11 : Histogramme représentant l'activité de transfert (intensité de fluorescence) *in vitro* des lipides 181GSCR (composé de Formule I.7) et 181GSGlu(CO)₂ (composé de Formule I.15) formulé avec DOPE dans les cellules en suspension Jurkat pour la délivrance intracellulaire d'un ARN-Si fluorescent.
Figure 12 : Photo représentant l'activité de transfert *in vitro* de la formulation 181GSCO (composé de Formule I.6) / DOPE dans les cellules en suspension Jurkat pour le transport d'une protéine (BSA) marquée à la TRITC.

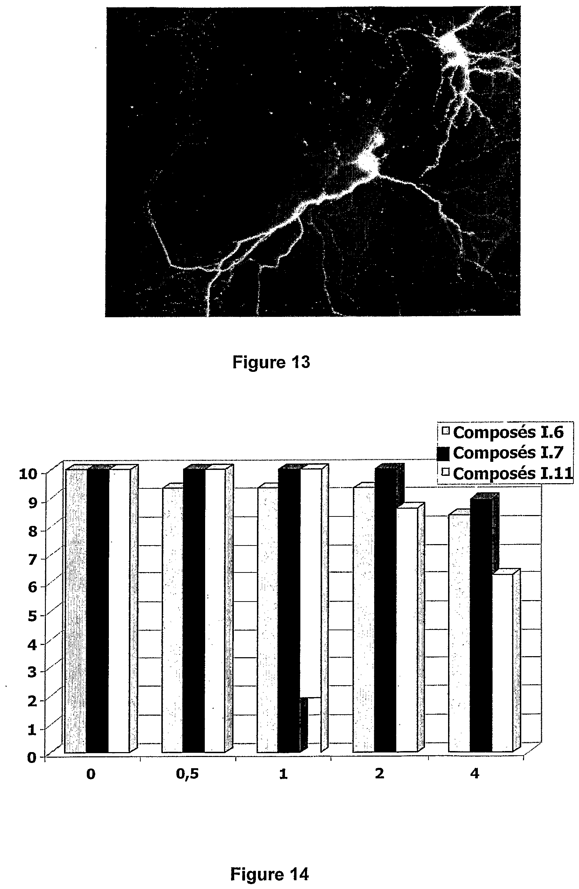

Figure 13 : Photo représentant l'activité de transfert *in vitro* de la formulation 181GluO2 (composé de Formule I.11)/ DOPE dans les neurones primaires pour le transport d'ADN (pCMV-EGFP).
Figure 14 : Histogramme représentant la quantité totale de protéines (µg) exprimées par les cellules Hela en fonction de la quantité de lipides I.6, I.7 et I.11 formulés avec du DOPE (µM). Les cellules ont été incubées 48 h avec ou sans lipides dans une plaque 96 puits et la quantité de protéines par puits a été déterminée par un test de Bradford.

### Exemples :

### B. SYNTHESES D'AGENTS DE TRANSFERT SELON L'INVENTION

### a) Matériel

La majorité des réactifs et des solvants proviennent de chez Merck (Darmstadt, Allemagne), VWR Prolabo (Briare, France), Sigma-Aldrich SARL (Saint Quentin Fallavier, France) et Fluka (Division de Sigma-Aldrich, Saint Quentin Fallavier, France). Les dérivés de glycérolipides (DOGS, DPGS, DMGS, DLGS) proviennent de chez Avanti Polar Lipids (Alabaster, AL, USA). Les acides aminés protégés (Boc-Orn(Boc)-OH, Boc-Lys(Boc)-OH, Boc-Arg(Boc)₂-OH, H-Glu(OtBu)-OtBu.HCl) proviennent de chez Bachem Biochimie SARL (Voisin-le-Bretonneux, France). Tous les solvants anhydres proviennent de chez Sigma-Aldrich et Fluka et utilisés tels quels.

### b) Méthodes

### • Techniques de chromatographie

Les chromatographies sur couches minces (CCM) sont réalisées sur des plaques d'aluminium 5 × 7,5 cm recouvertes de gel de silice 60 F₂₅₄ (Merck). Les composés sont révélés sous lumière UV (λ = 254 nm), à l'iode, par immersion dans un révélateur à la ninhydrine (0,2 % dans le butanol) suivi d'une étape de chauffage à 150°C pour les composés possédant une fonction amine primaire, ou par immersion dans un révélateur au cérium / molybdate (H₂O / H₂SO₄ concentré / (NH₄)₆Mo₇O₂₄.4H₂O / Ce(SO₄)₂.3H₂O : 90 / 10 / 15 / 1) suivi d'une étape de chauffage à 110°C pour les composés soufrés.

Les produits de synthèse sont purifiés sur colonnes de chromatographie sur silice. Les séparations chromatographiques flash sont effectuées sur gel de silice 60 (230-400 mesh ASTM) (Merck).

### • Spectrométrie de masse

### Préparation des échantillons :

Les produits à analyser sont dissous (0,01 mg.mL⁻¹) dans un mélange méthanol/eau 50/50 (v/v) ou acétonitrile/eau 50/50 (v/v) et les solutions sont directement introduites (5µL.min⁻¹) dans la source électrospray par l'intermédiaire d'une pompe à seringue (Harvard Apparatus, Les Ulis, France).

### Appareillage :

Les spectres de masse sont réalisés sur un appareil Waters-Micromass (Manchester, U.K.) Q-TOF équipé d'une source d'ion électrospray assistée pneumatiquement (Z-Spray). L'azote est utilisé comme gaz de désolvatation et de nébulisation avec un débit de 250 et 50 L/h, respectivement. Les températures de la source et du gaz de désolvatation sont respectivement fixées à 80 et 150 °C. La tension du capillaire est de ± 180 V (± ESI). Pour les expériences de dissociation induite par collision (CID : Collision Induced Dissociation), l'argon est utilisé comme gaz de collision, à une pression de l'analyseur indiqué à 5 × 10⁻⁵ Torr et une énergie de collision réglée à 90 V.

Les mesures de masse exacte sont effectuées sur un appareil Waters-Micromass (Manchester, U.K.) LCT, équipé d'une source électrospray assistée pneumatiquement (Z-spray), et muni d'un nébulisateur additionnel (Lockspray) pour le composé de référence (Nal). L'azote est utilisé comme gaz de désolvatation et de nébulisation avec un débit de 500 et 20 L/h, respectivement. Les températures de la source et du gaz de désolvatation sont respectivement fixées à 80 et 120 °C. La tension du capillaire est de ± 3,0 kV et la tension de cône de ± 100 V (± ESI).

Les spectres sont accumulés à une vitesse de 3 secondes par scan pour une gamme de masse comprise entre 100 et 3500 uma. La résolution utilisée est de 9000 FWHM pour le Q-TOF et 3000 FWHM pour le LCT. L'acquisition des données et leur traitement sont réalisés avec le programme MassLynx V3.5.

### • Couplage LC/MS

Certains composés sont analysés par Chromatographie Liquide Haute Performance couplé à la Spectrométrie de Masse (LC/MS). La CLHP est réalisée avec un appareil Waters Alliance 2695, muni d'une colonne analytique Alltech Prevail^{™} C18 (Lexington, KY, USA). La détection est effectuée en sortie de colonne CLHP par un spectromètre de masse Waters-Micromass Q-TOF.

### c) Synthèses

### Exemple 1 : Préparation du DiOleoyl-Glycero-Succinyl-Cystamido-Ornithine (composé 1d : 181GSCO)

On obtient le 181GSCO, ou composé **1d,** de formule : 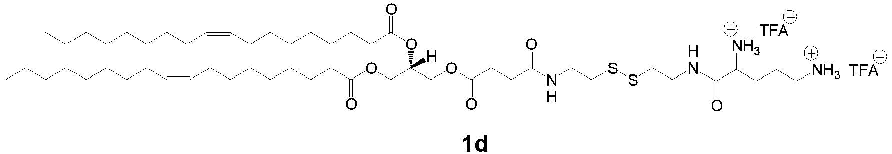 en trois étapes à partir de la N_{α},N_{ε}-di-Boc-ornithine **1a** (Figure 1)

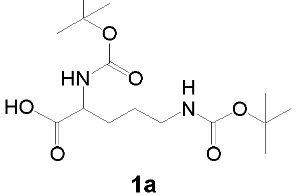

### 2.1 Etape 1 : préparation du Cystamido-di-Boc-Ornithine (1b)

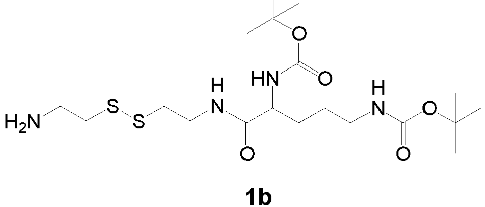

Dans un ballon de 100 mL sec, la N_{α},N_{ε}-di-Boc-ornithine **1a** (1,22 mmol ; 406 mg) est préalablement placée sous atmosphère inerte puis dissoute dans 20 mL de CH₂Cl₂ anhydre sous agitation. Le N,N'-dicyclohexylcarbodiimide (DCC) fraîchement recristallisé (1,83 mmol ; 378 mg) et en solution dans 5mL de CH₂Cl₂ anhydre, puis le N-Hydroxysuccinimide (NHS) (1,83 mmol ; 211 mg) en solution dans 5 mL de DMF anhydre, sont successivement additionnés dans le milieu réactionnel. La réaction est alors maintenue 2 h sous agitation et sous atmosphère inerte à température ambiante. Dans un ballon de 25 mL, le dichlorure de cystamine (6,1 mmol ; 1,37 g) est dissous dans 1 mL d'eau. A cette solution, est ajoutée de la triéthylamine (12,2 mmol ; 1,7 mL) sous agitation, puis 10 mL de DMF. Cette solution est agitée pendant 10 minutes à 50°C puis immédiatement ajoutée au milieu réactionnel. Après 24h d'agitation à température ambiante, un important précipité s'est formé dans le milieu réactionnel. La mixture est alors filtrée, le gâteau est lavé avec CH₂Cl₂ (2 × 20 mL), et les filtrats ainsi obtenus sont regroupés et évaporés sous vide primaire. Le produit de couplage est ensuite purifié par flash chromatographie sur gel de silice (élution : CH₂Cl₂/MeOH 95/5 puis 8/2 (v/v)). On isole ainsi 260 mg du composé **1b** soit un rendement de 46 %.

**CCM :** R_{f} **1b** = 0,6 (CH₂Cl₂ / MeOH 8/2 (v/v))

**ESI-MS** + : *m*/*z* mesuré à 467,1 [M+H]⁺, calculé à 467,2 pour C₁₉H₃₉N₄O₅S₂.

### 2.2 Etape 2 : préparation du DiOleoyl-Glycero-Succinyl-Cystamido-di-Boc-Ornithine (1c)

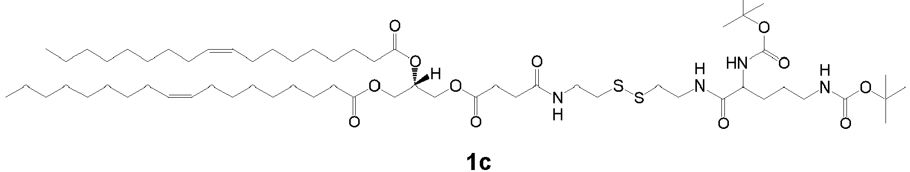

Dans un ballon de 100 mL sec, le 1,2-Dioleoyl-*sn*-Glycero-3-Succinate (DOGS) (0,485 mmol ; 350 mg) est préalablement placé sous atmosphère inerte puis est dissous dans 20 mL de CH₂Cl₂ anhydre sous agitation. Le DCC fraîchement recristallisé (0,727 mmol ; 150 mg) et en solution dans 5 mL de CH₂Cl₂ anhydre, puis le NHS (0,727 mmol ; 84 mg) en solution dans 5 mL de DMF anhydre, sont successivement additionnés dans le milieu réactionnel. La réaction est alors maintenue 2 h sous agitation et sous atmosphère inerte à température ambiante. Dans un ballon de 10 mL sec, le composé **1b** (0,535 mmol ; 250 mg) est préalablement placé sous atmosphère inerte, puis dissous dans 10 mL de CH₂Cl₂ anhydre. A cette solution, est ajoutée de la triéthylamine (0,535 mmol ; 75 µL) sous agitation. La solution est agitée pendant 10 min à température ambiante puis additionnée au milieu réactionnel. Après 24 h d'agitation à température ambiante sous atmosphère inerte, la réaction est stoppée et le milieu réactionnel est évaporé à sec sous vide primaire. Le produit de couplage est ensuite purifié par flash chromatographie sur gel de silice (gradient d'élution de 0 à 5% MeOH dans CH₂Cl₂). On isole ainsi 414 mg du composé **1c** soit un rendement de 73 %.

**CCM :** R_{f} **1c** = 0,8 (CH₂Cl₂ / MeOH 9/1 (v/v))

**ESI-MS +** : *m*/*z* mesuré à 1169,6 [M+H]⁺, calculé à 1169,8 pour C₆₂H₁₁₃N₄O₁₂S₂ ; *m*/*z* mesuré à 569,4 [M+H+Na]²⁺, calculé à 569,4 pour C₆₂H₁₁₃N₄O₁₂S₂Na.

### 2.3 Etape 3 : Préparation du 181GSCO (1d)

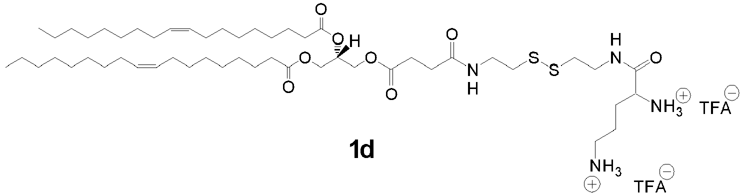

Dans un ballon de 50 mL, le composé **1c** (0,342 mmol ; 400 mg) est dissous dans 8 mL de CH₂Cl₂, puis 2 mL d'acide trifluoroacétique sont ajoutés dans le milieu réactionnel. La réaction est alors maintenue pendant 1 h sous agitation à température ambiante. Le milieu réactionnel est évaporé à sec sous vide primaire. Pour chasser les traces d'acide trifluoroacétique en excès, le résidu final est repris 3 fois de suite dans 5 mL de dichlorométhane puis évaporé à sec. On isole ainsi 410 mg du composé **1d** sous forme d'un sel de trifluoroacétate. La réaction est quantitative.

Le rendement total de la synthèse (3 étapes) est de 34%.

**CCM** : R_{f} **1d** = 0,1 (CH₂Cl₂ / MeOH 9/1 (v/v))

**CLHP :** Rt = 39,73 min

(gradient ternaire H₂O / CH₃CN / CH₃CN + 10 % CH₃COOH, Débit = 1 mL/min).

**ESI-HRMS** (haute résolution avec détection en mode positif) : *m*/*z* mesuré à 969,6739 [M+H]⁺, calculé à 969,6748 pour C₅₂H₉₇N₄O₈S₂ (déviation : 0,9 ppm).

### Exemple 2 : Préparation de la DiOleoyl-Glycero-Succinyl-Cystamido-Arginine 2d (181GSCR)

On obtient le 181GSCR, ou composé **2d,** de formule : 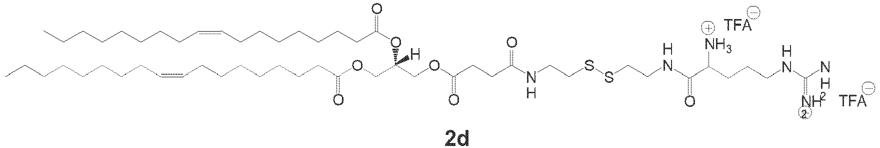 en trois étapes à partir de la N_{α},N_{ω},N_{ω'}-tris-Boc-L-Arginine **2a**.

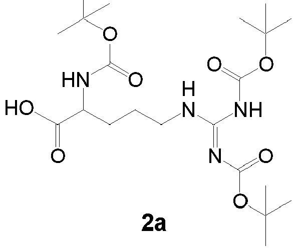

### 2.1 Etape 1 : préparation de la Cystamido-tris-Boc-L-Arginine (2b)

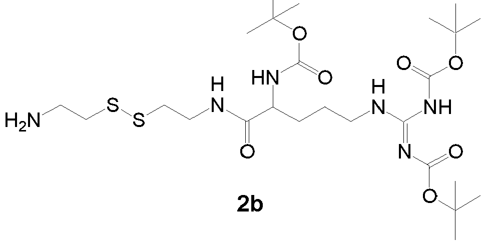

Dans un ballon de 250 mL sec, la N_{α},N_{ω},N_{ω'}-tris-Boc-L-Arginine **2a** (1,21 mmol ; 875 mg) est préalablement placée sous atmosphère inerte puis dissoute dans 30 mL de DMF anhydre sous agitation. Le N,N'-diisopropylcarbodiimide (DIC) (1,82 mmol ; 282 µL), puis le N-Hydroxysuccinimide (NHS) (1,82 mmol ; 209 mg) en solution dans 5 mL de DMF anhydre, sont successivement additionnés dans le milieu réactionnel. La réaction est alors maintenue pendant 2 h sous agitation et sous atmosphère inerte à température ambiante. Dans un ballon de 50 mL, le dichlorure de cystamine (6,1 mmol ; 1,37 g) est dissous dans 2 mL d'eau. A cette solution est ajoutée de la triéthylamine (12,2 mmol ; 1,7 mL) sous agitation, puis 20 mL de DMF. Cette solution est agitée pendant 5 minutes à 50°C puis immédiatement ajoutée au milieu réactionnel. La réaction est alors maintenue pendant 24 h sous agitation à température ambiante. Les solvants sont ensuite évaporés sous vide primaire jusqu'à obtenir un résidu huileux de 1 à 2 mL. Ce résidu est repris dans 50 mL de CH₂Cl₂ sous agitation. Le précipité qui s'est formé est alors filtré et lavé avec du CH₂Cl₂ (2 × 10 mL). Les filtrats sont recueillis et évaporés sous vide primaire. Le produit de couplage est ensuite purifié par flash chromatographie sur gel de silice (élution : CH₂Cl₂/MeOH 95/5 puis 9/1 (v/v)). On isole ainsi 345 mg du composé **2b** soit un rendement de 47 %.

**CCM :** R_{f} **2b** = 0,6 (CH₂Cl₂ / MeOH 8/2 (v/v))

**ESI-MS** + : *m*/*z* mesuré à 609,2 [M+H]⁺, calculé à 609,3 pour C₂₆H₄₉N₆O₇S₂.

### 2.2 Etape 2 : préparation de la DiOleoyl-Glycero-Succinyl-Cystamido-tris-Boc-L-Arginine (2c)

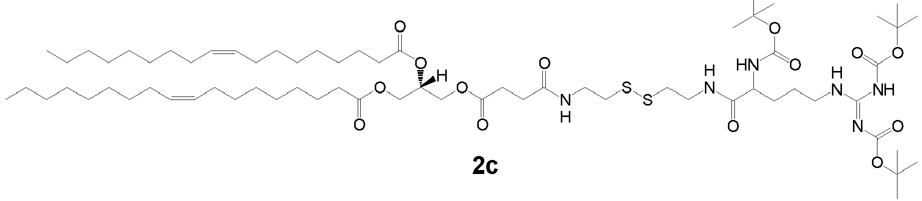

Dans un ballon de 250 mL sec, le 1,2-Dioleoyl-*sn*-Glycero-3-Succinate (DOGS) (0,33 mmol ; 240 mg) est préalablement placé sous atmosphère inerte puis est dissous dans 20 mL de CH₂Cl₂ anhydre sous agitation. Le DCC fraîchement recristallisé (0,50 mmol ; 103 mg) et en solution dans 5 mL de CH₂Cl₂ anhydre, puis le NHS (0,50 mmol ; 58 mg) en solution dans 5 mL de DMF anhydre, sont successivement additionnés dans le milieu réactionnel. La réaction est alors maintenue 2 h sous agitation et sous atmosphère inerte à température ambiante. Dans un ballon de 50 mL sec, le composé **2b** (0,50 mmol ; 305 mg) est préalablement placé sous atmosphère inerte, puis dissous dans 15 mL de CH₂Cl₂ anhydre. A cette solution, est ajoutée de la triéthylamine (0,50 mmol ; 70 µL) sous agitation. La solution est agitée pendant 10 min à température ambiante puis additionnée au milieu réactionnel. Après 24 h d'agitation à température ambiante sous atmosphère inerte, la réaction est stoppée et le milieu réactionnel est évaporé à sec sous vide primaire. Le produit de couplage est ensuite purifié par flash chromatographie sur gel de silice (gradient d'élution de 0 à 2 % MeOH dans CH₂Cl₂). On isole ainsi 239 mg du composé **2c** soit un rendement de 55 %.

**CCM :** R_{f} **2c** = 0,9 (CH₂Cl₂ / MeOH 9/1 (v/v))

**ESI-MS + :** *m*/*z* mesuré à 1311,7 [M+H]⁺, calculé à 1311,9 pour C₆₈H₁₂₃N₆O₁₄S₂ ; *m*/*z* mesuré à 667,4 [M+H+Na]²⁺, calculé à 667,4 pour C₆₈H₁₂₃N₆O₁₄S₂Na.

### 2.3 Etape 3 : Préparation du 181GSCR (2d)

Dans un ballon de 50 mL, le composé **2c** (0,17 mmol ; 220 mg) est dissous dans 5 mL de CH₂Cl₂, puis 5 mL d'acide trifluoroacétique sont ajoutés dans le milieu réactionnel. La réaction est alors maintenue pendant 1 h sous agitation à température ambiante. Le milieu réactionnel est évaporé à sec sous vide primaire. Pour chasser les traces d'acide trifluoroacétique en excès, le résidu final est repris 3 fois de suite dans 5 mL de dichlorométhane puis évaporé à sec. On isole ainsi 210 mg du composé **2d** sous forme d'un sel de trifluoroacétate. La réaction est quantitative.

Le rendement total de la synthèse (3 étapes) est de 26 %.

**CCM** : R_{f} **2d** = 0,1 (CH₂Cl₂ / MeOH 9/1 (v/v))

**CLHP** : Rt = 39,46 min

(gradient ternaire H₂O / CH₃CN / CH₃CN + 10 % CH₃COOH, Débit = 1 mL/min).

**ESI-HRMS** (haute résolution avec détection en mode positif) : *m*/*z* mesuré à 1011,6984 [M+H]⁺, calculé à 1011,6966 pour C₅₃H₉₉N₆O₈S₂ (déviation : 1,8 ppm).

### Exemple 3 : Préparation du DiLauroyl-Glycero-Succinyl-Cystamido-Lysine (3) (12GSCL)

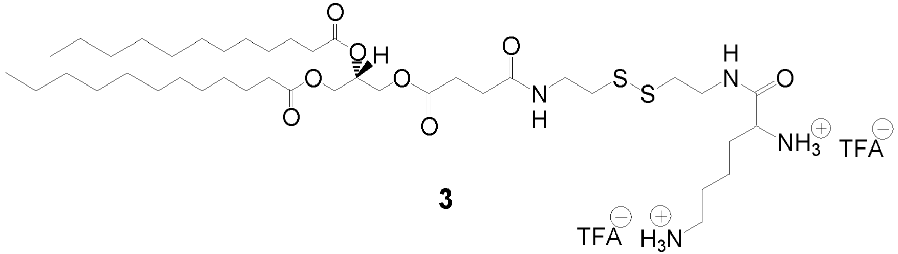

La procédure de synthèse est identique à la synthèse du 181GSCO (Exemple 1) mais en utilisant la N_{α},N_{δ}-di-Boc-L-Lysine au lieu de la N_{α},N_{ε},-di-Boc-L-Ornithine et le 1,2-Dilauroyl-*sn*-Glycero-3-Succinate (DLGS) au lieu du 1,2-Dioleoyl-*sn*-Glycero-3-Succinate (DOGS). On obtient un rendement total de 31 %.

**CCM** : R_{f} **3** = 0 (CH₂Cl₂ / MeOH 9/1 (v/v))

**ESI-HRMS** (haute résolution avec détection en mode positif) : *m*/*z* mesuré à 819,5356 [M+H]⁺, calculé à 819,5339 pour C₄₀H₇₇N₄O₈S₂ (déviation : 2,1 ppm).

### Exemple 4 : Préparation du DiOleoyl-Glycero-Succinyl-Glutamido-bis-(Cystamido-Ornithine) : 181GSGlu(CO)₂ ; (Composé 4e : Figure 2)

On obtient le 181GSGlu(CO)₂, ou composé **4e,** de formule : 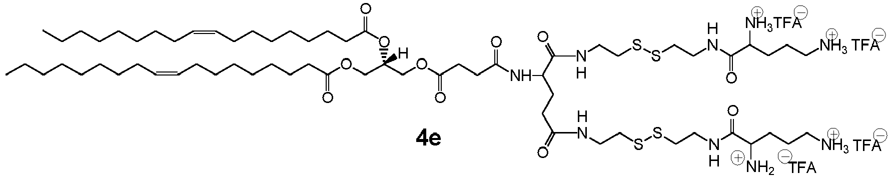 en quatre étapes à partir du 1,2-Dioleoyl-sn-Glycero-3-Succinate(DOGS) **4a** (Figure 2).

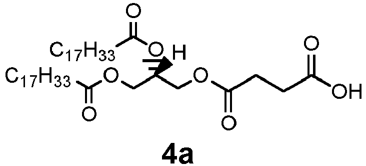

### 4.1 Préparation du DiOleoyl-Glycero-Succinyl-di-OtBu-Glutamate (4b)

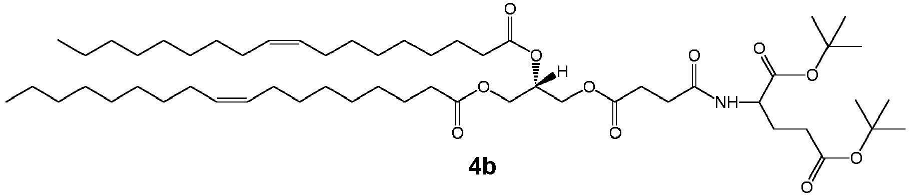

Dans un ballon de 10 mL sec, le 1,2-Dioleoyl-*sn*-Glycero-3-Succinate (DOGS) **4a** (0,031 mmol ; 22,5 mg) est préalablement placé sous atmosphère inerte puis est dissous dans 3 mL de CH₂Cl₂ anhydre sous agitation. Le DIC (0,047 mmol ; 7,3 µL) puis, HOBt (0,047 mmol ; 6,4 mg) en solution dans 500 µL de DMF anhydre, sont successivement additionnés dans le milieu réactionnel. La réaction est alors maintenue 2 h sous agitation et sous atmosphère inerte à température ambiante. Dans un deuxième ballon de 5 mL sec, le H-Glu(OtBu)-OtBu.HCl (0,047 mmol ; 14 mg) est préalablement placé sous atmosphère inerte, puis dissous dans 500 µL de CH₂Cl₂ anhydre. A cette solution, est ajoutée de la triéthylamine (0,047 mmol ; 6,6 µL) sous agitation. La solution est agitée pendant 10 min à température ambiante puis additionnée au milieu réactionnel. Après 24 h d'agitation à température ambiante sous atmosphère inerte, la réaction est stoppée et le milieu réactionnel est évaporé à sec sous vide primaire. Le produit de couplage est ensuite purifié par flash chromatographie sur gel de silice (gradient d'élution de 0 à 1 % MeOH dans CHCl₃). On isole ainsi 28,1 mg du composé **4b** soit un rendement de 93 %.

**CCM** : R_{f} **4b** = 0,9 (CHCl₃ / MeOH / AcOH 95/5/0,2 (v/v/v))

**ESI-MS + :** *m*/*z* mesuré à 984,8 [M+Na]⁺, calculé à 984,7 pour C₅₆H₉₉NO₁₁Na.

### 4.2 Préparation du DiOleoyl-Glycero-Succinyl-Glutamate (4c)

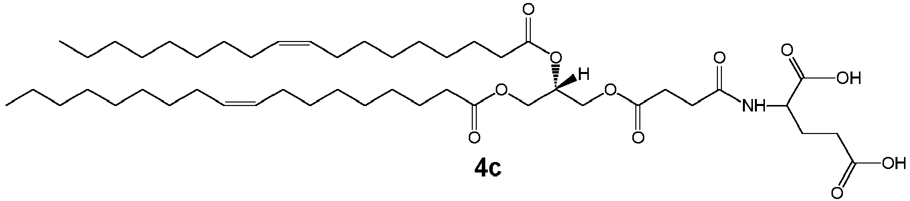

Dans un ballon de 10 mL, le composé **4b** (0,026 mmol ; 25,5 mg) est dissous dans 1,6 mL de CH₂Cl₂, puis 400 µL d'acide trifluoroacétique sont ajoutés dans le milieu réactionnel. La réaction est alors maintenue pendant 1 h sous agitation à température ambiante. Le milieu réactionnel est évaporé à sec sous vide primaire. Pour chasser les traces d'acide trifluoroacétique en excès, le résidu final est repris 3 fois de suite dans 5 mL de dichlorométhane puis évaporé à sec. On isole ainsi 24,4 mg du composé **4c**. La réaction est quantitative.

**CCM** : R_{f} **4c** = 0,1 (CHCl₃ / MeOH / AcOH 95/5/0,2 (v/v/v) ; R_{f} **4b** = 0,9)

**ESI-MS +** : *m*/*z* mesuré à 871,6 [M+Na]⁺, calculé à 871,6 pour C₄₈H₈₂NO₁₁Na.

### 4.3 Préparation du DiOleoyl-Glycero-Succinyl-Glutamido-di(Cystamido-di-Boc-Ornithine) (4d)

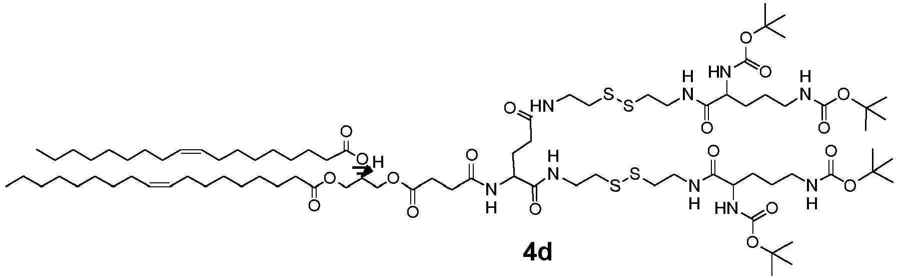

Dans un ballon de 10 mL sec, le composé **4c** (0,015 mmol ; 12,5 mg) est préalablement placé sous atmosphère inerte puis est dissous dans 3 mL de CH₂Cl₂ anhydre sous agitation. Le DIC (0,045 mmol ; 7,0 µL) puis, HOBt (0,045 mmol ; 6,1 mg) en solution dans 0,5 mL de DMF anhydre, sont successivement additionnés dans le milieu réactionnel. La réaction est alors maintenue 2 h sous agitation et sous atmosphère inerte à température ambiante. Dans un deuxième ballon de 10 mL sec, le composé **1b** (synthèse décrite précédemment dans l'exemple 1) (0,045 mmol ; 21,0 mg) est préalablement placé sous atmosphère inerte, puis dissous dans 1,5 mL de CH₂Cl₂ anhydre. A cette solution, est ajoutée de la triéthylamine (0,045 mmol ; 6,2 µL) sous agitation. La solution est agitée pendant 10 min à température ambiante puis additionnée au milieu réactionnel. Après 24 h d'agitation à température ambiante sous atmosphère inerte, la réaction est stoppée et le milieu réactionnel est évaporé à sec sous vide primaire. Le produit de couplage est ensuite purifié par flash chromatographie sur gel de silice (gradient d'élution de 0 à 5 % MeOH dans CH₂Cl₂). On isole ainsi 20,6 mg du composé **4d** soit un rendement de 80 %.

**CCM** : R_{f} **4d** = 0,35 (CH₂Cl₂ / MeOH 95/5 (v/v))

**ESI-MS + :** *m*/*z* mesuré à 1769,2 [M+Na]⁺, calculé à 1769,0 pour C₈₆H₁₅₅N₉O₁₉S₄Na.

### 4.4 Préparation du 181GSGlu(CO)₂ (4e)

Dans un ballon de 10 mL, le composé **4d** (0,011 mmol ; 20 mg) est dissous dans 1,6 mL de CH₂Cl₂, puis 400 µL d'acide trifluoroacétique sont ajoutés dans le milieu réactionnel. La réaction est alors maintenue pendant 1 h sous agitation à température ambiante. Le milieu réactionnel est évaporé à sec sous vide primaire. Pour chasser les traces d'acide trifluoroacétique en excès, le résidu final est repris 3 fois de suite dans 5 mL de dichlorométhane puis évaporé à sec. On isole ainsi 19,5 mg du composé **4e** sous forme d'un sel de trifluoroacétate. La réaction est quantitative.

**CCM** : R_{f} **4e** = 0 (CH₂Cl₂ / MeOH 9/1 (v/v) ; R_{f} **4d** = 0,9)

**ESI-MS** + : *m*/*z* mesuré à 1346,9 [M+H]⁺, calculé à 1346,8 pour C₆₆H₁₂₄N₉O₁₁S₄ ; *m*/*z* mesuré à 1368,9 [M+Na]⁺, calculé à 1368,8 pour C₆₆H₁₂₃N₉O₁₁ S₄Na.

### Exemple 5 : Préparation de la DiOleoyl-Glycero-Succinyl-Cystamido-bis-Lysine (5f) (181GSCL₂)

On obtient le 181GSCL₂, ou composé **5f,** de formule : 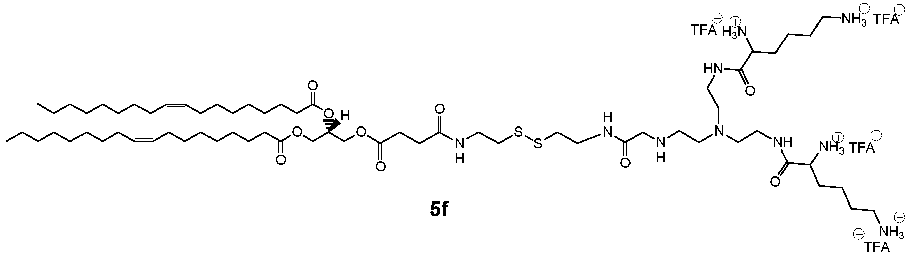 à partir du composé bis-lysine **5e** dont la préparation est décrite ci-dessous.

### 5.1 Préparation d'une bis-Lysine branchée comme précurseur de synthèse des agents de transfert : synthèse de [Boc-Lys(Boc)-NH-(CH₂)₂]₂-N-(CH₂)₂-NH-CH₂-COOH (5e) par SPPS (Figure 3)

On obtient le composé **5e,** de formule : 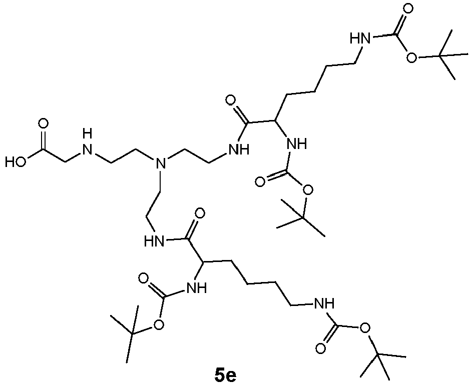 en quatre étapes à partir de la résine Chlorotrityl chloride **5a** (Figure 3)

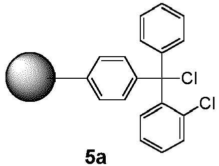

### • Etape 1 : ancrage d'une fonction acide sur un support polymérique 5a pour obtenir le composé 5b

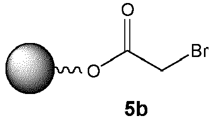

De la résine Chlorotrityl chloride **5a** (1,4 mmol Cl / g résine ; 5 g) est chargée dans un réacteur SPPS, 50 mL de CH₂Cl₂ sont ajoutés et le mélange est agité pendant 5 min. De l'acide bromoacétique (8,5 mmol ; 1,2 g) est ajouté, suivi de DIPEA (9 mmol ; 1,5 mL). Le réacteur est agité pendant 2 h à température ambiante. Le liquide est filtré et la résine est lavée successivement avec CH₂Cl₂ et iPrOH (10 × 50 mL) puis avec MeOH (2 × 50 mL). Enfin, la bromoacétyl-résine obtenue (composé **5b**, Figure 3) est séchée sous un courant d'azote.

### • Etape 2 : Couplage du tris-(2-aminoéthyl)amine avec la bromoacetyl-résine 5b pour obtenir le composé 5c

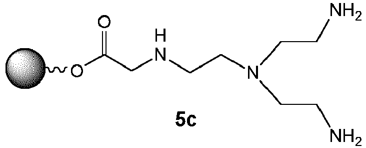

Le tris-(2-aminoethyl)amine (70 mmol ; 10,2 g) est dissous dans 50 mL CH₂Cl₂ et chargé dans le réacteur contenant la bromoacétyl-résine **5b** (étape 1). Le réacteur est agité pendant 2 h à température ambiante. Le solvant est filtré et la résine lavée successivement avec CH₂Cl₂ et iPrOH (10 × 50 mL). Enfin, la bis-aminoéthyl-résine obtenue (composé **5c**, Figure 3) est séchée sous un courant d'azote. Le test de Kaiser est positif.

### • Etape 3 : Couplage de la N_{α},N_{ε}-di-Boc-L-Lysine avec la bis-aminoéthyl-résine 5c pour obtenir le composé 5d

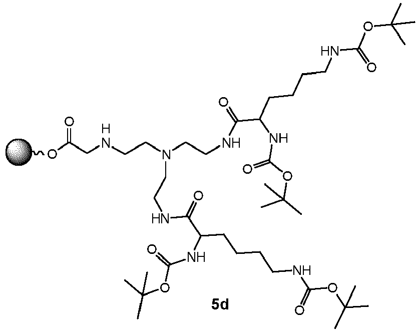

Les N_{α},N_{ε}-di-BocL-Lysine (28 mmol ; 9,70 g) et 1-Hydroxybenzotriazole (HOBt) (30 mmol ; 4,05 g) sont dissous dans 50 mL CH₂Cl₂ / DMF 4 / 1 (v/v). Le DIC (30 mmol ; 5,1 mL) est additionné à la solution d'acide aminée et le milieu réactionnel est maintenu sous agitation et sous atmosphère inerte pendant 30 min. La solution est ensuite chargée dans le réacteur contenant la bis-aminoéthyl-résine **5c** (étape 2). Le réacteur est agité pendant 18 h à température ambiante. Le solvant est filtré et la résine lavée successivement avec CH₂Cl₂ et iPrOH (10 × 50 mL) puis avec MeOH et éther (2 × 50 mL). Enfin, la bis-(di-Boc-Lysine)-résine obtenue (composé **5d**, Figure 3) est séchée sous un courant d'azote. Le test de Kaiser est négatif.

### • Etape 4 : Clivage de la bis-(di-Boc-Lysine)-résine 5d pour obtenir le composé 5e

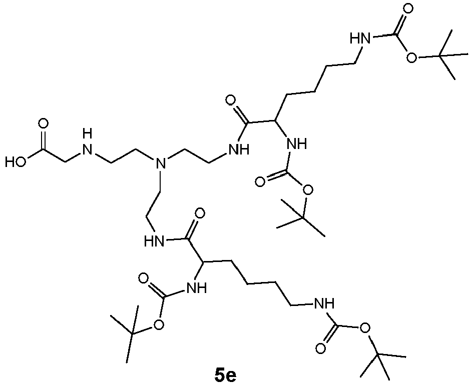

La bis-(di-Boc-Lysine)-résine **5d** (étape 3) est chargée dans un ballon de 250 mL équipée d'un barreau magnétique. Une solution composée de 50 mL de CH₂Cl₂ et 25 mL de CF₃CH₂OH est ajoutée et le mélange est agité pendant 2 h à température ambiante. La solution est filtrée, la résine est lavée avec CH₂Cl₂ (2 × 10 mL) et les phases organiques ainsi obtenues sont rassemblées et évaporées sous vide primaire. Le produit clivé est ensuite purifié par flash chromatographie sur gel de silice avec comme éluant CH₂Cl₂ / MeOH 9 / 1 (v/v). On isole ainsi 1,98 g du composé **5e** soit un rendement total de 34 % à partir de la résine Chlorotrityl chloride.

**CCM :** R_{f} **5e** = 0,4 (CHCl₃ / MeOH 9/1 (v/v))

**ESI-MS + :** *m*/*z* mesuré à 854,4 [M+Na]⁺, calculé à 854,5 pour C₃₈H₇₁N₈O₁₂Na.

### 5.2 Préparation de 181GSCL₂ (5f)

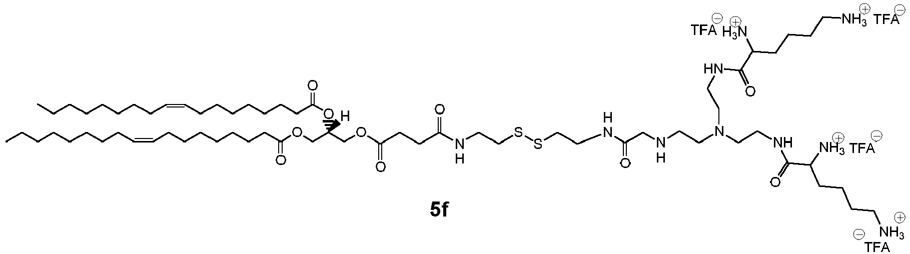

La procédure de synthèse est identique à la synthèse du 181GSCO (Exemple 1) mais en utilisant le composé **5e** au lieu de la N_{α},N_{ε}-di-Boc-ornithine (**1a**). On obtient un rendement total de 25 %.

**CCM :** R_{f} **5f** = 0 (CH₂Cl₂ / MeOH 9/1 (v/v))

**ESI-HRMS** (haute résolution avec détection en mode positif) : *m*/*z* mesuré à 1269,9072 [M+H]⁺, calculé à 1269,9021 pour C₆₅H₁₂₅N₁₀O₁₀S₂ (déviation : 4,0 ppm).

### B. PREPARATION DE FORMULATIONS CYTOFECTANTES A PARTIR DES AGENTS DE TRANSFERT SELON L'INVENTION

### Exemple 6 : Formulation sous forme de liposomes, vésicules ou micelles dans l'eau

Un des lipides cationiques décrits dans les exemples précédents est dissous à une concentration donnée dans du chloroforme. D'autre part, un co-lipide (dioléoylphosphatidyléthanolamine, dioléoylphosphatidylcholine, cholestérol, cholestérol-amine...) est également dissous à une concentation donnée dans du chloroforme. En combinant différentes quantités de ces deux solutions dans un pilulier, différentes compositions d'un mélange de lipide cationique et de co-lipide sont obtenues. Le chloroforme est ensuite évaporé sous vide primaire pour obtenir un film lipidique le long des parois du pilulier. Ce film est réhydraté avec une quantité donnée d'eau déionisée stérile. Après complète réhydratation du film, la dispersion est soumise à sonication pour former de petits liposomes unilamellaires.

Par exemple, on mélange dans un pilulier 600 µL (6 mg ; 5 µmol) d'une solution de 181GSCO (exemple 1) à 10 mg.mL⁻¹ dans du chloroforme et 372 µL (3,72 mg ; 5 µmol) d'une solution de dioléoylphosphatidyléthanolamine (DOPE) à 10 mg.mL⁻¹ dans du chloroforme. Le chloroforme est évaporé à sec sous vide primaire et le film lipidique obtenu est remis en suspension dans 5 mL d'eau déionisée stérile. Après réhydratation pendant une nuit à 4°C, la dispersion est soumise à sonication pendant 15 minutes.

### Exemple 7 : Formulation sous forme de liposomes, vésicules ou micelles par injection éthanolique dans l'eau

Un des lipides cationiques décrits dans les exemples précédents est dissous à une concentration donnée dans du chloroforme. D'autre part, un co-lipide (dioléoylphosphatidyléthanolamine, dioléoylphosphatidylcholine, cholestérol, cholestérol-amine...) est également dissous à une concentration donnée dans du chloroforme. En combinant différentes quantités de ces deux solutions dans un pilulier, différentes compositions d'un mélange de lipide cationique et de co-lipide sont obtenues. Le chloroforme est ensuite évaporé sous vide primaire pour obtenir un film lipidique le long des parois du pilulier. Ce film est alors re-dissous dans une petite quantité d'éthanol (entre 3 et 5 % du volume finale de la formulation). Cette solution éthanolique est ensuite injectée rapidement à l'aide d'une seringue Hamilton dans un volume donné d'eau deionisée sous agitation.

Par exemple, on mélange dans un pilulier 600 µL (6 mg ; 5 µmol) d'une solution de 181GSCO (exemple 1) à 10 mg.mL⁻¹ dans du chloroforme et 372 µL (3,72 mg ; 5 µmol) d'une solution de dioléoylphosphatidyléthanolamine (DOPE) à 10 mg.mL⁻¹ dans du chloroforme. Le chloroforme est évaporé à sec sous vide primaire et le film lipidique obtenu est re-dissous dans 150 µL d'éthanol. La solution éthanolique est ensuite injectée rapidement à l'aide d'une seringue Hamilton dans un ballon contenant 5 mL d'eau déionisée sous forte agitation. L'agitation est maintenue pendant quelques minutes.

### Exemple 8 : Formulation dans une solution éthanolique

Un des lipides cationiques décrits dans les exemples précédents est dissous à une concentration donnée dans du chloroforme. D'autre part, un co-lipide (dioléoylphosphatidyléthanolamine, dioléoylphosphatidylcholine, cholestérol, cholestérol-amine...) est également dissous à une concentration donnée dans du chloroforme. En combinant différentes quantités de ces deux solutions dans un pilulier, différentes compositions d'un mélange de lipide cationique et de co-lipide sont obtenues. Le chloroforme est ensuite évaporé sous vide primaire pour obtenir un film lipidique le long des parois du pilulier. Ce film est alors re-dissous dans une solution éthanol / eau déionisée 80 / 20 (v/v) sous forte agitation.

Par exemple, on mélange dans un pilulier 600 µL (6 mg ; 5 µmol) d'une solution de 181GSCO (exemple 1) à 10 mg.mL⁻¹ dans du chloroforme et 372 µL (3,72 mg ; 5 µmol) d'une solution de dioléoylphosphatidyléthanolamine (DOPE) à 10 mg.mL⁻¹ dans du chloroforme. Le chloroforme est évaporé à sec sous vide primaire et le film lipidique obtenu est re-dissous dans 5 mL d'une solution éthanol / eau 80 / 20 (v/v).

### C. APPLICATIONS : TRANSPORT DE MOLECULES ACTIVES DANS LES CELLULES VIVANTES

### Exemple 9 : Utilisation des agents de transfert selon l'invention pour le transport d'ADN dans les cellules vivantes

### 9.1 Matériel

Le plasmide, avec lequel on souhaite transfecter les cellules, est soit un pCMV-LacZ, soit un pCMV-EGFP. pCMV-LacZ comporte le gène codant pour une enzyme, la β-galactosidase, sous promoteur du gène CMV. L'activité de la β-galactosidase produite dans les cellules est facilement mesurable à l'aide de tests colorimétriques. Le pCMV-EGFP comporte le gène codant pour une protéine fluorescente, la GFP, sous contrôle du promoteur du gène CMV. La GFP produite dans les cellules est observée au microscope à fluorescence et quantifiée par FACS. Les plasmides sont préparés à partir de cultures bactériennes (*E. Coli)* et purifiés sur colonne d'affinité. Les solutions d'acides nucléiques obtenues sont diluées à 1 mg.mL⁻¹ dans l'eau et stockées à -20°C.

### 9.2 Protocole de transfection

### 1. Préparation des cellules :

Les cellules immortalisées adhérentes (Vero, NIH-3T3, HeLa) sont mises en culture dans une plaque de culture cellulaire 96 puits (15000 cellules par puit dans 100 µL de DMEM) un jour avant l'essai de transfection et sont transfectées alors qu'elles sont en phase exponentielle de croissance et à 80 % de la confluence.

Les cellules immortalisées en suspension (Jurkat, K562) sont préparées le jour avant la transfection à une densité de 2 à 5 × 10⁶ cellules par mL. Le jour de la transfection 0.5 à 1 × 10⁵ cellules sont mises en culture dans une plaque de culture cellulaire 96 puits.

Les solutions d'ADN et les formulations lipidiques obtenues selon l'exemple 6 sont portées à température ambiante, et sont délicatement agitées en pipetant plusieurs fois les solutions, par aspiration et refoulement, avant d'être utilisées pour la transfection. On prépare ensuite les complexes ADN / formulation lipidique à différentes teneurs en ADN et en formulation lipidique de la façon suivante.

### 2. Dilution de la formulation lipidique :

Dans des tubes Eppendorf de 1,5 mL, la formulation lipidique est diluée avec du milieu de culture (DMEM). On prépare trois solutions diluées à différentes concentrations de formulation lipidique (Table1).

**Table 1 : Solutions diluées de la formulation lipidique**

| | Volume formulation lipidique (µL) | Volume de DMEM (µL) | Volume formulation lipidique / puits (µL) |
|---|---|---|---|
| Solution A | 2 | 198 | 0,5 |
| Solution B | 4 | 196 | 1 |
| Solution C | 8 | 192 | 2 |

3. Dans des tubes Eppendorf de 1,5 mL, la solution mère d'ADN (1 mg.mL⁻¹) est diluée dans du milieu de culture (DMEM). On prépare trois solutions diluées à différentes concentrations d'ADN (Table 2).

**Table 2 : Solutions diluées d'ADN**

| | Volume solution mère d'ADN (µL) | Volume de DMEM (µL) | Quantité d'ADN / puits (µg) |
|---|---|---|---|
| Solution D | 1 | 199 | 0,25 |
| Solution E | 2 | 198 | 0,5 |
| Solution F | 4 | 196 | 1 |

4. Les complexes ADN / formulation lipidique sont préparés dans des tubes Eppendorf 1,5 mL, en mélangeant 50 µL de chaque solution diluée de formulation lipidique avec 50 µL de chaque solution diluée d'ADN. Les mixtures sont agitées délicatement en pipetant plusieurs fois les solutions, par aspiration et refoulement, puis incubées pendant 20 minutes à température ambiante.

5. Les 100 µL de complexes sont ajoutés sur les cellules en culture en milieu complet (transfection en présence de sérum), et la mixture est homogénéisée par agitation de la plaque de culture cellulaire pour permettre une distribution uniforme sur les cellules.

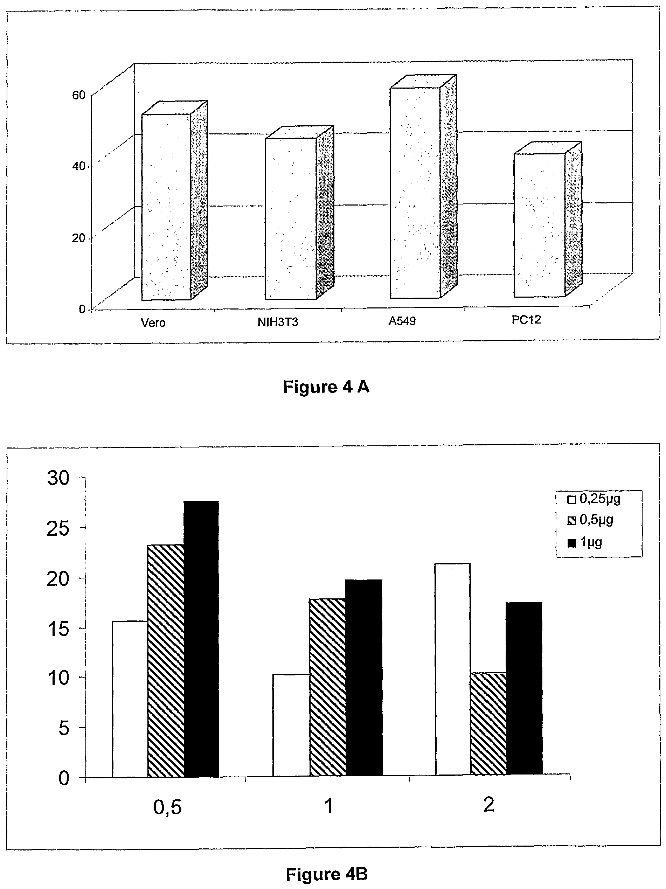

6. Les cellules sont incubées à 37°C en atmosphère humide contenant 5 % de CO₂ jusqu'à la mesure de l'expression du transgène. En fonction de l'activité du promoteur, l'efficacité de la transfection peut être évaluée de 24 à 72 heures post-transfection. Un changement de milieu peut-être effectué 24 h post-transfection (Figure 4 et 9).

### Exemple 10 : Utilisation des produits selon l'invention pour le transport d'ARN si dans les cellules vivantes

### 10.1 Matériel

Le plasmide avec lequel on souhaite transfecter les cellules est un ARN Si anti-EGFP (Ambion, TX, USA), dont la séquence est
5'-GCAAGCUGACCCUGAAGUUCUU (sens)
et
5'-GAACUUCAGGGUCAGCUUGCUU (antisense).

L'efficacité de la séquence à inhiber la GFP produite dans les cellules est observée au microscope à fluorescence et mesurée par FACS. Un ARN Si contrôle a été utilisée pour mesurer l'inhibition non spécifique. Les solutions d'ARN sont à 1 µmol.L⁻¹ dans l'eau et sont stockées à -20°C.

### 10.2 Protocole de transfection

### 1. Préparation des cellules :

Les cellules Hela-GFP sont mises en culture dans une plaque de culture cellulaire 24 puits (60000 cellules par puit dans 400 µL de DMEM) un jour avant l'essai de transfection et sont transfectées alors qu'elles sont en phase exponentielle de croissance et à 80 % de confluence.

2. La solution d'ARNSi et la formulation lipidique obtenue selon l'exemple 6 sont portées à température ambiante, et sont délicatement agités en pipetant plusieurs fois les solutions, par aspiration et refoulement, avant d'être utilisées pour la transfection.

3. Dans un tube Eppendorf de 1,5 mL, la solution mère d'ARN Si (1µmol.L⁻¹) est diluée dans un volume total de 50 µL avec du milieu de culture (DMEM). Une série de plusieurs solutions diluées sont préparées (Table 3) afin de tester l'efficacité d'inhibition du plasmide à plusieurs concentrations finales (1nM, 10nM et 20nM).

**Table 3 : Solutions diluées d'ARN Si**

| Concentration finale en ARN Si (nmol.L⁻¹) | Quantité d'ARN Si (ng) | Volume d'ARN Si à 1 µmol.L⁻¹ (µL) | Volume de DMEM (µL) |
|---|---|---|---|
| 1 | 6,75 | 0,5 | 49,5 |
| 10 | 67,5 | 5 | 45 |
| 20 | 135 | 10 | 40 |

4. Dans un tube Eppendorf de 1,5 mL, la formulation lipidique est diluée dans un volume total de 50 µL avec du milieu de culture (DMEM). Une série de plusieurs solutions diluées sont préparées pour chaque concentration finale d'ARN Si (Table 4).

**Table 4: Solutions diluées de la formulation lipidique**

| Concentration finale en ARN Si (nmol.L⁻¹) | Volume formulation lipidique (µL) | Volume de DMEM (µL) |
|---|---|---|
| 1 | 1 | 49 |
| 10 | 2 | 48 |
| 20 | 3 | 47 |

5. Les complexes ARN Si / formulation lipidique sont préparés en additionnant les 50µL de la solution diluée de plasmide aux 50µL de la solution diluée de formulation lipidique. La mixture est agitée délicatement en pipetant plusieurs fois la solution, par aspiration et refoulement, puis incubée pendant 20 minutes à température ambiante.

6. Les 100 µL de complexes sont ajoutés sur les cellules en culture en milieu complet (transfection en présence de sérum), et la mixture est homogénéisée par agitation de la plaque de culture cellulaire pour permettre une distribution uniforme sur les cellules.

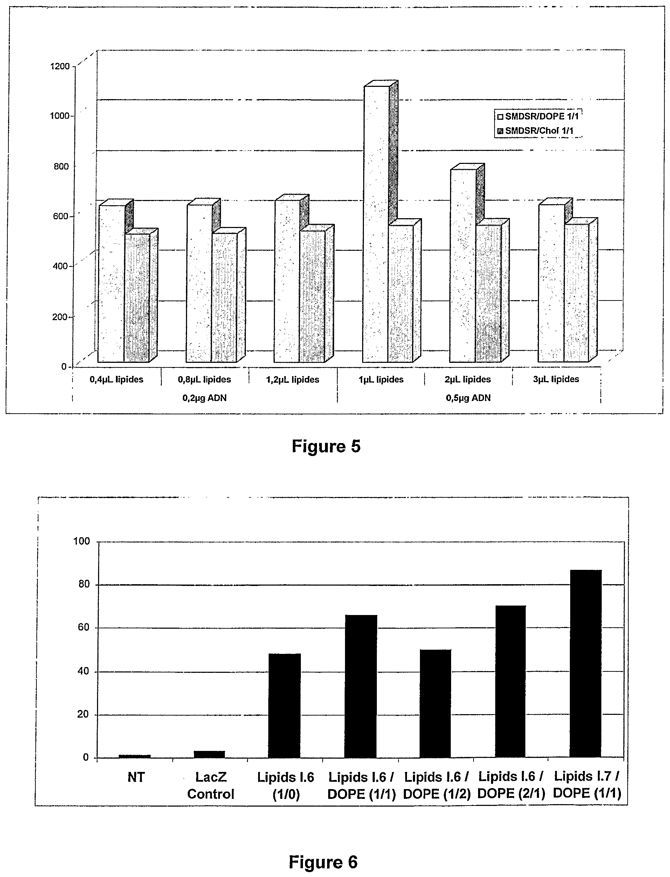

7. Les cellules sont incubées à 37°C en atmosphère humide contenant 5 % de CO₂ pendant 72 h. Les cellules sont ensuite tripsinisées et l'efficacité d'inhibition est observée par microscopie électronique et/ou analysée quantitativement par FACS (Figures 5 et 6).

### Exemple 11 : Utilisation des produits selon l'invention pour le transport de protéines dans les cellules vivantes

### 11.1 Matériel

Les polypeptides que l'on souhaite délivrer dans les cellules sont une IgG de chèvre purifiée (Sigma-Aldrich SARL, Saint Quentin Fallavier, France), préalablement marquée à la fluorescéine, une R-Phycoerythrin (Invitrogen, San Diego, CA, USA) et une β-galactosidase (Merck KGaA, Darmstadt, Germany). Les polypeptides sont utilisés en solution à 100 µg.mL⁻¹ dans du PBS. L'efficacité de transport des polypeptides marqués dans les cellules est observée au microscope à fluorescence et est quantifiée par FACS. L'activité de la β-galactosidase produite dans les cellules est facilement mesurable à l'aide de tests colorimétriques.

### 11.2 Procédure

### 1. Préparation des cellules :

Les cellules immortalisées adhérentes (Vero, NIH-3T3, HeLa) sont mises en culture dans une plaque de culture cellulaire 24 puits (75000 cellules par puit dans 400 µL de DMEM) un jour avant l'essai de délivrance des polypeptides, ou protéines ou anticorps afin qu'elles soient en phase exponentielle de croissance et à 80 % de la confluence lors de l'expérience.

Les cellules immortalisées en suspension (Jurkat, K562) sont préparées le jour avant la transfection à une densité de 2 à 5 x 10⁶ cellules par mL. Le jour de la transfection 1.5 à 5 x 10⁵ cellules sont mises en culture dans une plaque de culture cellulaire 96 puits.

2. La solution de polypeptide et la formulation lipidique obtenue selon l'exemple 8 sont portées à température ambiante, et sont délicatement agités en pipetant plusieurs fois les solutions, par aspiration et refoulement, avant d'être utilisée pour l'essai de délivrance.

3. Dans le fond d'un tube Eppendorf de 1,5 mL, on ajoute 2 µL de la formulation lipidique.

4. On additionne alors 1 µg de polypeptide dans le tube contenant la formulation lipidique. La mixture est agitée délicatement en pipetant plusieurs fois la solution, par aspiration et refoulement, puis incubée pendant 10 minutes à température ambiante.

5. Puis 100 µL de milieu de culture (DMEM) sont additionnés à la mixture polypeptide / formulation lipidique et la solution est agitée délicatement en pipetant plusieurs fois, par aspiration et refoulement.

6. Les complexes sont alors immédiatement ajoutés sur les cellules en culture en milieu complet (transfection en présence de sérum), et la mixture est homogénéisée par agitation de la plaque de culture cellulaire pour permettre une distribution uniforme sur les cellules.

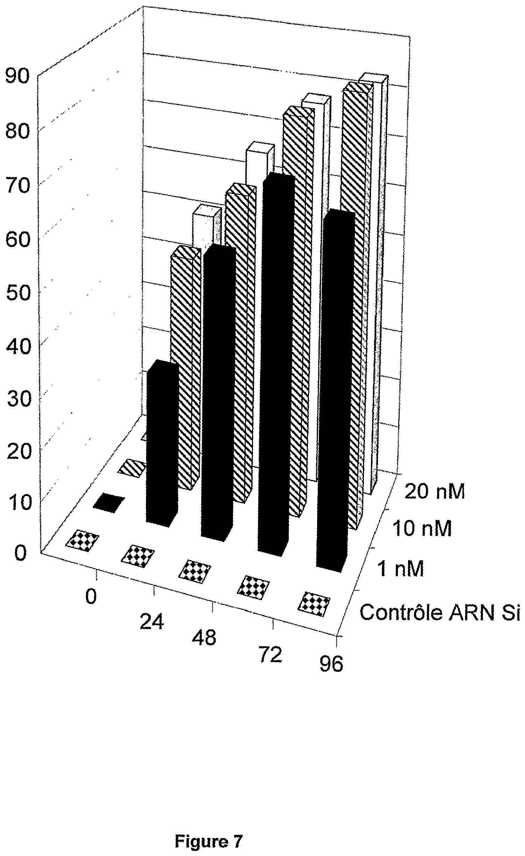

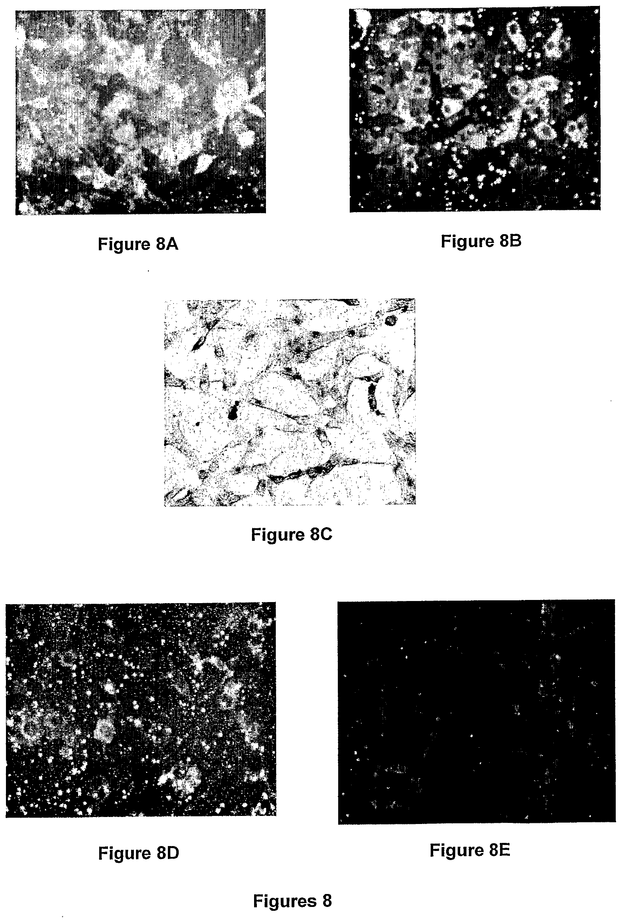

7. Les cellules sont incubées à 37°C en atmosphère humide contenant 5 % de CO₂ selon des conditions standard. L'efficacité de délivrance intracellulaire des polypeptides ou protéines ou anticorps est analysée après 3 à 48 h d'incubation (Figures 7, 8 et 10).

## Revendications

1. Composé amphipathique cationique de Formule (I) : 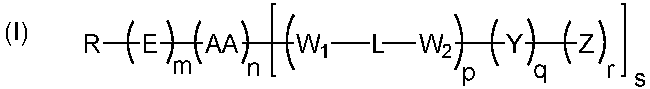 dans laquelle :
R répond à la formule (II) : 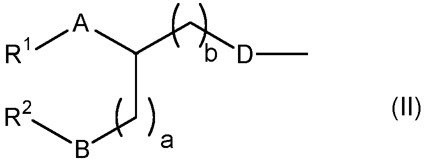
dans laquelle :
• R¹ et R², identiques ou différents, représentent un groupe hydrocarboné, linéaire, ramifié et/ou cyclique, saturé ou insaturé, comprenant de 6 à 24 atomes de carbone ;
• A et B, identiques, représentent un groupement -C(O)-O- ; -CO-NH- ; -NH-CO- ; -NH- ou -O- ;
• A et B, différents, représentent un groupement -C(O)-O- ; -O-C(O)- ; -CO-NH- ; -NH-CO- ; -NH- ou -O- ;
• a est un entier compris entre 1 et 6 ;
• b est un entier compris entre 0 et 6 ;
• D représente un groupement -NH-, -CO-, ou -S- ;
• E représente un groupe hydrocarboné, linéaire ou ramifié, de formule (V) : - G₁-X₁-G₁-, dans laquelle
- X₁ représente un groupe alkylène, linéaire ou ramifié, formant pont et comprenant de 1 à 8 atomes de carbone, et
- G₁ représente un groupe -CO- ou -NH- ;
• m est 1 ;
• AA représente le reste d'un acide aminé ;
• n est un entier égal à 0 ou à 1 ;
• W₁ répond à la formule (VI) : -G₂-X₂- et W₂ répond à la formule (VII) : -X₃-G₃- dans lesquelles X₂ et X₃, identiques ou différents, représentent un groupe alkylène, linéaire ou ramifié, formant pont et comprenant de 1 à 8 atomes de carbone, tandis que G₂ et G₃, identiques ou différents, représentent un groupe -CO-, -NH- ou -O- ;
• L représente un groupement ester (-CO-O-), disulfide (-S-S-), éther de vinyle (-O-C=C-), acylhydrazone (-CO-NR-N=CR'R") ;
• p est un entier égal à 0 ou à 1 ;
• Y est un groupement ramifié qui répond à la formule (VIII) : -CO-X₄-NH-X₅-N-[X₆-NH]₂- ou (IX) : -NH-X₅-N-[X₆-NH]₂-, dans lesquelles X₄, X₅ et X₆, identiques ou différents, représentent un groupe alkylène, linéaire ou ramifié, formant pont et comprenant de 1 à 8 atomes de carbone ;
• q est un entier compris entre 0 et 8 ;
• Z représente un acide aminé basique ou la sérine ;
• r est un entier compris entre 1 et 16, étant entendu que si q est égal à 1 alors r est au moins égal à 2 et que si r est supérieur à 1, alors les groupements Z peuvent être identiques ou différents ;
• s est un entier égal à 1 ou 2 ;
et ses sels d'addition physiologiquement acceptables.

2. Composé selon la revendication 1, **caractérisé en ce que** R répond à la formule (IV) : 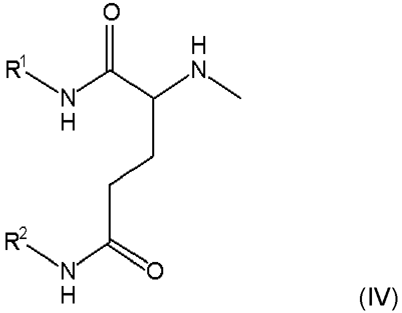 dans laquelle R¹ et R² ont la même signification que précédemment.

3. Composé selon les revendications 1 ou 2, **caractérisé en ce que** E répond à la formule CO-X₁-CO dans laquelle X₁ à la même signification que celle définie à la revendication 1.

4. Composé selon les revendications 1 à 3, **caractérisé en ce que** X₄ représente un méthylène.

5. Composé selon l'une quelconque des revendications 1 à 4, **caractérisé en ce que** X₅ et X₆ représentent, un groupe alkylène formant pont et comprenant de 1 à 4 atomes de carbone.

6. Composé selon l'une quelconque des revendications 1 à 5, **caractérisé en ce que** le contre ion des sels d'addition physiologiquement acceptables est choisi parmi des anions organiques ou inorganiques.

7. Composé choisi parmi

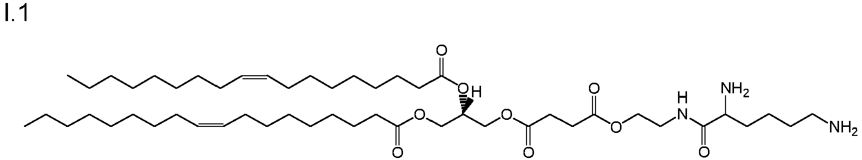

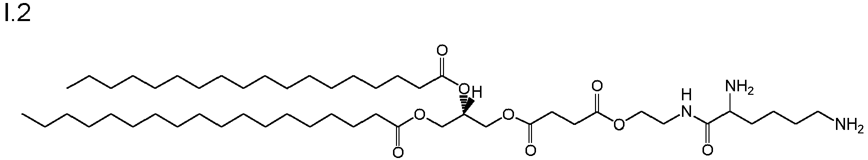

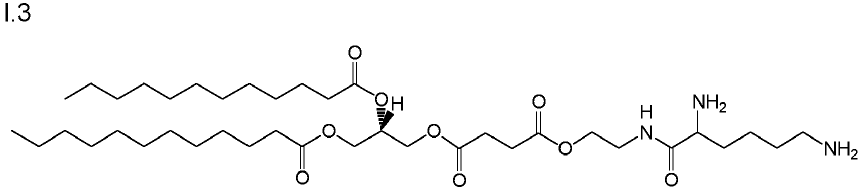

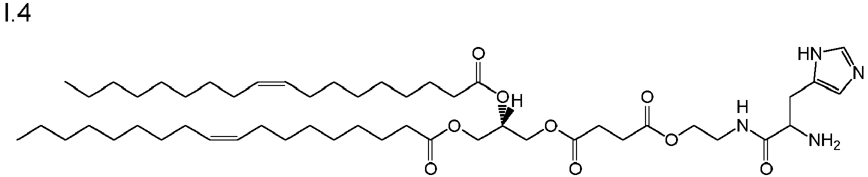

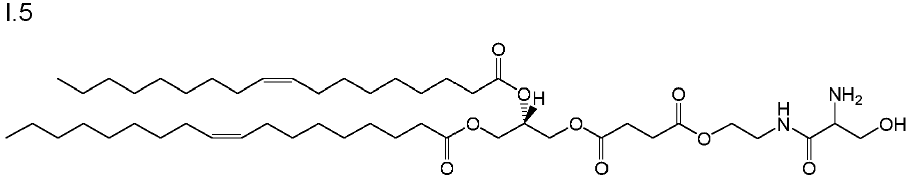

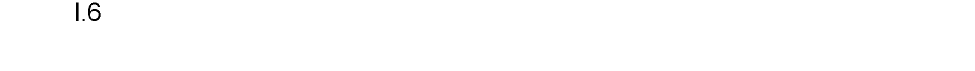

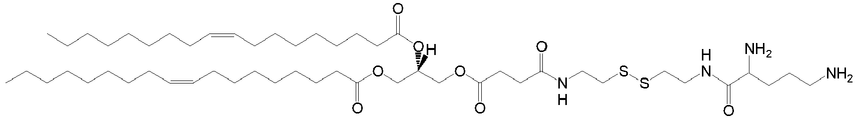

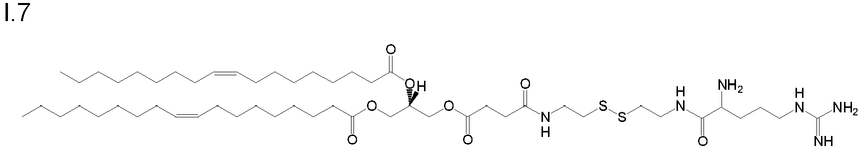

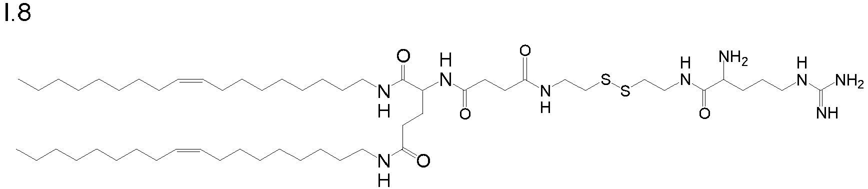

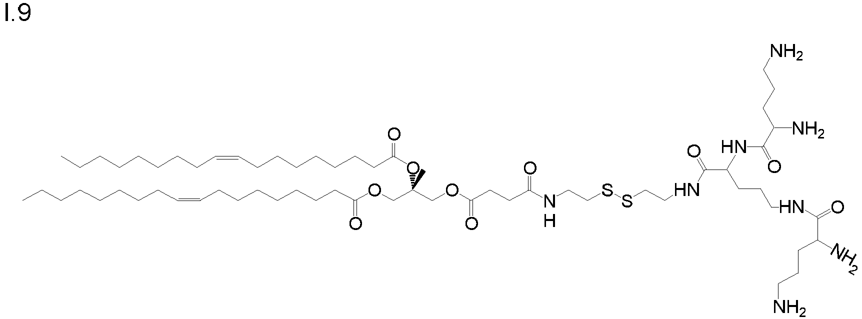

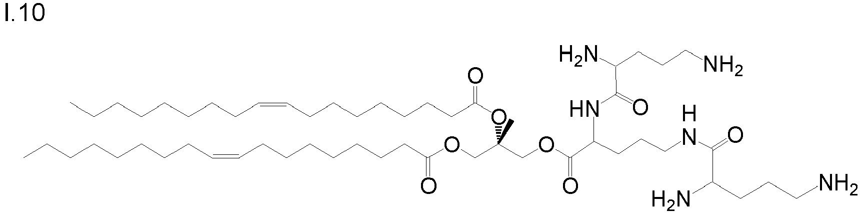

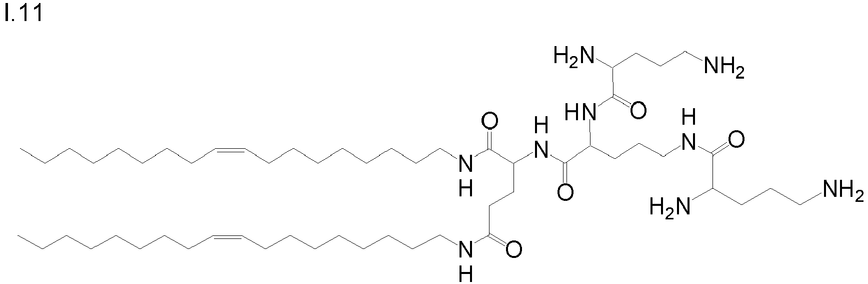

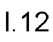

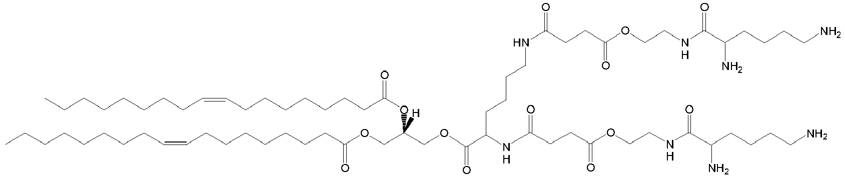

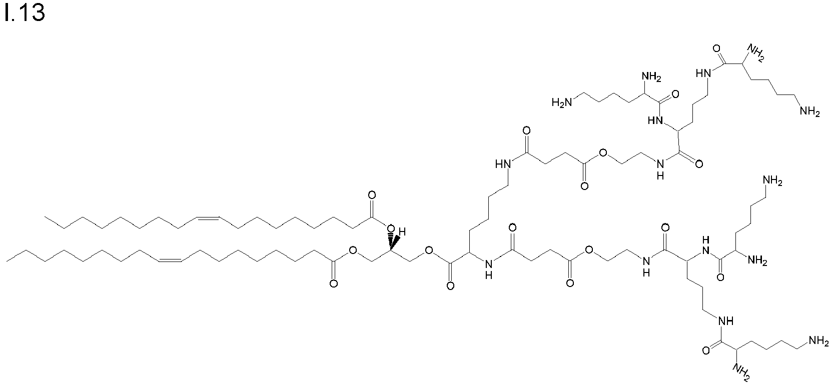

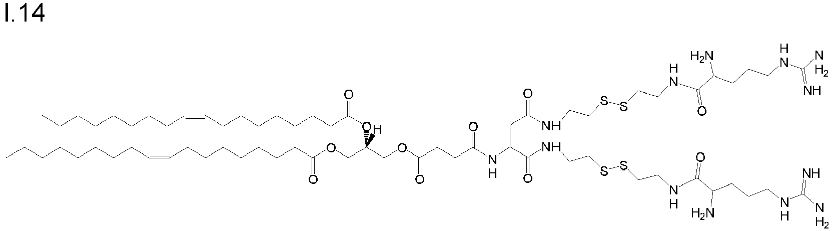

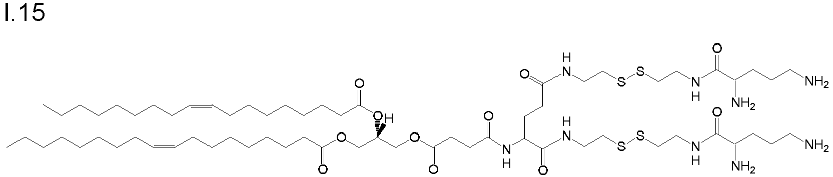

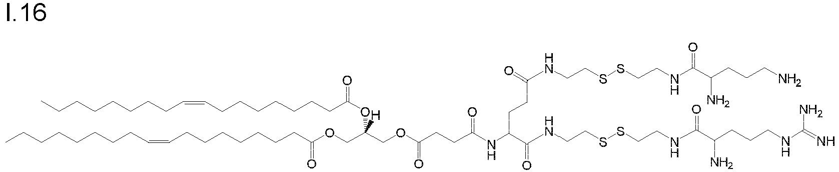

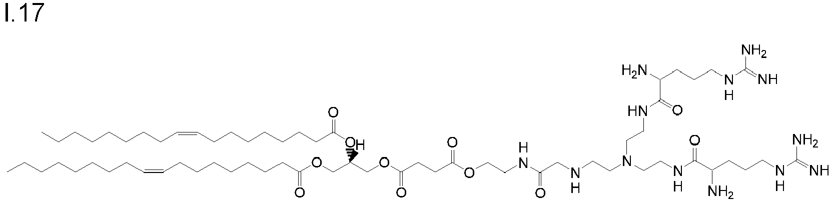

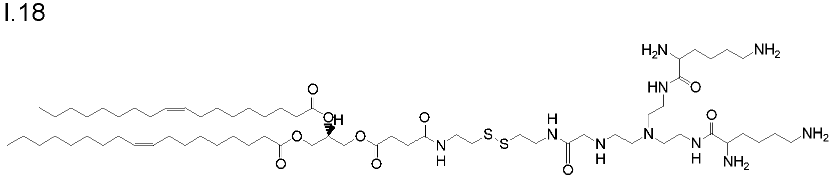

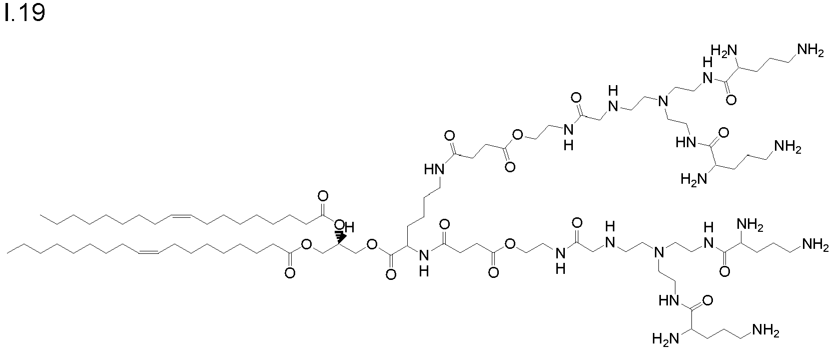

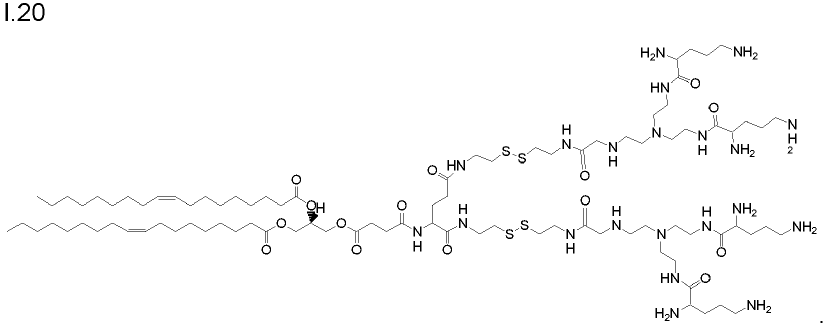

8. Composition, comprenant un composé tel que défini à l'une quelconque des revendications 1 à 7.

9. Composition selon la revendication 8, **caractérisée en ce qu'**elle comprend en outre au moins un acide nucléique ou un polynucléotide.

10. Composition selon la revendication 9, **caractérisée en ce que** le composé et l'acide nucléique sont présents en quantité telle que le rapport de charges positives du composé sur les charges négatives de l'acide nucléique est compris entre 0,1 et 50.

11. Composition selon l'une quelconque des revendications 8 à 10, **caractérisée en ce que** la quantité du composé est comprise entre 1 et 12 nanomoles par µg d'acide nucléique.

12. Composition selon l'une quelconque des revendications 8 à 11, **caractérisée en ce qu'**elle comprend en outre au moins un polypeptide ou une protéine.

13. Composition selon l'une quelconque des revendications 8 à 12, **caractérisée en ce que** le composé et le polypeptide ou la protéine sont présents en quantité telle que la quantité du composé est comprise entre 1 et 10 nanomoles de composé par µg de polypeptide.

14. Composition selon l'une quelconque des revendications 8 à 13, **caractérisée en ce qu'**elle comprend en outre au moins une molécule biologiquement active autre qu'un acide nucléique ou un polypeptide.

15. Composition selon l'une quelconque des revendications 8 à 14, **caractérisée en ce qu'**elle comprend en outre au moins un adjuvant choisi parmi
- un ou plusieurs lipides zwitterioniques ou dépourvu de charges ioniques, ou anioniques ou cationiques,
- ou les éthers lipides,
- ou des lipides comportant une seule chaîne grasse,
- ou les acides lysophosphatidique, qu'ils soient naturels ou synthétiques,
- ou un ou plusieurs polymères, naturels ou synthétiques, co-polymères et/ou dendrimères, cationiques tels que les polyamines, parmi lesquelles la polyéthylènimine, la polylysine, la polyornithine, ou encore le polybrène et le chitosane,
- ou un ou plusieurs polymères, naturels ou synthétiques, co-polymères et/ou dendrimères anioniques tels que l'acide polyglutamique, l'acide polypropylacrylique, l'acide hyaluronique et l'acide polylactique-co-glycolique (PGLA),
- ou un ou plusieurs polymères, naturels ou synthétiques, co-polymères et/ou dendrimères neutres tels que le polyéthylène glycol (PEG) ou encore certains polysaccharides tels que les galactomannanes,
- ou des nanoparticules,
- ou des polypeptides, des protéines, des oses, du glycérol, des cyclodextrines, des histones, l'acide désoxycholique et
- tout autre "activateur" ("enhancer") qui améliore l'efficacité de livraison et la pharmacologie,
- ou des adjuvants capables de cibler spécifiquement un déterminant à la surface et/ou à l'intérieur des cellules, éventuellement attaché de manière covalente ou non covalente audit composé ou à toutes autres molécules contenues dans la composition comprenant ledit composé,
- ou un fluorophore,
- ou la biotine,
- ou des virus, et/ou des organismes unicellulaires.

16. Composé tel que défini à l'une quelconque des revendications 1 à 7, pour son utilisation dans le transfert d'acides nucléiques, de polypeptides ou de tout autre molécule biologiquement active dans les cellules.

17. Utilisation d'un composé tel que défini à l'une quelconque des revendications 1 à 7, pour la préparation d'une composition destinée au transfert d'acides nucléiques, de polypeptides ou de tout autre molécule biologiquement active dans les cellules.

18. Composition comprenant au moins un composé telle que définie à l'une quelconque des revendications 8 à 15 pour son utilisation dans le transfert d'acides nucléiques, de polypeptides ou de tout autre molécule biologiquement active dans les cellules.

19. Méthode *in vitro ou ex vivo* de transfert d'une molécule d'intérêt biologique dans les cellules comprenant les étapes suivantes :
1. la mise en contact de la molécule d'intérêt biologique avec un composé tel que défini à l'une quelconque des revendications 1 à 7, ou avec une composition telle que définie à l'une quelconque des revendications 8 à 15, pour former un complexe molécule active / agent de transfert,
2. la mise en contact des cellules avec le complexe formé en 1.

20. Méthode selon la revendication 19, **caractérisée en ce que** les cellules utilisées sont des cellules préalablement isolées.

21. Méthode selon l'une quelconque des revendications 19 à 20, **caractérisée en ce qu'**elle comprend en outre une ou plusieurs étapes de mise en contact du composé avec un ou plusieurs autre(s) agent(s) de transfection et/ou un ou plusieurs adjuvants.

22. Méthode selon l'une quelconque des revendications 19 à 21, **caractérisée en ce que** l'étape 1 est précédée d'une étape de mise en contact du composé avec un ou d'autres agents de transfection et/ou d'une étape de mise en contact du composé avec le ou les adjuvants.

23. Kit de transfert de matériel biologique, **caractérisé en ce qu'**il comprend au moins un composé tel que défini dans les revendications 1 à 7 ou une composition telle que définie à l'une des revendications 8 à 15.

24. Kit de transfert pour la mise en œuvre de la méthode telle que définie dans l'une quelconque des revendications 19 à 22, **caractérisé en ce qu'**il comprend au moins un composé tel que défini dans les revendications 1 à 7 ou une composition telle que définie à l'une des revendications 8 à 15.

## Patentansprüche

1. Amphipathische kationische Verbindung der Formel (I) : 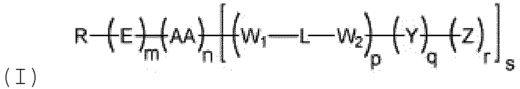 in welcher:
R der Formel (II) entspricht: 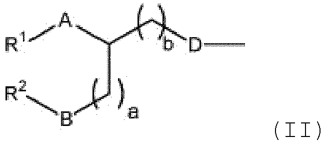
in welcher:
• R¹ und R², identisch oder unterschiedlich, eine lineare, verzweigte und/oder zyklische, gesättigte oder ungesättigte Kohlenwasserstoffgruppe darstellen, die 6 bis 24 Kohlenstoffatome umfasst;
• A und B, identisch, eine -C(O)-O-; -CO-NH-; -NH-CO-; -NH- oder -O-Gruppe darstellen;
• A und B, unterschiedlich, eine -C(O)-O-; -O-C(O)-; -CO-NH-; -NH-CO-; -NH- oder -O-Gruppe darstellen,
• a eine Ganzzahl zwischen 1 und 6 ist;
• b eine Ganzzahl zwischen 0 und 6 ist;
• D eine -NH-, -CO-, -O- oder -S-Gruppe darstellt;
• E eine lineare oder verzweigte Kohlenwasserstoffgruppe der Formel (V) -G₁-X₁-G₁- darstellt, in welcher
- X₁ eine lineare oder verzweigte Alkylengruppe darstellt, die eine Brücke bildet und 1 bis 8 Kohlenstoffatome umfasst, und
- G₁ eine -CO- oder -NH-Gruppe darstellt;
• m 1 ist;
• AA den Rest einer Aminosäure darstellt;
• n eine Ganzzahl gleich 0 oder 1 ist;
• W₁ der Formel (VI) -G₂-X₂- entspricht und W₂ der Formel (VII) -X₃-G₃- entspricht, in denen X₂ und X₃, identisch oder unterschiedlich, eine lineare oder verzweigte Alkylengruppe darstellen, die eine Brücke bildet und 1 bis 8 Kohlenstoffatome umfasst, wohingegen G₂ und G₃, identisch oder unterschiedlich, eine -CO-, -NH- oder -O-Gruppe darstellen;
• L eine Ester- (-CO-O-), Disulfid- (-S-S-), Vinylether- (-O-C=C-), Acylhydrazongruppe (-CO-NR-N=CR'R") darstellt;
• p eine Ganzzahl gleich 0 oder 1 ist;
• Y eine verzweigte Gruppe ist, die der Formel (VIII) -CO-X₄-NH-X₅-N-[X₆-NH]₂- oder (IX) -NH-X₅-N-[X₆-NH]₂-entspricht, in welchen X₄, X₅ und X₆, identisch oder unterschiedlich, eine lineare oder verzweigte Alkylengruppe darstellen, die eine Brücke bildet und 1 bis 8 Kohlenstoffatome umfasst;
• q eine Ganzzahl zwischen 0 und 8 ist;
• Z eine basische Aminosäure oder Serin darstellt;
• r eine Ganzzahl zwischen 1 und 16 ist, wobei gilt, dass, wenn q gleich 1 ist, r mindestens gleich 2 ist und dass, wenn r größer als 1 ist, die Z-Gruppen identisch oder unterschiedlich sein können;
• s eine Ganzzahl gleich 1 oder 2 ist;
und ihre physiologisch akzeptablen Additionssalze.

2. Verbindung nach Anspruch 1, **dadurch gekennzeichnet, dass** R der Formel (IV) entspricht: 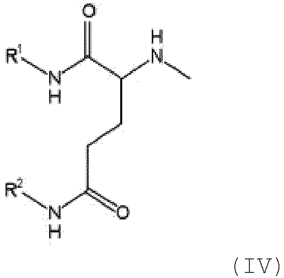 in welcher R¹ und R² dieselbe Bedeutung wie vorgenannt haben.

3. Verbindung nach den Ansprüchen 1 oder 2, **dadurch gekennzeichnet, dass** E der Formel CO-X₁-CO entspricht, in welcher X₁ dieselbe Bedeutung wie die hat, die in Anspruch 1 definiert ist.

4. Verbindung nach den Ansprüchen 1 bis 3, **dadurch gekennzeichnet, dass** X₄ ein Methylen darstellt.

5. Verbindung nach einem der Ansprüche 1 bis 4, **dadurch gekennzeichnet, dass** X₅ und X₆ eine Alkylengruppe darstellen, die eine Brücke bildet und 1 bis 4 Kohlenstoffatome umfasst.

6. Verbindung nach einem der Ansprüche 1 bis 5, **dadurch gekennzeichnet, dass** das Gegenion der physiologisch akzeptablen Additionssalze aus den organischen oder anorganischen Anionen ausgewählt ist.

7. Verbindung, ausgewählt aus:

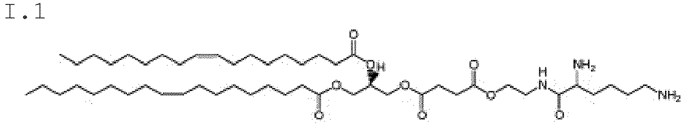

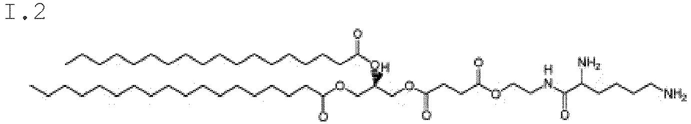

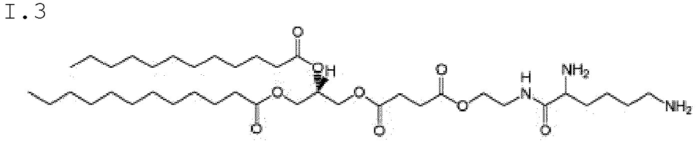

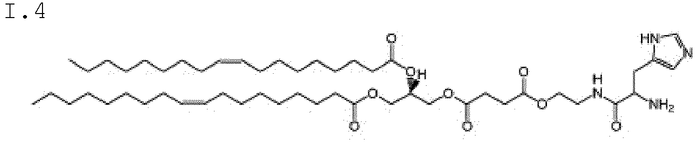

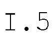

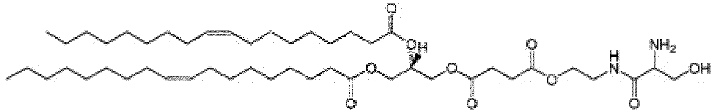

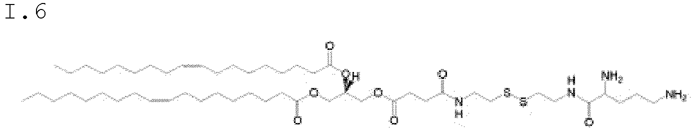

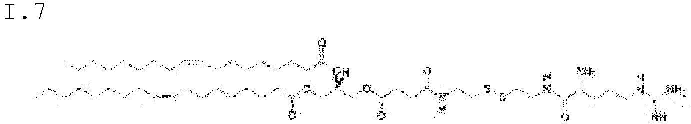

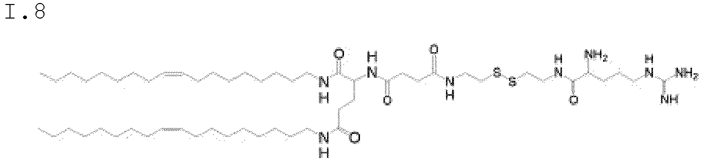

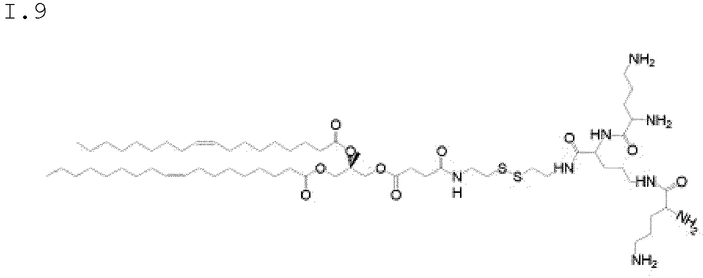

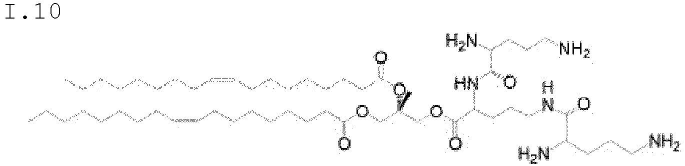

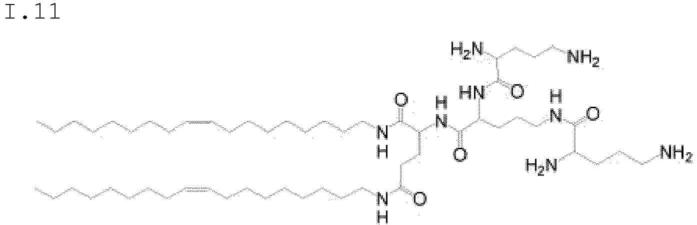

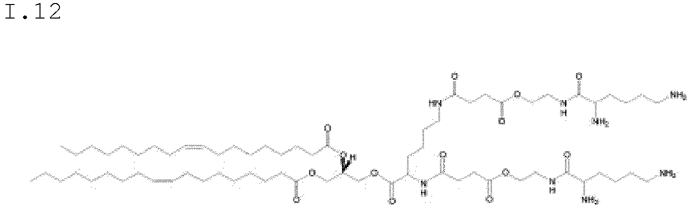

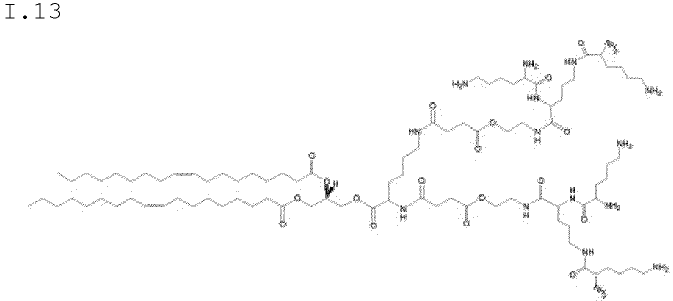

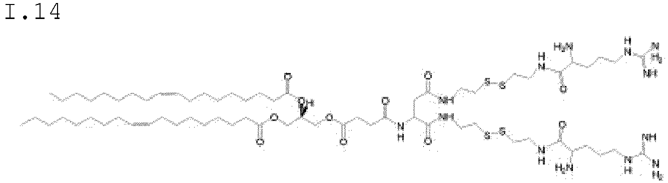

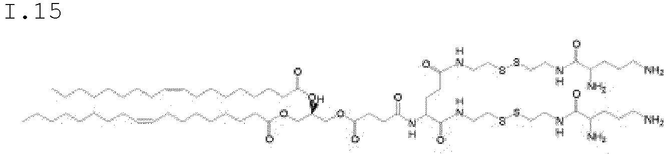

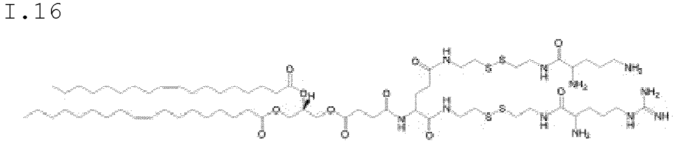

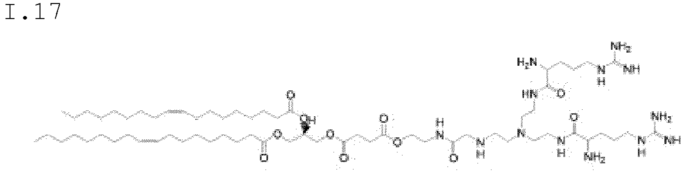

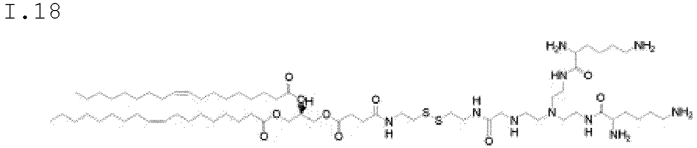

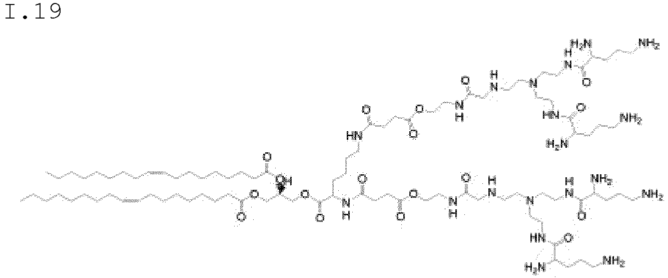

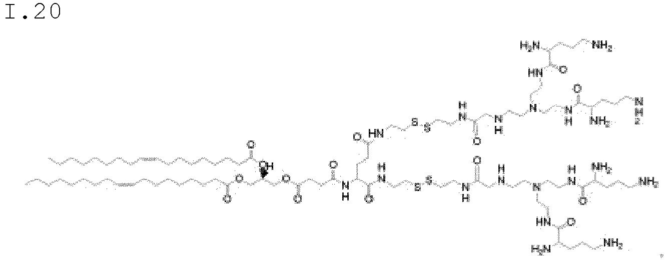

8. Zusammensetzung, umfassend eine Verbindung wie in einem der Ansprüche 1 bis 7 definiert.

9. Zusammensetzung nach Anspruch 8, **dadurch gekennzeichnet, dass** sie ferner mindestens eine Nukleinsäure oder ein Polynukleotid umfasst.

10. Zusammensetzung nach Anspruch 9, **dadurch gekennzeichnet, dass** die Verbindung und die Nukleinsäure in einer Menge vorhanden sind, die derart ist, dass das Verhältnis positiver Ladungen der Verbindung zu den negativen Ladungen der Nukleinsäure zwischen 0,1 und 50 liegt.

11. Zusammensetzung nach einem der Ansprüche 8 bis 10, **dadurch gekennzeichnet, dass** die Menge der Verbindung zwischen 1 und 12 Nanomol je µg Nukleinsäure liegt.

12. Zusammensetzung nach einem der Ansprüche 8 bis 11, **dadurch gekennzeichnet, dass** sie ferner mindestens ein Polypeptid oder ein Protein umfasst.

13. Zusammensetzung nach einem der Ansprüche 8 bis 12, **dadurch gekennzeichnet, dass** die Verbindung und das Polypeptid oder das Protein in einer Menge vorhanden sind, die derart ist, dass die Menge der Verbindung zwischen 1 et 10 Nanomol Verbindung je µg Polypeptid liegt.

14. Zusammensetzung nach einem der Ansprüche 8 bis 13, **dadurch gekennzeichnet, dass** sie ferner mindestens ein biologisch aktives Molekül umfasst, das keine Nukleinsäure oder ein Polypeptid ist.

15. Zusammensetzung nach einem der Ansprüche 8 bis 14, **dadurch gekennzeichnet, dass** sie ferner mindestens einen Hilfsstoff umfasst, der ausgewählt ist aus
- einem oder mehreren zwitterionischen oder ohne ionische Ladungen oder anionischen oder kationischen Lipiden,
- oder den Etherlipiden,
- oder Lipiden mit einer einzigen Fettkette,
- oder den natürlichen oder synthetischen Lysophosphatidinsäuren,
- oder einem oder mehreren Polymeren, natürlichen oder synthetischen, Copolymeren und/oder Dendrimeren, kationischen, wie die Polyamine, darunter Polyethylenimin, Polylysin, Polyornithin, oder auch Polybren und Chitosan,
- oder einem oder mehreren Polymeren, natürlichen oder synthetischen, Copolymeren und/oder Dendrimeren, anionischen, wie Polyglutaminsäure, Polypropylacrylsäure, Hyaluronsäure und Polylactid-co-Glycolid-Säure (PGLA),
- oder einem oder mehreren Polymeren, natürlichen oder synthetischen, Copolymeren und/oder Dendrimeren, neutralen, wie Polyethylenglycol (PEG) oder auch bestimmten Polysacchariden wie die Galactomannane,
- oder Nanopartikeln,
- oder Polypeptiden, Proteinen, Osen, Glycerol, Cyclodextrinen, Histonen, Desoxycholsäure und
- jedem anderen "Aktivator" ("Enhancer"), der die Bereitstellungseffizienz und die Pharmakologie verbessert,
- oder Hilfsstoffen, die imstande sind, speziell einen Determinator an der Oberfläche und/oder im Inneren der Zellen zu bestimmen, der eventuell kovalent oder nicht kovalent mit der Verbindung oder mit allen anderen Molekülen verbunden ist, die in der Zusammensetzung enthalten sind, die die Verbindung umfasst,
- oder einem Fluorophor,
- oder Biotin,
- oder Viren und/oder einzelligen Organismen.

16. Verbindung, wie in einem der Ansprüche 1 bis 7 definiert, für ihre Verwendung beim Transfer von Nukleinsäuren, von Polypeptiden oder von jedem anderen Molekül, das in den Zellen biologisch aktiv ist.

17. Verwendung einer Verbindung, wie in einem der Ansprüche 1 bis 7 definiert, für die Herstellung einer Zusammensetzung, die zum Transfer von Nukleinsäuren, von Polypeptiden oder von jedem anderen Molekül, das in den Zellen biologisch aktiv ist, bestimmt ist.

18. Zusammensetzung, umfassend mindestens eine Verbindung, wie in einem der Ansprüche 8 bis 15 definiert, für ihre Verwendung beim Transfer von Nukleinsäuren, von Polypeptiden oder von jedem anderen Molekül, das in den Zellen biologisch aktiv ist.

19. In vitro- oder ex vivo-Methode zum Transfer eines biologisch interessierenden Moleküls in die Zellen, umfassend die folgenden Schritte:
1. Inkontaktversetzen des biologisch interessierenden Moleküls mit einer Verbindung wie in einem der Ansprüche 1 bis 7 definiert, oder mit einer Zusammensetzung wie in einem der Ansprüche 8 bis 15 definiert, um einen Komplex aktives Molekül/Transfermittel zu bilden,
2. Inkontaktversetzen der Zellen mit dem bei 1 gebildeten Komplex.

20. Methode nach Anspruch 19, **dadurch gekennzeichnet, dass** die verwendeten Zellen zuvor isolierte Zellen sind.

21. Methode nach einem der Ansprüche 19 bis 20, **dadurch gekennzeichnet, dass** sie ferner einen oder mehrere Schritte des Inkontaktversetzens der Verbindung mit einem oder mehreren anderen Transfektionsmittel/n und/oder einem oder mehreren Hilfsstoff/en umfasst.

22. Methode nach einem der Ansprüche 19 bis 21, **dadurch gekennzeichnet, dass** vor dem Schritt 1 ein Schritt des Inkontaktversetzens der Verbindung mit einem oder anderen Transfektionsmitteln und/oder ein Schritt des Inkontaktversetzens der Verbindung mit dem oder den Hilfsstoffen liegt.

23. Kit zum Transfer von biologischem Material, **dadurch gekennzeichnet, dass** es mindestens eine Verbindung wie in den Ansprüchen 1 bis 7 definiert oder eine Zusammensetzung wie in einem der Ansprüche 8 bis 15 definiert, umfasst.

24. Transferkit zur Durchführung der Methode wie in einem der Ansprüche 19 bis 22 definiert, **dadurch gekennzeichnet, dass** es mindestens eine Verbindung wie in den Ansprüchen 1 bis 7 definiert oder eine Zusammensetzung wie in einem der Ansprüche 8 bis 15 definiert, umfasst.

## Claims

1. Cationic amphipathic compound of formula (I): 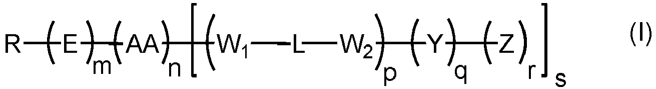 in which:
- R corresponds to formula (II): 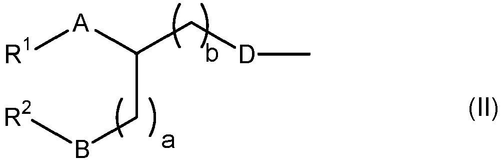 in which:
• R¹ and R², identical or different, represent a linear, branched and/or cyclic, saturated or unsaturated hydrocarbon group comprising from 6 to 24 carbon atoms;
• A and B, identical, represent a -C(O)-O-; -CO-NH-; -NH-CO-; -NH- or -O-group;
• A and B, different, represent a -C(O)-O-; -O-C(O)-; CO-NH-; -NH-CO-; -NH- or -O- group;
• a is an integer comprised between 1 and 6;
• b is an integer comprised between 0 and 6;
• D represents an -NH-, -CO-, or -S- group;
• E represents a linear or branched hydrocarbon group of formula (V) : -G₁-X₁-G₁- in which
- X₁ represents a bridging linear or branched alkylene group comprising from 1 to 8 carbon atoms, and
- G₁ represents a -CO- or -NH- group ;
• m is 1;
• AA represents an amino acid radical;
• n is an integer equal to 0 or 1;
• W₁ corresponds to the formula (VI): -G₂-X₂- and W₂ corresponds to formula (VII): -X₃-G₃- in which X₂ and X₃, identical or different, represents a bridging linear or branched alkylene group comprising from 1 to 8 carbon atoms, whilst G₂ and G₃, identical or different, represent a -CO-, -NH- or -O- group;
• L represents an ester (-CO-O-), disulphide (-S-S-), vinyl ether (-O-C=C-), acylhydrazone (-CO-NR-N=CR'R") group;
• p is an integer equal to 0 or 1;
• Y is a branched group which corresponds to formula (VIII): -CO-X₄-NH-X₅-N-[X₆-NH]₂- or (IX): -NH-X₅-N-[X₆-NH]₂-, in which X₄, X₅ and X₆, identical or different, represent a bridging linear or branched alkylene group comprising from 1 to 8 carbon atoms;
• q is an integer comprised between 0 and 8;
• Z represents a basic amino acid or serine;
• r is an integer comprised between 1 and 16, it being understood that if q is equal to 1 then r is at least equal to 2 and that if r is greater than 1, then the Z groups can be identical or different;
• s is an integer equal to 1 or 2; and its physiologically acceptable addition salts.

2. Compound according to claim 1, **characterized in that** R corresponds to formula (IV) 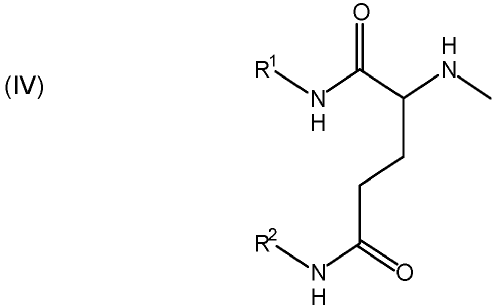 in which R¹ and R² have the same meaning as previously.

3. Compound according to claim 1 or 2, **characterized in that** E corresponds to the formula CO-X₁-CO in which X₁ has the same meaning as defined in claim 1.

4. Compound according to claims 1 to 3, **characterized in that** X₄ represents a methylene.

5. Compound according to any one of claims 1 to 4, **characterized in that** X₅ and X₆ represent a bridging alkylene group comprising from 1 to 4 carbon atoms.

6. Compound according to any one of claims 1 to 5, **characterized in that** the counter-ion of the physiologically acceptable addition salts is chosen from organic or inorganic anions.

7. Compound chosen from :

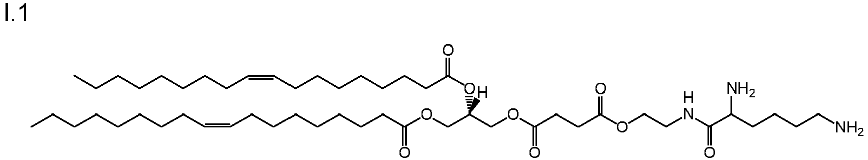

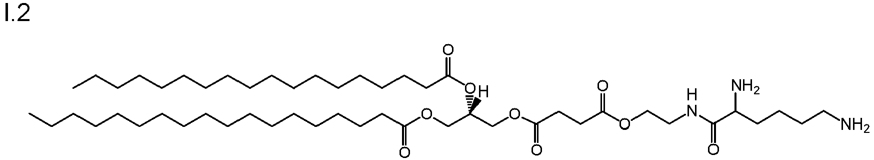

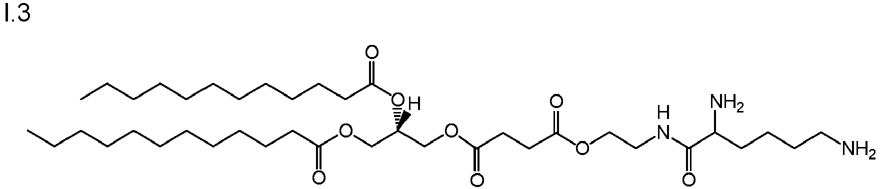

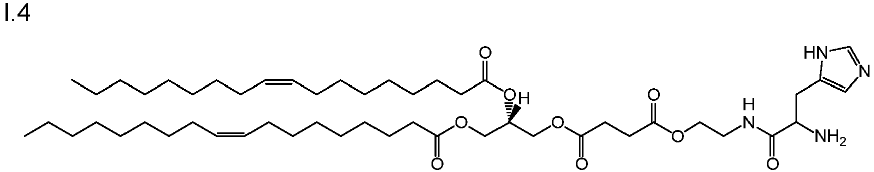

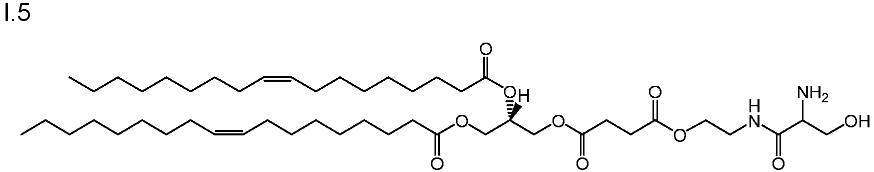

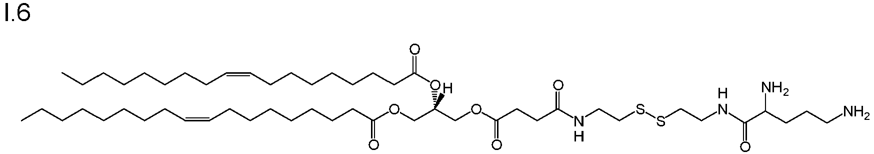

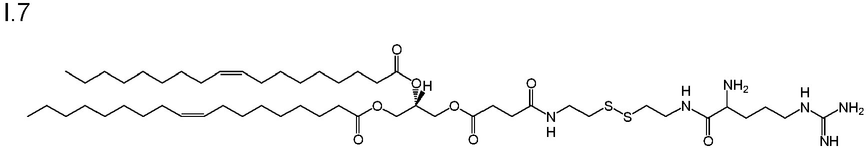

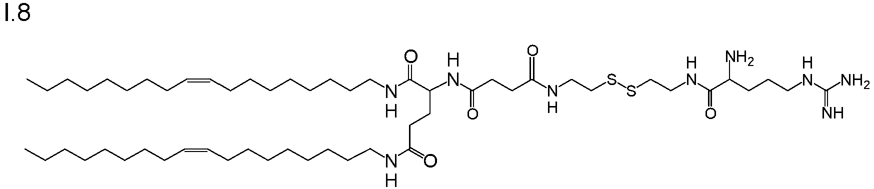

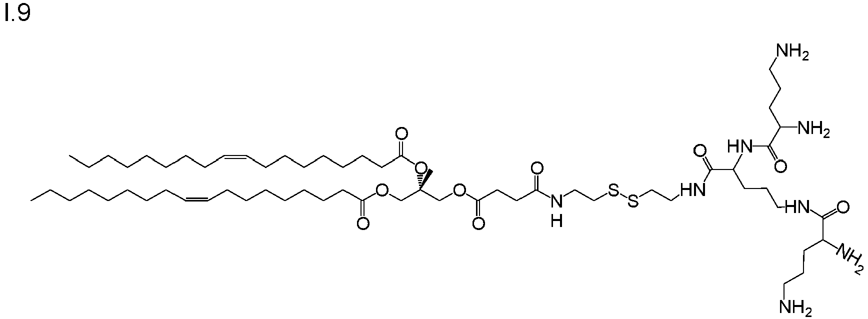

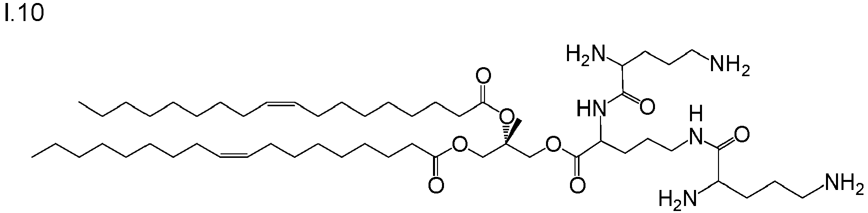

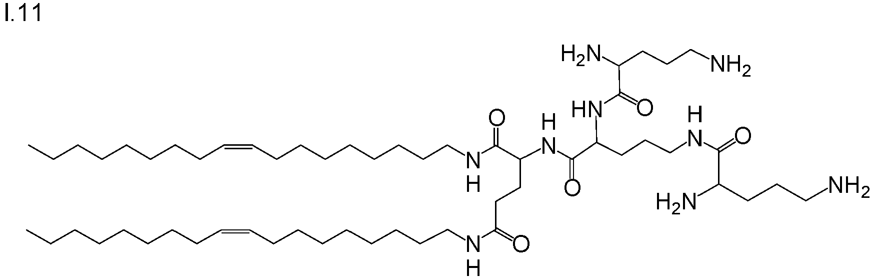

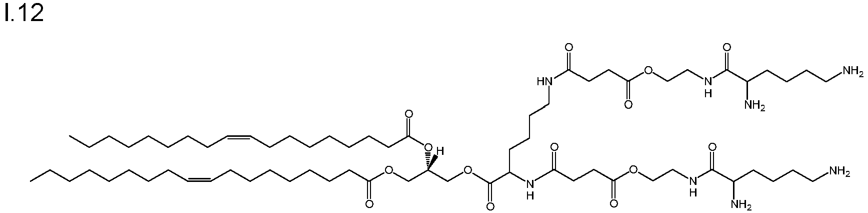

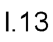

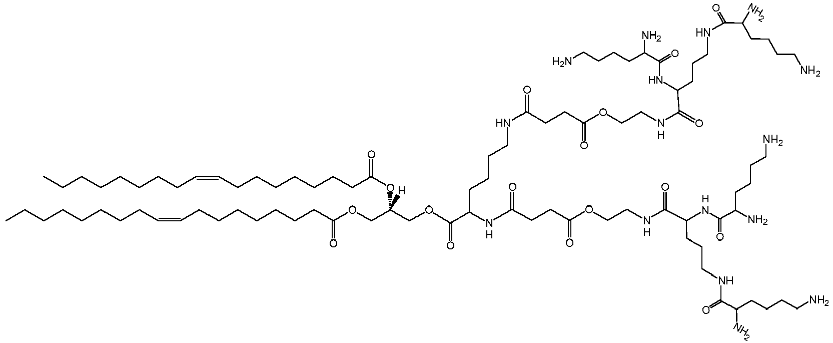

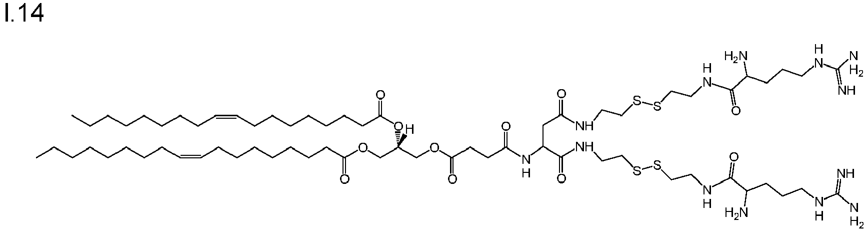

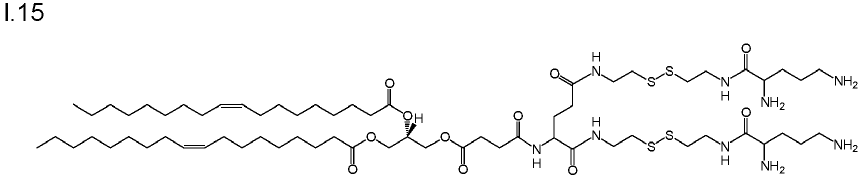

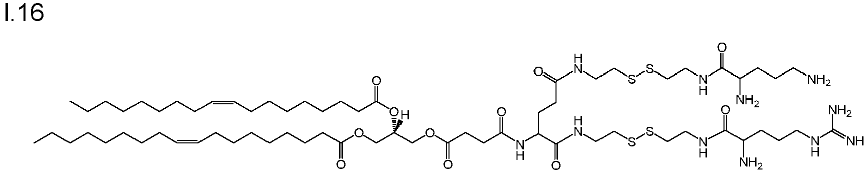

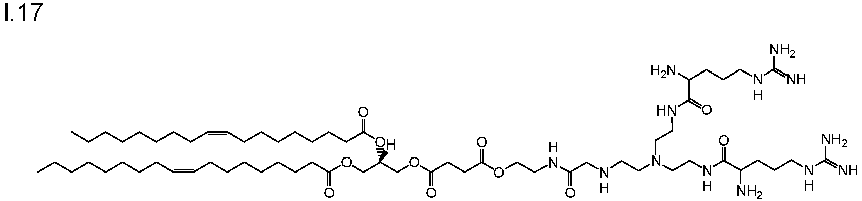

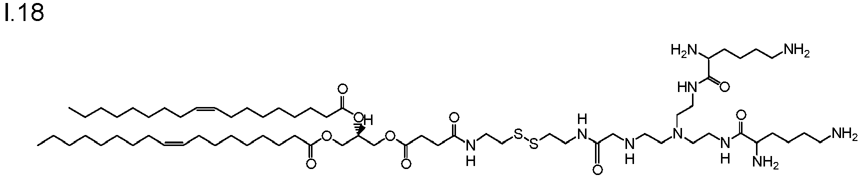

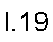

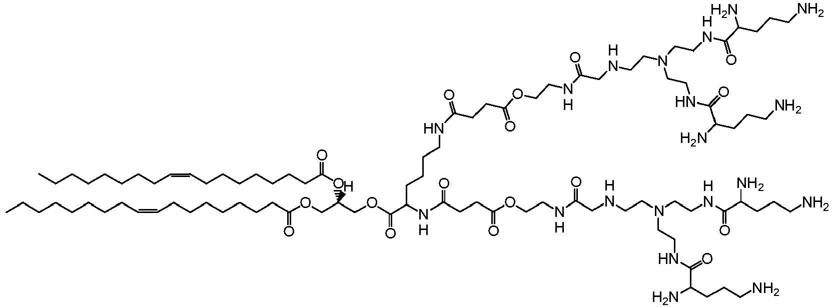

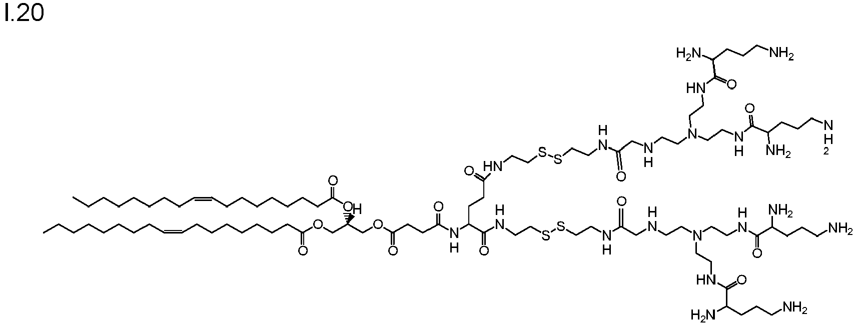

8. Composition, comprising a compound as defined in any one of claims 1 to 7.

9. Composition according to claim 8, **characterized in that** it further comprises at least one nucleic acid or a polynucleotide.

10. Composition according to claim 9, **characterized in that** the compound and the nucleic acid are present in quantities such that the ratio of positive charges to the negative charges of the nucleic acid is comprised between 0.1 and 50.

11. Composition according to any one of claims 8 to 10, **characterized in that** the quantity of compound is comprised between 1 and 12 nanomoles per µg of nucleic acid.

12. Composition according to any one of claims 8 to 11, **characterized in that** it further comprises at least one polypeptide or a protein.

13. Composition according to any one of claims 8 to 12, **characterized in that** the compound and the polypeptide or the protein are present in a quantity such that the quantity of compound is comprised between 1 and 10 nanomoles of compound per µg of polypeptide.

14. Composition according to any one of claims 8 to 13, **characterized in that** it also comprises at least one biologically active molecule other than a nucleic acid or a polypeptide.

15. Composition according to any one of claims 8 to 14, **characterized in that** it further comprises at least one adjuvant selected from:
- one or more zwitterionic or free of ionic charges, anionic or cationic lipids,
- or lipid ethers,
- or lipids comprising a single fatty chain,
- or lysophosphatidic acids, whether natural or synthetic,
- or one or more polymers, natural or synthetic, co-polymers and/or dendrimers cationic such as the polyamines, including polyethylenimine, polylysine, polyornithine, or also polybrene and chitosan,
- or one or more polymers, natural or synthetic, co-polymers and/or dendrimers anionic such as polyglutamic acid, polypropylacrylic acid, hyaluronic acid and polylactic-co-glycolic acid (PGLA),
- or one or more polymers, natural or synthetic, co-polymers and/or dendrimers neutral such as polyethylene glycol (PEG) or also certain polysaccharides such as the galactomannans,
- or nanoparticles,
- or polypeptides, proteins, monosaccharides, glycerol, cyclodextrins, histones, deoxycholic acid and,
- any other "activator" ("enhancer") which improves the efficacy of delivery and the pharmacology,
- or adjuvants capable of specifically targeting a determinant at the surface and/or inside the cells, optionally covalently or non-covalently attached to the compound or to any other molecules contained in the composition comprising the compound,
- or a fluorophore,
- or biotin,
- or viruses, and/or unicellular organisms.

16. Compound as defined in any one of claims 1 to 7, for its use for transferring nucleic acids, polypeptides or any other biologically active molecule into the cells.

17. Use of a compound as defined in any one of claims 1 to 7, for the preparation of a composition intended for the transfer of nucleic acids, polypeptides or any other biologically active molecule into the cells.

18. Composition comprising at least one compound as defined in any one of claims 8 to 15, for its use in the transfer of nucleic acids, polypeptides or any other biologically active molecule into the cells.

19. Method *in vivo* or *ex vivo* for transferring a molecule of biological interest into cells, comprising the following steps:
1. bringing the molecule of biological interest into contact with a compound as defined in any one of claims 1 to 7, or with a composition as defined in any one of claims 8 to 15, in order to form an active molecule / transfer agent complex,
2. bringing the cells into contact with the complex formed in 1.

20. Method according to claim 19, **characterized in that** the cells used are previously isolated cells.

21. Method according to any one of claims 19 to 20, **characterized in that** it further comprises one or more stages of bringing the compound into contact with one or more other transfection agent(s) and/or one or more adjuvants.

22. Method according to any one of claims 19 to 21, **characterized in that** stage 1 is preceded by a stage of bringing the compound into contact with one or more other transfection agents and/or a stage of bringing the compound into contact with the adjuvant or adjuvants.

23. Kit for the transfer of biological material, **characterized in that** it comprises at least one compound as defined in claims 1 to 7 or a composition as defined in any of claims 8 to 15.

24. Transfer kit for the implementation of the method as defined in any one of claims 19 to 22, **characterized in that** it comprises at least one compound as defined in any one of claims 1 to 7 or a composition as defined in any one of claims 8 to 15.
